(19) **Europäisches Patentamt European Patent Office Office européen des brevets**

(11) **EP 4 003 400 B1**

(12) **EUROPEAN PATENT SPECIFICATION**

(45) Date of publication and mention
of the grant of the patent:
**03.04.2024 Bulletin 2024/14**

(21) Application number: **20757034.2**

(22) Date of filing: **24.07.2020**

(51) International Patent Classification (IPC):
**A61K 38/20** (2006.01)     **A61P 37/06** (2006.01)
**C07K 14/54** (2006.01)     **C07K 19/00** (2006.01)

(52) Cooperative Patent Classification (CPC):
**A61K 38/20; A61P 37/06; C07K 14/54;**
**C07K 2319/30**

(86) International application number:
**PCT/US2020/043542**

(87) International publication number:
**WO 2021/021647 (04.02.2021 Gazette 2021/05)**

(54) **DOSING FOR PREVENTION OR TREATMENT OF GRAFT VERSUS HOST DISEASE (GVHD) WITH IL-22 FC FUSION PROTEINS**

DOSIERUNG ZUR VORBEUGUNG ODER BEHANDLUNG VON
GRAFT-VERSUS-HOST-KRANKHEIT (GVHD) MIT IL-22-FC-FUSIONSPROTEINEN

DOSAGE POUR LA PRÉVENTION OU LE TRAITEMENT DE LA MALADIE DU GREFFON CONTRE
L'HÔTE (GVHD) AVEC DES PROTÉINES DE FUSION IL-22-FC

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.07.2019   US 201962879306 P**
       **13.02.2020   US 202062976321 P**

(43) Date of publication of application:
**01.06.2022   Bulletin 2022/22**

(73) Proprietor: **F. Hoffmann-La Roche AG**
**4070 Basel (CH)**

(72) Inventors:
• **KALO, Matthew**
**South San Francisco, CA 94080-4990 (US)**
• **LU, Timothy Then-Chioh**
**South San Francisco, CA 94080-4990 (US)**
• **PECK, Melicent Clare**
**South San Francisco, CA 94080-4990 (US)**
• **SUKUMARAN, Siddharth**
**South San Francisco, CA 94080-4990 (US)**
• **WANG, Yehong**
**South San Francisco, CA 94080-4990 (US)**
• **WONG, Chin Yat**
**South San Francisco, CA 94080-4990 (US)**
• **DAY, Peter William**
**South San Francisco, CA 94080-4990 (US)**

(74) Representative: **Klostermeyer-Rauber, Dörte
F. Hoffmann-La Roche AG
Corporate Law Patents (CLP)
Grenzacherstrasse 124
4070 Basel (CH)**

(56) References cited:
**WO-A1-2015/070077     WO-A1-2019/148020
WO-A1-2019/148026     WO-A1-2019/165140
WO-A2-2014/145016     US-A1- 2018 355 009**

• **LINDEMANS CAROLINE A ET AL: "IL-22 Is an
Intestinal Stem Cell Growth Factor, and IL-22
Administration in Vivo Reduces Morbidity and
Mortality in Murine GvHD", BLOOD, vol. 124, no.
21, December 2014 (2014-12), XP9523729, & 56TH
ANNUAL MEETING OF THE
AMERICAN-SOCIETY-OF-HEMATOLOGY; SAN
FRANCISCO, CA, USA; DECEMBER 06 -09, 2014**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **LINDEMANS CAROLINE A ET AL: "IL-22 Directly Regulates Intestinal Stem Cells, Protecting Epithelium from GvHD and Reducing GvHD Mortality", BIOLOGY OF BLOOD AND MARROW TRANSPLANTATION, vol. 21, no. 2, 2015, XP029133484, ISSN: 1083-8791, DOI: 10.1016/J.BBMT.2014.11.058**
- **CAROLINE A. LINDEMANS ET AL: "Interleukin-22 promotes intestinal-stem-cell-mediated epithelial regeneration", NATURE, vol. 528, no. 7583, 9 December 2015 (2015-12-09), pages 560-564, XP055383955, London ISSN: 0028-0836, DOI: 10.1038/nature16460**
- **OLIVIA B. PARKS ET AL: "Interleukin-22 Signaling in the Regulation of Intestinal Health and Disease", FRONTIERS IN CELL AND DEVELOPMENTAL BIOLOGY, vol. 3, no. 85, 13 January 2016 (2016-01-13), pages 1-13, XP055580794, DOI: 10.3389/fcell.2015.00085**

- **YUSUKE SHONO ET AL: "Gut microbiota injury in allogeneic haematopoietic stem cell transplantation", NATURE REVIEWS CANCER, vol. 18, no. 5, 16 February 2018 (2018-02-16), pages 283-295, XP055719222, London ISSN: 1474-175X, DOI: 10.1038/nrc.2018.10**
- **MICHAEL E. ROTHENBERG ET AL: "-22Fc): A Potential Therapy for Epithelial Injury", CLINICAL PHARMACOLOGY AND THERAPEUTICS, vol. 105, no. 1, 9 January 2019 (2019-01-09), pages 177-189, XP55744981, US ISSN: 0009-9236, DOI: 10.1002/cpt.1164**

Remarks:

The complete document including Reference Table(s) and the Sequence Listing(s) can be downloaded from the EPO website

**Description**

**FIELD OF THE INVENTION**

The present invention relates to interleukin-22 (IL-22) Fc fusion proteins for use in preventing or treating graft versus host disease (GVHD), including acute GVHD (aGVHD) or chronic GVHD (cGVHD).

**BACKGROUND OF THE INVENTION**

Acute GVHD (aGVHD) is a common and life-threatening complication of allogeneic hematopoietic stem cell transplantation (HSCT). Prevention of aGVHD is an integral component of management for patients undergoing HSCT. To date, no pharmacologic therapies have been approved for prophylaxis against aGVHD. Most treatment centers use a combination of immunosuppressive agents targeted against T-cell activation, including a calcineurin inhibitor (CNI, cyclosporine or tacrolimus), methotrexate, mycophenolate, and mTOR inhibitors. Other therapies include T-cell depletion agents, such as anti-thymocyte globulin (ATG) given prior to transplant, and cytotoxic agents, such as cyclophosphamide given after transplant to eliminate activated T cells. Current immunosuppressive strategies to prevent aGVHD increase the incidence of serious infections, delay hematologic recovery, and reduce graft-versus-tumor effects, leading to an increased rate of relapse. Despite the use of standard-of-care (SOC) prophylaxis, Grade II-IV aGVHD develops in approximately 35%-50% of patients after HSCT, with approximately 15% of patients developing severe aGVHD (Grade III-IV). Up to 75% of patients who develop Grade II-IV aGVHD will develop chronic GVHD (cGVHD).

Thus, there is a significant unmet need for new non-immunosuppressive therapies to prevent aGVHD (e.g., corticosteroid-refractory acute GVHD) and the associated significant long-term morbidity and mortality in patients undergoing HSCT. There is also a significant unmet need for new non-immunosuppressive therapies to reduce the risk of developing chronic GVHD.

WO 2014/145016 A2 and US 2018/0355009 A1 disclose IL22 Fc fusion proteins as used herein. WO 2015/070077 A1, Lindemans et al., Blood 2014, Vol. 124 (21), 651, and Lindemans et al., Biology of Blood and Marrow Transplantation 2015, Vol. 21 (2), S58-S59, describe that IL22 directly regulates intestinal stem cells and may be useful for the treatment of conditions of intestinal injury and inflammatory conditions such as graft vs. host disease.

**SUMMARY OF THE INVENTION**

The present invention provides, interleukin-22 (IL-22) Fc fusion proteins for use in of preventing GVHD (e.g., aGVHD or cGVHD), reducing the risk of developing cGVHD, and reducing the risk of corticosteroid-refractory acute GVHD,.

The invention features an interleukin-22 (IL-22) Fc fusion protein for use in a preventing acute GVHD, reducing the risk of developing chronic GVHD, or reducing the risk of corticosteroid-refractory acute GVHD in a subject comprising administering to a subject in need thereof an IL-22 Fc fusion protein in a dosing regimen comprising a dosing cycle, wherein the dosing cycle comprises up to and no more than six total doses of the IL-22 Fc fusion protein, wherein the dosing cycle comprises a first dose (C1 D1) and one or more further doses, wherein each dose is about 60 µg/kg, and wherein the doses are administered to the subject q1w, q2w, q3w, or q4w, and wherein the IL-22 Fc fusion protein comprises the amino acid sequence of SEQ ID NO:8. In some embodiments, the C1D1 is administered to the subject prior to (e.g., 1 day, 2 days, 3 days, 4 days, 5 days, 6 days, 1 week, 2 weeks or 3 weeks, prior to), concurrently with, or after allo-HSCT.

In some embodiments of any of the preceding aspects, the C1D1 is administered to the subject prior to allo-HSCT. In some embodiments, the C1D1 is administered to the subject 1 to 3 days prior to allo-HSCT. In some embodiments, the C1D1 is administered to the subject 1 day prior to allo-HSCT. In some embodiments, the one or more further doses comprise at least a second dose (C1D2). In some embodiments, the one or more further doses comprise at least a C1D2 and a third dose (C1D3). In some embodiments, the one or more further doses comprise at least a C1D2, a C1D3, and a fourth dose (C1D4). In some embodiments, the one or more further doses comprise at least a C1D2, a C1D3, a C1D4, and a fifth dose (C1D5). In some embodiments, the dosing cycle comprises the C1D1, a C1D2, a C1D3, a C1 D4, a C1D5, and a sixth dose (C1D6) of the IL-22 Fc fusion protein.

In some embodiments of any of the preceding aspects, the doses are administered to the subject q2w. In some embodiments, the dosing cycle has a length of about 70 (t3) days. In some embodiments, the dosing cycle has a length of about 70 days. In some embodiments, the dosing cycle consists of a C1D1, a C1D2, a C1D3, a C1D4, a C1D5, and a C1D6, and wherein the C1D1 is administered to the subject 1 day prior to allo-HSCT, the C1D2 is administered to the subject 13 days after allo-HSCT, the C1D3 is administered to the subject 27 days after allo-HSCT, the C1D4 is administered to the subject 41 days after allo-HSCT, the C1D5 is administered to the subject 55 days after allo-HSCT, and the C1D6 is administered to the subject 69 days after allo-HSCT. I

In some embodiments of any of the preceding aspects, the C1D1 is administered to the subject after allo-HSCT. In some embodiments, the C1D1 is administered to the subject 1 to 3 days after allo-HSCT. In some embodiments, the C1D1 is administered to the subject within 2 days of allo-HSCT. In some embodiments, the C1D1 is administered to the subject one day after allo-HSCT.

In some embodiments of any of the preceding aspects, the IL-22 Fc fusion protein is administered to the subject in a pharmaceutical composition.

In some embodiments of any of the preceding aspects, the IL-22 Fc fusion protein is administered to the subject as a monotherapy.

In other embodiments of any of the preceding aspects, the IL-22 Fc fusion protein is administered to the subject as a combination therapy.

## BRIEF DESCRIPTION OF THE DRAWINGS

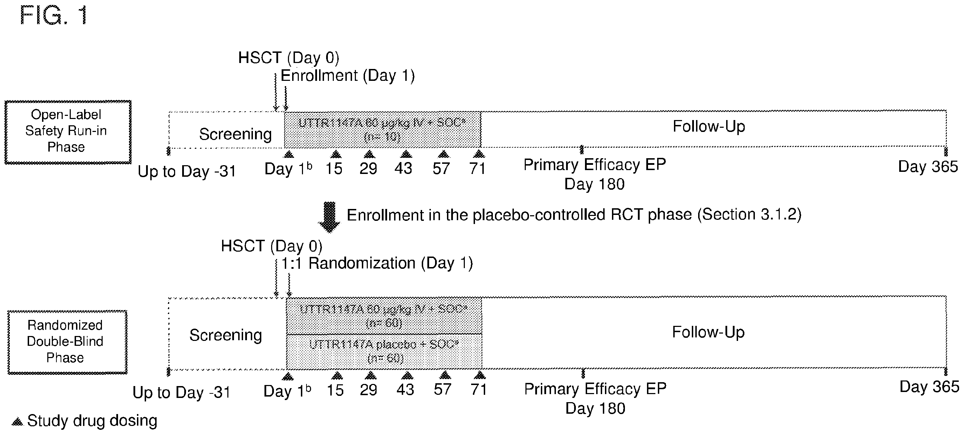

**FIG. 1** is a diagram showing a study schema for the clinical trial described in Example 1. HSCT, allogeneic hematopoietic stem cell transplantation; EP, endpoint; RCT, randomized controlled trial; SOC, standard of care. [a]SOC is as described in Example 1. [b]First dose of study drug administered on Day 1 (+ 1 day) after HSCT. HSCT performed on Day 0.

**FIG. 2** shows an amino acid sequence alignment of mature IL-22 from different mammalian species: human (GenBank Accession No.Q9GZX6, SEQ ID NO:4, chimpanzee (GenBank Accession No.XP_003313906, SEQ ID NO:48), orangutan (GenBank Accession No. XP_002823544, SEQ ID NO:49), mouse (GenBank Accession No. Q9JJY9, SEQ ID NO:50), and dog (GenBank Accession No. XP_538274, SEQ ID NO:51).

**FIG. 3** shows a study schema of the Phase 1b clinical trial described in Example 20.

**FIGS. 4A** and **4B** are a series of graphs showing pharmacokinetic (PK) results from the Phase 1b clinical trial described in Example 20. Fig. 4A shows UTTR1147A serum-concentration profiles in healthy volunteers (HVs) and ulcerative colitis (UC) patients. Fig. 4B shows relative exposures for HVs and UC patients at the Q4W and Q2W regimens. Data are mean (SD).

**FIG. 5** is a series of graphs showing mean percent change from baseline in serum C-reactive protein (CRP) and REG3A in HVs and UC patients. Box plots indicate data distribution and are connected by lines indicating the mean. Insets show timepoints from Days 1-15.

**FIG. 6** shows clinical responses and clinical remission in UC patients at Week 4 and Week 12 of the Phase 1b clinical trial described in Example 20.

**FIG. 7** shows a study schema of the Phase 1b clinical trial described in Example 21. DLT, dose-limiting toxicity; EOS, end of study; Q2W, every two weeks; Q4W, every four weeks. Note: hematopoietic stem cell transplantation = Day 0.

## DETAILED DESCRIPTION

## I. DEFINITIONS

Unless otherwise defined, all terms of art, notations, and other scientific terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art.

The term "about" as used herein refers to the usual error range for the respective value readily known to the skilled person in this technical field. Reference to "about" a value or parameter herein includes (and describes) embodiments that are directed to that value or parameter *per se*.

As used herein, the singular forms "a," "an," and "the" include plural referents unless the context clearly dictates otherwise. For example, reference to "an isolated peptide" means one or more isolated peptides.

Throughout this specification and claims, the word "comprise," or variations such as "comprises" or "comprising" will be understood to imply the inclusion of a stated integer or group of integers but not the exclusion of any other integer or group of integers.

The term "IL-22 Fc fusion protein" or "IL-22 fusion protein" or "IL-22 Ig fusion protein" as used herein refers to a fusion protein in which IL-22 protein or polypeptide is linked, directly or indirectly, to an IgG Fc region. In some embodiments, the IL-22 protein or polypeptide is glycosylated. In certain preferred aspects, the IL-22 Fc fusion protein comprises a human IL-22 protein or polypeptide linked to a human IgG Fc region. In certain aspects, the human IL-22 protein comprises the amino acid sequence of SEQ ID NO:4. However, it is understood that minor sequence variations such as insertions, deletions, substitutions, especially conservative amino acid substitutions of IL-22 or Fc that do not affect the function and/or activity of IL-22 or IL-22 Fc fusion protein are also contemplated herein. The IL-22 Fc fusion protein can bind to IL-22 receptor, which can lead to IL-22 receptor downstream signaling. In certain aspects, the IL-22 Fc fusion protein is capable of binding to IL-22 receptor, and/or is capable of leading to IL-22 receptor downstream signaling. The functions and/or activities of the IL-22 Fc fusion protein can be assayed by methods known in the art, including without limitation, enzyme-linked immunosorbent assay (ELISA), ligand-receptor binding assay and Stat3 luciferase assay. In certain aspects, disclosed is an IL-22 Fc fusion protein that binds to IL-22 receptor, in which the binding can lead to IL-22 receptor downstream signaling, the IL-22 Fc fusion protein comprising an amino acid sequence having at least 95% sequence identity to the amino acid sequence selected from the group consisting of SEQ ID NO:8, SEQ ID NO:10, SEQ ID NO:12, SEQ ID NO:14, and SEQ ID NO:16, and wherein the Fc region is not glycosylated. In certain particular aspects, the Fc region of the IL-22 fusion protein does not possess effector activities (e.g., does not bind to FcγIIIR) or exhibits substantially lower effector activity than a whole (e.g., wild-type) IgG antibody. In certain other aspects, the Fc region of the IL-22 Fc fusion protein does not trigger cytotoxicity such as antibody-dependent cellular cytotoxicity (ADCC) or complement dependent cytotoxicity (CDC). Unless otherwise specified, "IL-22 fusion protein," "IL-22 Fc fusion," "IL-22 Ig fusion protein," "IL-22 Fc fusion protein," or "IL-22 Fc" are used interchangeably throughout this application.

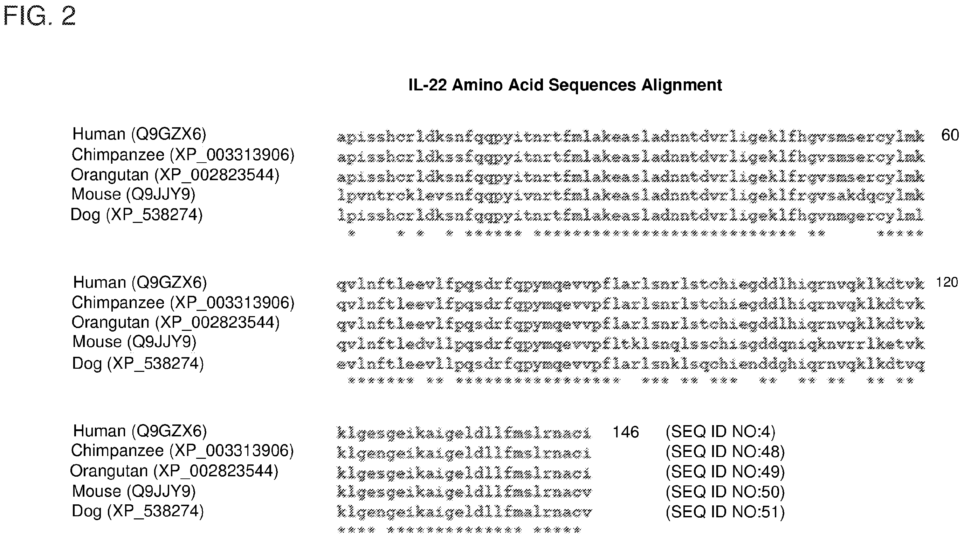

The term "IL-22" or "IL-22 polypeptide" or "IL-22 protein" as used herein, broadly refers to any native IL-22 from any mammalian source, including primates (e.g. humans) and rodents (e.g., mice and rats), unless otherwise indicated. The term encompasses "full-length," unprocessed IL-22 as well as any forms of IL-22 that result from processing in the cell. For example, both full-length IL-22 containing the N-terminal leader sequence and the mature form IL-22 are encompassed herein. The leader sequence (or signal peptide) can be the endogenous IL-22 leader sequence or an exogenous leader sequence of another mammalian secretary protein. In certain aspects, the leader sequence can be from a eukaryotic or prokaryotic secretary protein. The term also encompasses naturally occurring variants of IL-22, e.g., splice variants or allelic variants. The amino acid sequence of an exemplary human IL-22 is shown in SEQ ID NO:4 (mature form, without a signal peptide). In certain aspects, the amino acid sequence of full-length IL-22 protein with the endogenous leader sequence is provided in SEQ ID NO:71; while in other aspects, the amino acid sequence of mature IL-22 protein with an exogenous leader sequence is provided in SEQ ID NO:2. Minor sequence variations, especially conservative amino acid substitutions of IL-22 that do not affect the IL-22's function and/or activity (e.g., binding to IL-22 receptor), are also contemplated. Fig. 2 shows an amino acid sequence alignment of mature IL-22 from several exemplary mammalian species. The asterisks indicate highly conserved amino acid residues across species that are likely important for the functions and/or activities of IL-22. Accordingly, in certain aspects, the IL-22 Fc fusion protein comprises an IL-22 polypeptide comprising an amino acid sequence having at least 95%, at least 96%, at least 97%, at least 98%, or at least 99% sequence identity to SEQ ID NO:4. In certain other aspects, the IL-22 protein has 95% or more sequence identity to SEQ ID NO:71; 96% or more sequence identity to SEQ ID NO:71; 97% or more sequence identity to SEQ ID NO:71; 98% or more sequence identity to SEQ ID NO:71; or 99% or more sequence identity to SEQ ID NO:71. The IL-22 polypeptides described herein can be isolated from a variety of sources, such as from human tissue or from another source, or prepared by recombinant or synthetic methods.

The term "IL-22 receptor" or "IL-22R" refers to a heterodimer consisting of IL-22R1 and IL-10R2 or naturally occurring allelic variants thereof. See, e.g., Ouyang et al., Annu. Rev. Immunol. 29:159-63, 2011. IL-10R2 is ubiquitously expressed by many cell types, and IL-22R1 is expressed only in innate cells such as epithelial cells, hepatocytes and keratinocytes. IL-22R1 is also known as IL-22Ra1 or IL-22Rα1. IL-22R1 may be paired with other polypeptides to form heterodimeric receptors for other IL-10 family members, for example IL-20 or IL-24. See, e.g., Ouyang et al., 2011, *supra.*

A "native sequence IL-22 polypeptide" or a "native sequence IL-22R polypeptide" refers to a polypeptide comprising the same amino acid sequence as a corresponding IL-22 or IL-22R polypeptide derived from nature. Such native sequence IL-22 or IL-22R polypeptides can be isolated from nature or can be produced by recombinant or synthetic means. The terms specifically encompass naturally-occurring truncated or secreted forms of the specific IL-22 or IL-22R polypeptide (e.g., an IL-22 lacking its associated signal peptide), naturally-occurring variant forms (e.g., alternatively spliced forms), and naturally-occurring allelic variants of the polypeptide. In various aspects, the native sequence IL-22 or IL-22R polypeptides disclosed herein are mature or full-length native sequence polypeptides. An exemplary full length native human IL-22 is shown in SEQ ID NO:70 (DNA) and SEQ ID NO:71 (protein). While the IL-22 and IL-22R polypeptide sequences are shown to begin with methionine residues designated herein as amino acid position 1, it is conceivable and possible that other methionine residues located either upstream or downstream from the amino acid position 1 can be employed as the starting amino acid residue for the IL-22 or IL-22R polypeptides.

An "IL-22 variant," an "IL-22R variant," an "IL-22 variant polypeptide," or an "IL-22R variant polypeptide" means an active IL-22 or IL-22R polypeptide as defined above having at least about 80% amino acid sequence identity with a full-length native sequence IL-22 or IL-22R polypeptide sequence. Ordinarily, an IL-22 or IL-22R polypeptide variant will have at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity, and alternatively at least about 99% amino acid sequence identity to a full-length or mature native sequence IL-22 or IL-22R polypeptide sequence.

The term "Fc region," "Fc domain," or "Fc" refers to a C-terminal non-antigen binding region of an immunoglobulin heavy chain that contains at least a portion of the constant region. The term includes native Fc regions and variant Fc regions. In certain aspects, a human IgG heavy chain Fc region extends from Cys226 to the carboxyl-terminus of the heavy chain. However, the C-terminal lysine (Lys447) of the Fc region may or may not be present, without affecting the structure or stability of the Fc region. Unless otherwise specified herein, numbering of amino acid residues in the IgG or Fc region is according to the EU numbering system for antibodies, also called the EU index, as described in Kabat et al., Sequences of Proteins of Immunological Interest, 5th Ed. Public Health Service, National Institutes of Health, Bethesda, MD, 1991.

In certain aspects , Fc region refers to an immunoglobulin IgG heavy chain constant region comprising a hinge region (starting at Cys226), an IgG CH2 domain, and CH3 domain. The term "hinge region" or "hinge sequence" as used herein refers to the amino acid sequence located between the linker and the CH2 domain. In certain aspects, the hinge region comprises the amino acid sequence CPPCP (SEQ ID NO:31). In certain aspects, the hinge region for IL-22 IgG4 Fc fusion protein comprises the CPPCP sequence (SEQ ID NO:31), a sequence found in the native IgG1 hinge region, to facilitate dimerization. In certain other aspects, the Fc region starts at the hinge region and extends to the C-terminus of the IgG heavy chain. In certain particular aspects, the Fc region comprises the Fc region of human IgG1, IgG2, IgG3 or IgG4. In certain particular aspects, the Fc region comprises the CH2 and CH3 domain of IgG4. In certain other particular aspects, the Fc region comprises the CH2 and CH3 domain of IgG1.

In certain aspects, the IgG CH2 domain starts at Ala 231. In certain other aspects, the CH3 domain starts at Gly 341. It is understood that the C-terminus Lys residue of human IgG can be optionally absent. It is also understood that conservative amino acid substitutions of the Fc region without affecting the desired structure and/or stability of Fc is contemplated herein.

In certain aspects, the IL-22 is linked to the Fc region via a linker. In certain particular aspects, the linker is a peptide that connects the C-terminus of IL-22 to the Fc region as described herein. In certain aspects, native IgG sequences are present in the linker and/or hinge region to minimize and/or avoid the risk of immunogenicity. In other aspects, minor sequence variations can be introduced to the native sequences to facilitate manufacturing. IL-22 Fc fusion constructs comprising exogenous linker or hinge sequences that exhibit high activity (as measured, e.g., by a luciferase assay) are also within the scope of the invention. In certain aspects, the linker comprises an amino acid sequence that is 8-20 amino acids, 8-16, 8-15, 8-14, 8-13, 8-12, 8-11, 8-10, 8-9, 10-11, 10-12, 10-13, 10-14, 10-15, 10-16, 11-16, 8, 9, 10, 11, 12, 13, 14, 15, or 16 amino acids long. In certain other aspects, the linker comprises the amino acid sequence DKTHT (SEQ ID NO:32). In certain particular aspects, the linker does not comprise the sequence Gly-Gly-Ser (SEQ ID NO:45), Gly-Gly-Gly-Ser (SEQ ID NO:46), or Gly-Gly-Gly-Gly-Ser (SEQ ID NO:47).

In certain aspects, the IL-22 Fc fusion protein comprises an IL-22 polypeptide linked to an Fc region by a linker. The term "linked to" or "fused to" refers to a covalent bond, e.g., a peptide bond, formed between two moieties.

The terms "glycosylation" and "glycosylated" as used herein refers to the presence of a carbohydrate (e.g., an oligosaccharide or a polysaccharide, also referred to as a "glycan") attached to biological molecule (e.g., a protein or a lipid). In particular aspects, glycosylation refers to the presence of a glycan (e.g., an N-glycan) attached to a protein (e.g., an IL-22 Fc fusion protein) or a portion of a protein of interest (e.g., an IL-22 polypeptide moiety of an IL-22 Fc fusion protein). N-linked glycosylation refers to the attachment of the carbohydrate moiety to the side-chain of an asparagine residue. The tripeptide sequences, asparagine-X-serine and asparagine-X-threonine, wherein X is any amino acid except proline, are recognition sequences for enzymatic attachment of the carbohydrate moiety to the asparagine side chain. O-linked glycosylation refers to the attachment of one of the sugars N-acetylgalactosamine, galactose, or xylose to a hydroxyamino acid, most commonly serine or threonine, although 5-hydroxyproline or 5-hydroxylysine can also be involved in O-linked glycosylation. For a review of glycosylation, see, e.g., Varki et al., Essentials of Glycobiology, 3rd Edition, Cold Spring Harbor Laboratory Press, 2015-2017.

The terms "aglycosylated" and "not glycosylated," as used interchangeably herein, refer to a protein or a portion of a protein of interest (e.g., the Fc region of an IL-22 Fc fusion protein) that is not glycosylated (e.g., not N-glycosylated). It is to be understood that in some aspects, a portion of a protein of interest (e.g., an IL-22 Fc fusion protein) is glycosylated (e.g., the IL-22 polypeptide portion of an IL-22 Fc fusion protein), while another portion of the protein of interest is not glycosylated (e.g., the Fc region of the IL-22 Fc fusion protein).

In some aspects, provided herein are IL-22 Fc fusion proteins in which the Fc region or CH2 domain is not glycosylated. In certain aspects, the N-glycosylation site in the CH2 domain is mutated to prevent glycosylation. For example, an IL-22 Fc fusion protein with an aglycosylated Fc region can be made by mutagenizing the amino acid residue at position 297 as in the EU index in the CH2 domain of the Fc region (e.g., N297). In certain aspects, the glycosylation in the CH2 domain of the Fc region can be eliminated by altering the glycosylation consensus site, i.e., Asn at position 297 followed by any amino acid residue (in the case of human IgG, Ser) and Thr. The glycosylation site can be altered by amino acid insertions, deletions, and/or substitutions. For example, one or more amino acid residues can be inserted between Asn and Ser or between Ser and Thr to alter the original glycosylation site, wherein the insertions do not regenerate an N-glycosylation site. In certain particular aspects, the amino acid residue at position 297 as in the EU index (e.g., the N-glycosylated site in Fc) within the CH2 domain of human IgG Fc is mutated to abolish the glycosylation site. In certain particular aspects, the amino acid residue at position 297 as in the EU index (e.g., N297) is changed to Gly, Ala, Gln, Asp, or Glu. In some particular aspects, the amino acid residue at position 297 as in the EU index (e.g., N297) is changed to Gly or Ala. In other particular aspects, the amino acid residue at position 297 as in the EU index (e.g., N297) is changed to Gly. In certain other aspects, the amino acid residue at position 299 as in the EU index can be substituted with another amino acid, for example, Ala, Val, or Gly. In certain particular embodiments, the mutations that result in an aglycosylated Fc do not affect the structure and/or stability of the IL-22 Fc fusion protein.

In certain aspects, the IL-22 Fc fusion protein comprises an Fc region in which the amino acid residue at position 297 as in the EU index in the CH2 domain is mutated. In certain aspects, the amino acid residue at position 297 as in the EU index is changed to Gly or Ala, preferably to Gly. In certain other aspects, the amino acid residue at position 297 as in the EU index is deleted. In certain aspects s, the IL-22 Fc fusion protein comprising an Fc having an amino acid substitution at the amino acid residue at position 297 as in the EU index is aglycosylated or not glycosylated.

In other aspects, the N-glycan attached to the wild type amino acid residue at position 297 as in the EU index (e.g., N297) can be removed enzymatically, e.g., by deglycosylation. Suitable glycolytic enzymes include without limitation, peptide-N-glycosidase (PNGase).

The term "glycosylation occupancy" as used herein refers to the probability that a protein is glycosylated at a particular glycosylation site (e.g., an Asn residue of a consensus glycosylation site) or the percentage of proteins in a population of proteins that are glycosylated at a particular glycosylation site. For example, an IL-22 polypeptide may be glycosylated on amino acid residues Asn21, Asn35, Asn64, and/or Asn143 of SEQ ID NO: 4. In a further specific example, (a) the percent N-glycosylation site occupancy at residue Asn21 may be in the range of 70 to 90; (b) the percent N-glycosylation site occupancy at residue Asn35 may be in the range of 90 to 100; (c) the percent N-glycosylation site occupancy at residue Asn64 may be in the range of 90 to 100; and/or (d) the percent N-glycosylation site occupancy at residue Asn143 may be in the range of 25 to 35.

The terms "sialylation" and "sialylated" refers to the presence of sialic acid on a protein or a portion of a protein of interest, particularly as a component of a glycan (e.g., N-glycan) chain attached to a protein. Sialic acid (also referred to herein as a "sialic acid moiety") refers generally to N- or O-substituted derivatives of neuraminic acid. N-acetylneuraminic acid (5-acetamido-2-keto-3,5-dideoxy-D-glycero-D-galactonononic acid; also known as NANA or Neu5Ac) is the most common sialic acid in mammals. Other exemplary sialic acids include , 2-keto-3-deoxy-D-glycero-D-galactonononic acid (also known as Kdn), N-glycolylneuraminic acid (also known as Neu5Gc or NGNA), neuraminic acid (also known as Neu), and 2-deoxy-2,3-didehydro-Neu5Ac (also known as Neu2en5Ac). Free sialic acid (Sia) can be used for glycan synthesis after activation onto the nucleotide donor CMP-Sia. Transfer of Sia from CMP-Sias onto newly synthesized glycoconjugates (e.g., glycoproteins) in the Golgi system of eukaryotes is catalyzed by a family of linkage-specific sialyl-transferases (STs). Sialic acids are typically the terminating residues of glycan (e.g., N-glycan) branches. In some aspects, sialic acids can occupy internal positions within glycans, most commonly when one sialic acid residue is attached to another. For a review of sialylation and sialic acid, see, e.g., Chapter 15 of Varki et al., Essentials of Glycobiology, 3rd Edition, Cold Spring Harbor Laboratory Press, 2015-2017.

The term "sialic acid content" refers to the level or amount of sialylation of a glycosylated protein (e.g., an IL-22 Fc fusion protein) or a portion of a protein of interest. In some aspects, an IL-22 Fc fusion protein has a sialic acid content of from about 4 to about 16 moles (e.g., about 4, about 5, about 6, about 7, about 8, about 9, about 10, about 11, about 12, about 13, about 14, about 15, or about 16 moles) of sialic acid per mole of the IL-22 Fc fusion protein. In some aspects, an IL-22 Fc fusion protein has a sialic acid content of about 8, 9, 10, 11, or 12 moles of sialic acid per mole of the IL-22 Fc fusion protein.

The term "average sialic acid content" with respect to a composition containing an IL-22 Fc fusion protein (e.g., a pharmaceutical composition or a batch) refers to the total number of moles of sialic acid in the composition per mole of IL-22 Fc fusion protein in the composition. Thus, for example, such a composition may contain a heterogeneous pool of IL-22 Fc fusion proteins with individual IL-22 Fc fusion proteins within the composition having varying levels of sialylation (e.g., in the range of 0-25 moles of sialic acid per mole of IL-22 Fc fusion protein). Unless indicated otherwise, all values for sialic acid content, including average sialic acid content, described herein refer to dimeric IL-22 Fc fusion proteins.

The term "afucosylation," "afucosylated," "defucosylation," or "defucosylated" refers to the absence or removal of core-fucose from an N-glycan, e.g., an N-glycan attached to a protein or a portion of a protein (e.g., the CH2 domain of Fc).

The term "dimeric IL-22 Fc fusion protein" refers to a dimer in which each monomer comprises an IL-22 Fc fusion protein. The term "monomeric IL-22 Fc fusion protein" refers to a dimer in which one monomer comprises an IL-22 Fc fusion protein (the IL-22 Fc arm), while the other monomer comprises an Fc region without the IL-22 polypeptide (the Fc arm). Accordingly, the dimeric IL-22 Fc fusion protein is bivalent with respect to IL-22R binding, whereas the monomeric IL-22 Fc fusion protein is monovalent with respect to IL-22R binding. The heterodimerization of the monomeric IL-22 Fc fusion protein can be facilitated by methods known in the art, including without limitation, heterodimerization by the knob-into-hole technology. The structure and assembly method of the knob-into-hole technology can be found in, e.g., US5,821,333, US7,642,228, US 2011/0287009, and PCT/US2012/059810,. This technology was developed by introducing a "knob" (or a protuberance) by replacing a small amino acid residue with a large one in the CH3 domain of one Fc, and introducing a "hole" (or a cavity) in the CH3 domain of the other Fc by replacing one or more large amino acid residues with smaller ones. In certain aspects, the IL-22 Fc fusion arm comprises a knob, and the Fc only arm comprises a hole.

The preferred residues for the formation of a knob are generally naturally occurring amino acid residues and are preferably selected from arginine (R), phenylalanine (F), tyrosine (Y), and tryptophan (W). Most preferred are tryptophan and tyrosine. In one aspect, the original residue for the formation of the knob has a small side chain volume, such as alanine, asparagine, aspartic acid, glycine, serine, threonine or valine. Exemplary amino acid substitutions in the CH3 domain for forming the knob include without limitation the T366W, T366Y, or F405W substitution.

The preferred residues for the formation of a hole are usually naturally occurring amino acid residues and are preferably selected from alanine (A), serine (S), threonine (T), and valine (V). In one embodiment, the original residue for the formation of the hole has a large side chain volume, such as tyrosine, arginine, phenylalanine, or tryptophan. Exemplary amino acid substitutions in the CH3 domain for generating the hole include without limitation the T366S, L368A, F405A, Y407A, Y407T, and Y407V substitutions. In certain aspects, the knob comprises T366W substitution, and the hole comprises the T366S/L368A/Y407V substitutions. In certain particular aspects, the Fc region of the monomeric IL-22 Fc fusion protein comprises an IgG1 Fc region. In certain particular aspects, the monomeric IL-22 IgG1 Fc fusion comprises an IL-22 Fc knob arm and an Fc hole arm. In certain embodiments, the IL-22 Fc knob arm comprises a T366W substitution (SEQ ID NO:61), and the Fc hole arm comprises T366S, L368A, and Y407V (SEQ ID NO:62). In certain other aspects, the Fc region of both arms further comprises an N297G or N297A mutation. In certain aspects, the monomeric IL-22 Fc fusion protein is expressed in E. *coli* cells. It is understood that other modifications to the Fc region known in the art that facilitate heterodimerization are also contemplated and encompassed by the instant application.

"Affinity" refers to the strength of the sum total of non-covalent interactions between a single binding site of a molecule (e.g., a ligand or an antibody) and its binding partner (e.g., a receptor or an antigen). Unless indicated otherwise, as used herein, "binding affinity" refers to intrinsic binding affinity which reflects a 1:1 interaction between members of a binding pair (e.g., IL-22 Fc fusion protein and IL-22 receptor). The affinity of a molecule X for its partner Y can generally be represented by the dissociation constant (Kd). Affinity can be measured by common methods known in the art, including those described herein. Specific illustrative and exemplary aspects for measuring binding affinity are described in the following.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')$_2$, diabodies, linear antibodies, single-chain antibody molecules (e.g. scFv), and multispecific antibodies formed from antibody fragments.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG$_1$, IgG$_2$, IgG$_3$, IgG$_4$, IgA$_1$, and IgA$_2$. The heavy chain constant domains that correspond to the different classes of immunoglobulins are called $\alpha$, $\delta$, $\varepsilon$, $\gamma$, and $\mu$, respectively.

"Effector functions" or "effector activities" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation. In certain aspects, the IL-22 Fc fusion protein does not exhibit any effector function or any detectable effector function. In certain other embodiments, the IL-22 Fc fusion protein exhibits substantially reduced effector function, e.g., about 50%, 60%, 70% 80%, or 90% reduced effector function.

The terms "full length antibody," "intact antibody," and "whole antibody" are used herein interchangeably to refer to an antibody having a structure substantially similar to a native antibody structure or having heavy chains that contain an Fc region as defined herein.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. The transformed cell includes transiently or stably transformed cell. Progeny may not be completely identical in nucleic acid content to a parent cell, but may contain mutations. Mutant progeny that have the same function or biological activity as screened or selected for in the originally transformed cell are included herein. In certain aspects, the host cell is transiently transfected with the exogenous nucleic acid. In certain other aspects, the host cell is stably transfected with the exogenous nucleic acid.

An "immunoconjugate" is an antibody or a fragment of an antibody conjugated to one or more heterologous molecule(s), including but not limited to a cytotoxic agent.

An "isolated" IL-22 Fc fusion protein is one which has been separated from the environment of a host cell that recombinantly produces the fusion protein. In some embodiments, an IL-22 Fc fusion protein is purified to greater than 80%, 85%, 90%, 95%, 96%, 97%, 98%, or 99% purity as determined by, for example, electrophoretic (e.g., SDS-PAGE, isoelectric focusing (IEF), capillary electrophoresis) or chromatographic (e.g., ion exchange or reverse phase HPLC) approaches.

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

The term "isolated nucleic acid encoding an IL-22 Fc fusion protein" refers to one or more nucleic acid molecules encoding an IL-22 Fc fusion protein, including such nucleic acid molecule(s) in a single vector or separate vectors, such nucleic acid molecule(s) transiently or stably transfected into a host cell, and such nucleic acid molecule(s) present at one or more locations in a host cell.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N-to C-terminus, each heavy chain has a variable region (VH), also called a variable heavy domain or a heavy chain variable domain, followed by three constant domains (CH1, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa (κ) and lambda (λ), based on the amino acid sequence of its constant domain.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007)). A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

A "native sequence Fc region" comprises an amino acid sequence identical to the amino acid sequence of an Fc region found in nature. Native sequence human Fc regions include, without limitation, a native sequence human IgG1 Fc region (non-A and A allotypes); native sequence human IgG2 Fc region; native sequence human IgG3 Fc region; and native sequence human IgG4 Fc region, as well as naturally occurring variants thereof.

A "variant Fc region" comprises an amino acid sequence which differs from that of a native sequence Fc region by virtue of at least one amino acid modification, preferably one or more amino acid substitution(s). Preferably, the variant Fc region has at least one amino acid substitution compared to a native sequence Fc region or to the Fc region of a parent polypeptide, e.g., from about one to about ten amino acid substitutions, and preferably from about one to about five amino acid substitutions in a native sequence Fc region or in the Fc region of the parent polypeptide. The variant Fc region herein will preferably possess at least about 80% homology with a native sequence Fc region and/or with an Fc region of a parent polypeptide, and most preferably at least about 90% homology therewith, more preferably at least about 95% homology therewith. In certain aspects, the variant Fc region is not glycosylated.

The term "package insert" is used to refer to instructions customarily included in commercial packages of therapeutic products, that contain information about the indications, usage, dosage, administration, combination therapy, contraindications, and/or warnings concerning the use of such therapeutic products.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

Below are examples of how to calculate the % amino acid sequence identity of the amino acid sequence designated "Comparison Protein" or "Reference Protein" to the amino acid sequence designated "IL-22," wherein "IL-22" represents the amino acid sequence of an IL-22 polypeptide of interest, "Comparison Protein" represents the amino acid sequence of a polypeptide against which the "IL-22 " polypeptide of interest is being compared, and "X," "Y," and "Z" each represent different amino acid residues.

| IL-22 | XXXXXXXXXXXXXXX | (Length = 15 amino acids) |
| Reference Protein | XXXXXYYYYYYY | (Length = 12 amino acids) |

% amino acid sequence identity =

(the number of identically matching amino acid residues between the two polypeptide sequences)

divided by (the total number of amino acid residues of the IL-22 polypeptide) =

5 divided by 15 = 33.3%

| IL-22 | XXXXXXXXXX | (Length = 10 amino acids) |
| Reference Protein | XXXXXYYYYYYZZYZ | (Length = 15 amino acids) |

% amino acid sequence identity =

(the number of identically matching amino acid residues between the two polypeptide sequences)

divided by (the total number of amino acid residues of the IL-22 polypeptide) =

5 divided by 10 = 50%

The term "agonist" is used in the broadest sense and includes any molecule that partially or fully mimics a biological activity of an IL-22 polypeptide. Also encompassed by "agonist" are molecules that stimulate the transcription or translation of mRNA encoding the polypeptide.

Suitable agonist molecules include, e.g., agonist antibodies or antibody fragments; a native polypeptide; fragments or amino acid sequence variants of a native polypeptide; peptides; antisense oligonucleotides; small organic molecules; and nucleic acids that encode polypeptides agonists or antibodies. Reference to "an" agonist encompasses a single agonist or a combination of two or more different agonists.

The term "IL-22 agonist" is used in the broadest sense, and includes any molecule that mimics a qualitative biological activity (as hereinabove defined) of a native sequence IL-22 polypeptide. IL-22 agonists specifically include IL-22-Fc or IL-22 Ig polypeptides (immunoadhesins), but also small molecules mimicking at least one IL-22 biological activity. Preferably, the biological activity is binding of the IL-22 receptor, interacting with IL-22BP, or facilitating an innate immune response pathway.

IL-22R1 pairs with other proteins to form heterodimers as the receptors for certain IL-10 family members. See Ouyang et al., 2011, *supra.* Thus, in certain embodiments, IL-22 agonists may include an IL-22 receptor agonist, including a cytokine (or a fusion protein or agonist thereof) that binds to and triggers downstream signaling of the IL-22R1. In certain aspects, the IL-22 agonists include an IL-22R1 agonist, including without limitation an anti-IL-22R1 agonist antibody; an IL-20 agonist, including without limitation IL-20 polypeptide or IL-20 Fc fusion protein; and an IL-24 agonist, including without limitation IL-24 polypeptide or IL-24 fusion protein. In certain other aspects, the IL-22R1 agonists include an IL-19 agonist, including without limitation IL-19 polypeptide or IL-19 Fc fusion protein; and an IL-26 agonist, including without limitation IL-26 polypeptide or IL-26 Fc fusion protein. Exemplary sequences for IL-19 (GenBank Accession No. AAG16755.1, SEQ ID NO:77), IL-20 (GenBank Accession No. AAH69311.1, SEQ ID NO:78), IL-24 (GenBank Accession No. AAH09681.1, SEQ ID NO:79) and IL-26 (GenBank Accession No. NP_060872.1, SEQ ID NO:80) are provided herein. In certain aspects, an IL-19 polypeptide comprises the amino acid sequence of SEQ ID NO:77 or the mature protein without the signal peptide. In certain other aspects, an IL-20 polypeptide comprises the amino acid sequence of SEQ ID NO:78 or the mature protein without the signal peptide. In yet other aspects, an IL-24 polypeptide comprises the amino acid sequence of SEQ ID NO:79 or the mature protein without the signal peptide. In certain other aspects, an IL-26 polypeptide comprises the amino acid sequence of SEQ ID NO:80 or the mature protein without the signal peptide.

A "small molecule" is defined herein to have a molecular weight below about 600, preferably below about 1000 daltons.

An "agonist antibody," as used herein, is an antibody which partially or fully mimics a biological activity of an IL-22 polypeptide.

The terms "pharmaceutical formulation" or "pharmaceutical composition" are used interchangeably herein and refer to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, diluent, stabilizer, or preservative.

As used herein, "biological activity" of protein (e.g., an IL-22 Fc fusion protein) refers to the ability of the protein (e.g., an IL-22 Fc fusion protein) to bind its target, for example, the ability of an IL-22 Fc fusion protein to bind an IL-22 receptor. It can further include a biological response which can be measured in vitro or in vivo. Such activity may be antagonistic or agonistic. In particular aspects, the activity is agonistic (e.g., receptor activation).

A "disorder," a "disease," or a "condition," as used interchangeably herein, is any condition that would benefit from treatment by a method described herein (e.g., a method that includes administering an IL-22 Fc fusion protein to the subject) or by a compound described herein (e.g., an IL-22 Fc fusion protein or a composition thereof (e.g., a pharmaceutical composition). This includes chronic and acute disorders or diseases including those pathological conditions which predispose the mammal to the disorder in question. In some aspects, the disorder is an IL-22 associated disorder. Exemplary disorders include graft versus host disease (GVHD) (e.g., acute or chronic GVHD, including, but not limited to, intestinal GVHD).

The terms "intestine" or "gut" as used interchangeably herein broadly encompasses the small intestine and large intestine.

The terms "graft versus host disease" and "GVHD," as used interchangeably herein, refer to a complication of allogeneic stem cell transplantation. In GVHD, donor hematopoietic stem cells recognize the transplant recipient as foreign and attack the patient's tissues and organs, which can impair the tissue or organ's function or cause it to fail. As used herein, GVHD includes, for example, acute GVHD or chronic GVHD. In particular aspects, the GVHD is acute GVHD. Further, non-limiting examples include intestinal GVHD (e.g., acute intestinal GVHD).

Acute GVHD is a disorder caused by donor immune cells in patients who have had an allogeneic marrow or blood cell transplantation. Acute GVHD occurs as a result of host (recipient) tissue damage caused by the underlying hematologic disease and associated treatments, infection, and the conditioning regimen. Damaged host tissues release signals, including pro-inflammatory cytokines, which activate host antigen-presenting cells (APCs). Conditioning regimen-mediated damage to the gastrointestinal (GI) tract allows translocation of microbes and microbial products that amplifies host APC activation and causes infection. Host APCs activate donor T cells that destroy healthy host tissue resulting in aGVHD. Risk factors for developing aGVHD include the degree of human leukocyte antigen (HLA) mismatch, the relatedness of the donor, the intensity of conditioning regimen, the source of graft, and the aGVHD prophylactic regimen used. The most common tissues affected by aGVHD are the skin, liver, and GI tract. Lower GI aGVHD is typically the most difficult to treat and is the greatest cause of GVHD-related morbidity and mortality. Signs and symptoms associated with aGVHD include but are not limited to rash, dermatitis, hepatitis, jaundice, abdominal pain, and diarrhea. Diagnosis of aGVHD depends on the clinical, laboratory, and pathologic assessment of target organs. The Mount Sinai Acute GVHD International Consortium (MAGIC) (Harris et al. Biol. Blood Marrow Transplant. 22:4-10, 2016, which is incorporated by reference herein in its entirety) provides a standardized approach to aGVHD clinical staging criteria and grading based on the Glucksberg scale (Glucksberg et al. Transplantation 18:295-304, 1974). Severity is categorized as Grade I-IV depending on the degree of skin, GI and/or liver involvement, with Grade IV representing the most severe disease. The term acute GVHD as used herein encompasses any stage or grade of acute GVHD affecting any organ (e.g., skin, liver, and/or GI tract). In some embodiments, acute GVHD includes classic acute GVHD (which may be characterized, for example, by maculopapular rash, nausea, vomiting, anorexia, diarrhea, ileus, and/or cholestatic hepatitis occurring within 100 days after allo-HSCT or donor lymphocyte infusions (DLI)) and persistent, recurrent, or late-onset acute GVHD (which may be characterized, for example, by features of classic acute GVHD without diagnostic or distinctive manifestations of chronic GVHD occurring beyond 100 days of allo-HSCT or DLI). See, e.g., Table 2 of Filipovich et al. Biol. Blood. Marrow Transplant. 11:945-955, 2005 and Jagasia et al. Biol. Blood. Marrow Transplant. 21(3):389-401, 2015.

Chronic GVHD usually begins later after allogeneic marrow or blood cell transplant and lasts longer than acute GVHD. Symptoms of chronic GVHD usually present within 3 years of allo-HSCT, and are often preceded by a history of acute GVHD. Manifestations of chronic GVHD may be widespread or restricted to a single organ. Chronic GVHD can lead to severe consequences, including but not limited to joint contractures, blindness, end-stage lung disease, or death. Historically, chronic GVHD was defined as any manifestation of GVHD that was present (or continued) at 100 days after allo-HSCT, even if the clinical manifestation was indistinguishable from that of acute GVHD. However, advances in GVHD treatment have altered the presentation of both acute and chronic GVHD. For example, acute GVHD may present beyond 3 months in patients who have received reduced-intensity conditioning, while manifestations of both acute GVHD and chronic GVHD can be present simultaneously, e.g., in patients treated with DLI. In some embodiments, chronic GVHD may be defined as described in Filipovich et al. *supra,* in which the diagnosis of chronic GVHD requires (i) distinction from acute GVHD, (ii) presence of at least one diagnostic clinical sign of chronic GVHD or presence of at least 1 distinctive manifestation confirmed by biopsy or other relevant tests, and (iii) exclusion of other possible diagnoses. Diagnostic clinical signs of chronic GVHD include those listed in Table 1 of Filipovich et al. *supra,* including poikiloderma, lichen planus-like features of the skin or genitalia, sclerotic features of the skin, morphea-like features of the skin, lichen sclerosis-like features of the skin, lichen-type features of the mouth, hyperkeratotic plaques of the mouth, restriction of the mouth opening from sclerosis, vaginal scarring or stenosis, esophageal web of the GI tract, strictures or stenosis in the upper to mid third of the esophagus, bronchiolitis obliterans, fasciitis, or joint stiffness or contractures secondary to sclerosis. Chronic GVHD may include classic chronic GVHD without features characteristic of acute GVHD and an overlap syndrome that includes the presence of acute GVHD features and chronic GVHD features. See Table 2 of Filipovich et al. *supra.* In other embodiments, chronic GVHD may be diagnosed as described in Jagasia et al. Biol. Blood. Marrow Transplant. 21(3):389-401, 2015 (see, e.g., Table 1), which differs from the Filipovich et al. criteria, *inter alia,* by removal of hyperkeratotic plaques of the mouth as a diagnostic feature, addition of phimosis or urethral/meatus scarring or stenosis as a diagnostic feature for males, and modification of the diagnostic features for the female genitalia to include vaginal scarring or clitoral/labial agglutination.

The terms "allogeneic hematopoietic stem cell transplantation" and "allo-HSCT" are used interchangeably to refer to transplantation of multipotent hematopoietic stem cells from a donor to a recipient. The hematopoietic stem cells may be, for example, peripheral blood stem cells, bone marrow stem cells, amniotic fluid stem cells, or umbilical cord blood stem cells. In some particular aspects, the stem cells are peripheral blood stem cells or bone marrow stem cells. A conditioning regimen, such as chemotherapy, irradiation, or a combination thereof is typically administered to a subject prior to allo-HSCT. For example, a "myeloablative" conditioning regimen destroys the recipient's bone marrow cells, and is typically performed using a combination of cyclophosphamide and total body irradiation. In other examples, non-myeloablative conditioning approaches which use doses of chemotherapy and/or radiation that are too low to eradicate all of the recipient's bone marrow cells may be used.

The allo-HSCT may be "HLA-matched related HSCT," which refers to allo-HSCT in which the donor is related to the recipient (usually a closely related family member such as a sibling) and in which eight out of eight of the donor's alleles at the *HLA-A, HLA-B, HLA-C,* and *HLA-DR81* loci match those of the recipient. In other examples, the allo-HSCT may be "HLA-matched unrelated HSCT," which refers to allo-HSCT in which the donor is unrelated to the recipient and in which eight out of eight of the donor's alleles at the *HLA-A, HLA-B, HLA-C,* and *HLA-DR81* loci match those of the recipient. In still other examples, the allo-HSCT may be "single-antigen HLA-mismatched unrelated HSCT," which refers to allo-HSCT in which the donor is unrelated to the recipient and in which seven out of eight of the donor's alleles at the *HLA-A, HLA-B, HLA-C,* and *HLA-DR81* loci match those of the recipient.

As used herein, the term "preventing acute GVHD" means that a prophylactic GVHD therapy (e.g., a method or composition for use provided herein) prevents the occurrence of Grade II-IV acute GVHD. The grade of acute GVHD can be assessed by any suitable method known in the art, such as but not limited to MAGIC (Harris et al. *supra*).

As used herein, the term "reducing the risk of developing chronic GVHD" means that a prophylactic GVHD (e.g., acute GVHD) therapy (e.g., a method or composition for use provided herein) reduces the likelihood of a subject developing chronic GVHD, e.g., characterized by active alloimmunity requiring systemic immunosuppression to improve symptoms and prevent ongoing organ damage, or as assessed by the National Institutes of Health (NIH) Chronic GVHD Diagnosis and Staging score (Jagasia et al. Biol. Blood Marrow Transplant. 21:389-401, 2015), as compared to a reference therapy (e.g., a therapy that does not include an IL-22 Fc fusion protein, e.g., an immunosuppressive agent).

As used herein "reducing the risk of corticosteroid-refractory acute GVHD" means that a prophylactic GVHD therapy (e.g., a method or composition for use provided herein) reduces the likelihood of a subject developing corticosteroid-refractory acute GVHD, as compared to a reference therapy (e.g., an immunosuppressive agent). By way of non-limiting example, corticosteroid-refractory acute GVHD may be diagnosed as described in Example 1 herein, (e.g., progression of aGVHD after 3 days of therapy with greater than or equal to 2 mg/kg/day of prednisone or equivalent; no improvement of aGVHD after 7 days of therapy with greater than or equal to 2 mg/kg/day of prednisone or equivalent; and/or skin and visceral organ-involved aGVHD and improvement in skin only after 7 days of therapy with greater than or equal to 2 mg/kg/day of prednisone or equivalent), however it is understood that other criteria may also be used to diagnose corticosteroid-refractory acute GVHD according to known standards.

The term "prior to allo-HSCT" means before a subject receives the transplant. Thus for example, one or more agents that are administered prior to allo-HSCT may be administered, for example, hours, days, weeks, or months prior to the allo-HSCT procedure.

The term "concurrently with allo-HSCT" means at the same time as a subject is undergoing an allo-HSCT procedure. For example, during an allo-HSCT procedure, a subject may be administered an IL-22 Fc fusion protein alone or in combination with one or more additional GVHD therapies (e.g., an immunosuppressive agent or other physician-selected therapy, e.g., a standard of care therapy).

The term "after allo-HSCT" any time after the transplant. Thus, for example, one or more agents may be administered, for example, hours, days, weeks, or months after the allo-HSCT procedure.

By "reduce or inhibit" is meant the ability to cause an overall decrease, preferably of 20% or greater, more preferably of 50% or greater, and most preferably of 75%, 85%, 90%, 95%, or greater. Reduce or inhibit can refer to the symptoms of the disorder being treated, e.g., the presence or amount of inflammation or ulcers.

A "subject," "individual," or "patient" is a mammal. Mammals include, but are not limited to, domesticated animals (e.g., cats, dogs, cows, sheep, and horses), primates (e.g., humans and non-human primates such as monkeys), rabbits, and rodents (e.g., mice and rats). In certain aspects, the individual, subject or patient is a human. In certain aspects, the human patient is at risk of developing GVHD (e.g., acute GVHD, or chronic GVHD)..

An "effective amount" or "therapeutically effective amount" of an agent, e.g., a pharmaceutical formulation, refers to an amount effective, at dosages and for periods of time necessary, to achieve the desired therapeutic or prophylactic result.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease (e.g., preventing GVHD (e.g., acute or chronic GVHD, including intestinal GVHD)), alleviation of symptoms, diminishment of any direct or indirect pathological consequences of the disease, preventing metastasis, decreasing the rate of disease progression, amelioration or palliation of the disease state, and remission or improved prognosis.

The "pathology" of a disease or condition includes all phenomena that compromise the well-being of the subject.

"Amelioration," "ameliorating," "alleviation," "alleviating," or equivalents thereof, refers to both therapeutic treatment and prophylactic or preventative measures, wherein the object is to ameliorate, prevent, slow down (lessen), decrease or inhibit a disease or condition, e.g., GVHD (e.g., acute or chronic GVHD, including intestinal GVHD). Those in need of treatment include those already with the disease or condition as well as those prone to having the disease or condition or those in whom the disease or condition is to be prevented.

As used herein, "administering" is meant a method of giving a dosage of a compound (e.g., an IL-22 Fc fusion protein (e.g., as described herein, e.g., an IL-22 Fc fusion protein comprising the amino acid sequence set forth in SEQ ID NO: 8 or 10)) or a composition (e.g., a pharmaceutical composition, e.g., a pharmaceutical composition including an IL-22 Fc fusion protein, optionally also including an additional therapeutic agent) to a subject. The compositions utilized in the methods described herein can be administered, for example, intravitreally, intramuscularly, intravenously, intradermally, percutaneously, intraarterially, intraperitoneally, intralesionally, intracranially, intraarticularly, intraprostatically, intrapleurally, intratracheally, intrathecally, intranasally, intravaginally, intrarectally, topically, intratumorally, peritoneally, subcutaneously, subconjunctivally, intravesicularly, mucosally, intrapericardially, intraumbilically, intraocularly, intraorbitally, orally, topically, transdermally, periocularly, conjunctivally, subtenonly, intracamerally, subretinally, retrobulbarly, intracanalicularly, by inhalation, by injection, by implantation, by infusion, by continuous infusion, by localized perfusion bathing target cells directly, by catheter, by lavage, in cremes, or in lipid compositions. The compositions utilized in the methods described herein can also be administered systemically or locally. The method of administration can vary depending on various factors (e.g., the compound or composition being administered and the severity of the condition, disease, or disorder being treated).

The term "$C_{max}$" refers to the maximum concentration that a therapeutic agent (e.g., an IL-22 Fc fusion protein) achieves in a compartment or test area of a subject's body after administration of a single dose of the therapeutic agent. In some embodiments, $C_{max}$ is assessed in serum.

The term "area under the curve (AUC)" refers to a measurement indicating exposure to a therapeutic agent (e.g., an IL-22 Fc fusion protein) over a period of time. For example, the AUC may be the definite integral of a curve that describes the variation in the concentration of a therapeutic agent over time. In other examples, the AUC may be an estimated value based on measurements at discrete time points, e.g., using non-compartmental analysis (NCA) technique using the trapezoidal rule. For example, $AUC_{0-\infty}$ indicates the total drug exposure across time, whereas $AUC_{0-14}$ indicates the total drug exposure from Day 0 (dosing day) to Day 14. In some aspects, AUC (e.g., $AUC_{0-14}$) is assessed in serum.

Within this application, unless otherwise stated, the techniques utilized may be found in any of several well-known references such as: Molecular Cloning: A Laboratory Manual (Sambrook, et al., 1989, Cold Spring Harbor Laboratory Press), PCR Protocols: A Guide to Methods and Applications (Innis, et al. 1990. Academic Press, San Diego, CA), and Harlow and Lane (1988) Antibodies: A Laboratory Manual ch.14 (Cold Spring Harbor Laboratory, Cold Spring Harbor, NY).

As appropriate, procedures involving the use of commercially available kits and reagents are generally carried out in accordance with manufacturer defined protocols and/or parameters unless otherwise noted.

## II. PREVENTING AND TREATING GVHD

Provided herein IL-22 Fc fusion proteins for use in preventing and treating GVHD (e.g., aGVHD (e.g., corticosteroid-refractory aGVHD) and/or cGVHD) in a subject that include administering to a subject in need thereof an IL-22 Fc fusion protein (e.g., as described herein, e.g., an IL-22 Fc fusion protein comprising the amino acid sequence set forth in SEQ ID NO: 8 or 10).

**A. Dosing Regimens and Administration**

The methods and uses described herein include administering an IL-22 Fc fusion protein (e.g., as described herein, e.g., an IL-22 Fc fusion protein comprising the amino acid sequence set forth in SEQ ID NO: 8) to a subject at risk for or having GVHD, thereby preventing or treating the GVHD. GVHD is the most frequent and potentially fatal complication of an allogeneic hematopoietic progenitor cell transplantation (allo-HSCT). It appears when immunocompetent T cells from donor origin recognize antigens from recipient origin as foreign. The immune response activates donor T cells and destroys recipient tissues. The clinical picture of this immune response is called acute and chronic GVHD. Acute GVHD (aGVHD) is the main fatal complication during the first months after allogeneic hematopoietic progenitor cell transplantation, while chronic GVHD (cGVHD) accounts for a significant long-term fraction of the mortality, morbidity, and reduced quality of life of patients.

aGVHD is a common and life-threatening complication of allo-HSCT with a high unmet need for effective, non-immunosuppressive therapies for prevention and treatment. aGVHD can be summarized in three stages: initial tissue damage (including the gastrointestinal (GI) tract) from the conditioning regimen (myeloablative versus non-myeloablative) that activates the host antigen-presenting cells (APCs), followed by APCs activating donor T cells that finally lead to destruction of host tissue, including the skin, GI tract and liver. Risk factors for developing aGVHD include degree of human leukocyte antigen (HLA) mismatch between donor and recipient, relatedness of donor and recipient, female donor-male recipient, use of peripheral blood stem cell grafts, and intensity of conditioning regimen.

Diagnosis of aGVHD depends on the clinical, laboratory and biopsy assessment of target organs. aGVHD severity can be categorized as Grade I-IV based on the Glucksberg scale (Glucksberg H, et al. Transplantation 1974;18(4):295-304), depending on the degree of skin, GI, and/or liver involvement, with Grade IV representing the most severe disease. The Mount Sinai Acute GVHD International Consortium (MAGIC) updated the clinical staging criteria of aGVHD to allow a more standardized approach to aGVHD grading (Harris et al. Biol Blood Marrow Transplant 2016;22:4-10).

Signs and symptoms associated with aGVHD include rash, dermatitis, hepatitis, jaundice, abdominal pain, and diarrhea. aGVHD most commonly involves the skin and GI tract, with the skin being the most frequent and usually the earliest clinical manifestation. aGVHD with GI involvement (GI or intestinal aGVHD) is the most difficult to treat and associated with the highest rates of GVHD-related morbidity and mortality.

Prevention is an integral component to the management of patients undergoing allo-HSCT. To date, no pharmacologic therapies have been approved for the prevention of aGVHD. Although there is no universal prophylaxis regimen for aGVHD, the majority of centers use a combination of a calcineurin inhibitor (e.g., cyclosporine or tacrolimus) and methotrexate. Additional prophylaxis agents include sirolimus, mycophenolate mofetil (MMF), anti-thymocyte thymoglobulin (ATG), and post-transplant cyclophosphamide. Due to their immunosuppressive nature, current prophylaxis agents can cause increased serious infections, delayed hematologic recovery, and reduce graft versus tumor effects leading to an increased rate of relapse. Despite the use of prophylaxis, Grade II-IV aGVHD develops in approximately 35%-50% of patients after allo-HSCT, with approximately 15% developing severe aGVHD (Grades III-IV). Subsequently, approximately 59%-85% of patients who develop Grade II-IV aGVHD will develop chronic GVHD. Thus, there is a significant unmet medical need for non-immunosuppressive effective therapies to prevent aGVHD and the significant long-term morbidity and mortality associated with the disease in patients undergoing allo-HSCT.

Novel treatment modalities are also needed for cGVHD. Patients who have an increased risk of developing cGVHD are those who have received stem cells/bone marrow from an HLA (human leukocyte antigen) mismatched related donor or from an HLA matched unrelated donor, patients that may have already experienced acute GVHD, and older recipients. Chronic GVHD can appear at any time after allogenic transplant or several years after the transplant. Chronic GVHD can occur in the skin, liver, eyes, mouth, lungs, gastrointestinal tract, neuromuscular system, or genitourinary tract.

Chronic GVHD presents with the following key clinical manifestations: mucocutaneous, myofascial, pulmonary, and "other," affecting essentially any organ system in the body. Characteristic features may include chronic inflammatory changes that can be relatively acellular involving ocular, oral, esophageal, skin, joint and fascial, and genital tissues. Progression to clinically significant fibrosis involving multiple organs in the integumentary, musculoskeletal, aerodigestive, gastrointestinal, cardiorespiratory, reproductive, and peripheral nervous systems occurs in severely affected individuals. Rare but severe clinical presentations of chronic GVHD also can include polyserositis (with pericardial and pleural effusions) or polymyositis with severe muscle weakness and elevated muscle enzyme levels. For a review of the signs and symptoms of cGVHD, as well as current treatment modalities, see Cooke et al. Biol. Blood Marrow Transplant 23 (2017) 211-234.

Thus, the disclosure provides methods, dosing regimens, and dosing cycles for preventing or treating GVHD in a subject. For example, any of the methods, dosing regimens, and/or dosing cycles described above or herein can be used in a method of preventing or treating GVHD. In some aspects, the GVHD is acute GVHD or chronic GVHD. In particular aspects, the GVHD is acute GVHD. In some aspects, the GVHD is intestinal GVHD. In other aspects, the GVHD is skin GVHD or liver GVHD. Such methods can provide a prophylactic effect against the development of, or a therapeutic effect against the progression of, clinical and/or histological and/or biochemical and/or pathological indicia (including both symptoms and signs) of GVHD. Administration of an IL-22 Fc fusion protein (e.g., as described herein, e.g., an IL-22 Fc fusion protein comprising the amino acid sequence set forth in SEQ ID NO: 8 or 10) or composition thereof as described herein may reduce one or more symptoms of GVHD, including pain, rashes, skin thickness, yellow skin or eyes, mouth dryness or ulcers, taste abnormalities, dry eyes, infections, or weight loss.

For example, provided herein is an IL-22 Fc fusion protein for use in a method of preventing acute GVHD, reducing the risk of developing chronic GVHD, or reducing the risk of corticosteroid-refractory acute GVHD in a subject comprising administering to a subject in need thereof an IL-22 Fc fusion protein (e.g., as described herein, e.g., an IL-22 Fc fusion protein comprising the amino acid sequence set forth in SEQ ID NO: 8) in a dosing regimen comprising a dosing cycle, wherein the dosing cycle comprises one total dose of the IL-22 Fc fusion protein that is administered to the subject concurrently with or after allogeneic hematopoietic stem cell transplantation (allo HSCT). In some aspects, the IL-22 Fc fusion protein is administered to the subject concurrently with allo-HSCT. In other aspects, the IL-22 Fc fusion protein is administered to the subject after allo-HSCT.

In one example, provided herein is an IL-22 Fc fusion protein for use in preventing acute GVHD in a subject, wherein the IL-22 Fc fusion protein is for administration to a subject in need thereof in a dosing regimen comprising a dosing cycle, wherein the dosing cycle comprises one total dose of the IL-22 Fc fusion protein that is administered to the subject concurrently with or after allo-HSCT.

In a further example, provided herein is an IL-22 Fc fusion protein for use in reducing the risk of developing chronic GVHD in a subject, wherein the IL-22 Fc fusion protein is for administration to a subject in need thereof in a dosing regimen comprising a dosing cycle, wherein the dosing cycle comprises one total dose of the IL-22 Fc fusion protein that is administered to the subject concurrently with or after allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises one total dose of the IL-22 Fc fusion protein that is administered to the subject concurrently with or after allo-HSCT.

In another example, provided herein is an IL-22 Fc fusion protein for use in preventing acute GVHD in a subject, wherein the IL-22 Fc fusion protein is for administration to a subject in need thereof in a dosing regimen comprising a dosing cycle, wherein the dosing cycle comprises a first dose (C1 D1) of the IL-22 Fc fusion protein that is administered to the subject concurrently with or after allo-HSCT, and one or more further doses.

In a further example, provided herein is an IL-22 Fc fusion protein for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1 D1) of the IL-22 Fc fusion protein that is administered to the subject concurrently with or after allo-HSCT, and one or more further doses.

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1 D1) of the IL-22 Fc fusion protein that is administered to the subject concurrently with or after allo-HSCT, and one or more further doses.

In some aspects, each dose in the dosing cycle is equal. In other aspects, the doses may be unequal.

In another example, provided herein is an IL-22 Fc fusion protein for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1) and one or more further doses of the IL-22 Fc fusion protein, wherein the dosing cycle results in a $C_{max}$ of the IL-22 Fc fusion protein of about 2350 ng/mL or lower (e.g., about 2350 ng/mL or lower, about 2300 ng/mL or lower, about 2250 ng/mL or lower, about 2200 ng/mL or lower, about 2150 ng/mL or lower, about 2100 ng/mL or lower, about 2050 ng/mL or lower, about 2000 ng/mL or lower, about 1950 ng/mL or lower, about 1900 ng/mL or lower, about 1850 ng/mL or lower, about 1800 ng/mL or lower, about 1750 ng/mL or lower, about 1700 ng/mL or lower, about 1650 ng/mL or lower, about 1600 ng/mL or lower, about 1550 ng/mL or lower, about 1500 ng/mL or lower, about 1450 ng/mL or lower, about 1400 ng/mL or lower, about 1350 ng/mL or lower, about 1300 ng/mL or lower, about 1250 ng/mL or lower, about 1200 ng/mL or lower, about 1150 ng/mL or lower, about 1100 ng/mL or lower, about 1050 ng/mL or lower, about 1000 ng/mL or lower, abour 950 ng/mL or lower, or about 900 ng/mL or lower) and/or an $AUC_{0-14}$ of about 5600 ng•day/mL or lower (e.g., about 5600 ng•day/mL or lower, about 5500 ng•day/mL or lower, about 5450 ng•day/mL or lower, about 5400 ng•day/mL or lower, about 5350 ng•day/mL or lower, about 5300 ng•day/mL or lower, about 5250 ng•day/mL or lower, about 5200 ng•day/mL or lower, about 5150 ng•day/mL or lower, about 5100 ng•day/mL or lower, about 5050 ng•day/mL or lower, about 5000 ng•day/mL or lower, about 4950 ng•day/mL or lower, about 4900 ng•day/mL or lower, about 4850 ng•day/mL or lower, about 4800 ng•day/mL or lower, about 4750 ng•day/mL or lower, about 4700 ng•day/mL or lower, about 4650 ng•day/mL or lower, about 4600 ng•day/mL or lower, about 4550 ng•day/mL or lower, about 4500 ng•day/mL or lower, about 4450 ng•day/mL or lower, about 4400 ng•day/mL or lower, about 4350 ng•day/mL or lower, about 4300 ng•day/mL or lower, about 4250 ng•day/mL or lower, about 4200 ng•day/mL or lower, about 4150 ng•day/mL or lower, about 4100 ng•day/mL or lower, about 4050 ng•day/mL or lower, about 4000 ng•day/mL or lower, about 3950 ng•day/mL or lower, about 3900 ng•day/mL or lower, about 3850 ng•day/mL or lower, about 3800 ng•day/mL or lower, about 3750 ng•day/mL or lower, about 3700 ng•day/mL or lower, about 3650 ng•day/mL or lower, about 3600 ng•day/mL or lower, about 3550 ng•day/mL or lower, about 3500 ng•day/mL or lower, about 3450 ng•day/mL or lower, about 3400 ng•day/mL or lower, about 3350 ng•day/mL or lower, about 3300 ng•day/mL or lower, about 3250 ng•day/mL or lower, about 3200 ng•day/mL or lower, about 3100 ng•day/mL or lower, about 3050 ng•day/mL or lower, about 3000 ng•day/mL or lower, about 2950 ng•day/mL or lower, about 2900 ng•day/mL or lower, about 2850 ng•day/mL or lower, about 2800 ng•day/mL or lower, about 2750 ng•day/mL or lower, about 2700 ng•day/mL or lower, about 2650 ng•day/mL or lower, about 2600 ng•day/mL or lower, about 2550 ng•day/mL or lower, about 2500 ng•day/mL or lower, about 2450 ng•day/mL or lower, about 2400 ng•day/mL or lower, about 2350 ng•day/mL or lower, about 2300 ng•day/mL or lower, about 2250 ng•day/mL or lower, or about 2200 ng•day/mL or lower).

In some aspects of the disclosure, the dosing cycle results in a $C_{max}$ of the IL-22 Fc fusion protein of about 900 ng/mL to about 2350 ng/mL, about 950 ng/mL to about 2350 ng/mL, about 1000 ng/mL to about 2350 ng/mL, about 1050 ng/mL to about 2350 ng/mL, about 1100 ng/mL to about 2350 ng/mL, about 1150 ng/mL to about 2350 ng/mL, about 1200 ng/mL to about 2350 ng/mL, about 1250 ng/mL to about 2350 ng/mL, about 1300 ng/mL to about 2350 ng/mL, about 1350 ng/mL to about 2350 ng/mL, about 1400 ng/mL to about 2350 ng/mL, about 1450 ng/mL to about 2350 ng/mL, about 1500 ng/mL to about 2350 ng/mL, about 1550 ng/mL to about 2350 ng/mL, about 1600 ng/mL to about 2350 ng/mL, about 1650 ng/mL to about 2350 ng/mL, about 1700 ng/mL to about 2350 ng/mL, about 1750 ng/mL to about 2350 ng/mL, about 1800 ng/mL to about 2350 ng/mL, about 900 ng/mL to about 2300 ng/mL, about 950 ng/mL to about 2300 ng/mL, about 1000 ng/mL to about 2300 ng/mL, about 1050 ng/mL to about 2300 ng/mL, about 1100 ng/mL to about 2300 ng/mL, about 1150 ng/mL to about 2300 ng/mL, about 1200 ng/mL to about 2300 ng/mL, about 1250 ng/mL to about 2300 ng/mL, about 1300 ng/mL to about 2300 ng/mL, about 1350 ng/mL to about 2300 ng/mL, about 1400 ng/mL to about 2300 ng/mL, about 1450 ng/mL to about 2300 ng/mL, about 1500 ng/mL to about 2300 ng/mL, about 1550 ng/mL to about 2300 ng/mL, about 1600 ng/mL to about 2300 ng/mL, about 1650 ng/mL to about 2300 ng/mL, about 1700 ng/mL to about 2300 ng/mL, about 1750 ng/mL to about 2300 ng/mL, about 900 ng/mL to about 2250 ng/mL, about 950 ng/mL to about 2250 ng/mL, about 1000 ng/mL to about 2250 ng/mL, about 1050 ng/mL to about 2250 ng/mL, about 1100 ng/mL to about 2250 ng/mL, about 1150 ng/mL to about 2250 ng/mL, about 1200 ng/mL to about 2250 ng/mL, about 1250 ng/mL to about 2250 ng/mL, about 1300 ng/mL to about 2250 ng/mL, about 1350 ng/mL to about 2250 ng/mL, about 1400 ng/mL to about 2250 ng/mL, about 1450 ng/mL to about 2250 ng/mL, about 1500 ng/mL to about 2250 ng/mL, about 1550 ng/mL to about 2250 ng/mL, about 1600 ng/mL to about 2250 ng/mL, about 1650 ng/mL to about 2250 ng/mL, about 1700 ng/mL to about 2250 ng/mL, about 900 ng/mL to about 2200 ng/mL, about 950 ng/mL to about 2200 ng/mL, about 1000 ng/mL to about 2200 ng/mL, about 1050 ng/mL to about 2200 ng/mL, about 1100 ng/mL to about 2200 ng/mL, about 1150 ng/mL to about 2200 ng/mL, about 1200 ng/mL to about 2200 ng/mL, about 1250 ng/mL to about 2200 ng/mL, about 1300 ng/mL to about 2200 ng/mL, about 1350 ng/mL to about 2200 ng/mL, about 1400 ng/mL to about 2200 ng/mL, about 1450 ng/mL to about 2200 ng/mL, about 1500 ng/mL to about 2200 ng/mL, about 1550 ng/mL to about 2200 ng/mL, about 1600 ng/mL to about 2200 ng/mL, about 1650 ng/mL to about 2200 ng/mL, about 900 ng/mL to about 2150 ng/mL, about 950 ng/mL to about 2150 ng/mL, about 1000 ng/mL to about 2150 ng/mL, about 1050 ng/mL to about 2150 ng/mL, about 1100 ng/mL to about 2150 ng/mL, about 1150 ng/mL to about 2150 ng/mL, about 1200 ng/mL to about 2150 ng/mL, about 1250 ng/mL to about 2150 ng/mL, about 1300 ng/mL to about 2150 ng/mL, about 1350 ng/mL to about 2150 ng/mL, about 1400 ng/mL to about 2150 ng/mL, about 1450 ng/mL to about 2150 ng/mL, about 1500 ng/mL to about 2150 ng/mL, about 1550 ng/mL to about 2150 ng/mL, about 1600 ng/mL to about 2150 ng/mL, about 900 ng/mL to about 2100 ng/mL, about 950 ng/mL to about 2100 ng/mL, about 1000 ng/mL to about 2100 ng/mL, about 1050 ng/mL to about 2100 ng/mL, about 1100 ng/mL to about 2100 ng/mL, about 1150 ng/mL to about 2100 ng/mL, about 1200 ng/mL to about 2100 ng/mL, about 1250 ng/mL to about 2100 ng/mL, about 1300 ng/mL to about 2100 ng/mL, about 1350 ng/mL to about 2100 ng/mL, about 1400 ng/mL to about 2100 ng/mL, about 1450 ng/mL to about 2100 ng/mL, about 1500 ng/mL to about 2100 ng/mL, about 1550 ng/mL to about 2100 ng/mL, about 900 ng/mL to about 2050 ng/mL, about 950 ng/mL to about 2050 ng/mL, about 1000 ng/mL to about 2050 ng/mL, about 1050 ng/mL to about 2050 ng/mL, about 1100 ng/mL to about 2050 ng/mL, about 1150 ng/mL to about 2050 ng/mL, about 1200 ng/mL to about 2050 ng/mL, about 1250 ng/mL to about 2050 ng/mL, about 1300 ng/mL to about 2050 ng/mL, about 1350 ng/mL to about 2050 ng/mL, about 1400 ng/mL to about 2050 ng/mL, about 1450 ng/mL to about 2050 ng/mL, about 1500 ng/mL to about 2050 ng/mL, about 900 ng/mL to about 2000 ng/mL, about 950 ng/mL to about 2000 ng/mL, about 1000 ng/mL to about 2000 ng/mL, about 1050 ng/mL to about 2000 ng/mL, about 1100 ng/mL to about 2000 ng/mL, about 1150 ng/mL to about 2000 ng/mL, about 1200 ng/mL to about 2000 ng/mL, about 1250 ng/mL to about 2000 ng/mL, about 1300 ng/mL to about 2000 ng/mL, about 1350 ng/mL to about 2000 ng/mL, about 1400 ng/mL to about 2000 ng/mL, about 1450 ng/mL to about 2000 ng/mL, about 900 ng/mL to about 1950 ng/mL, about 950 ng/mL to about 1950 ng/mL, about 1000 ng/mL to about 1950 ng/mL, about 1050 ng/mL to about 1950 ng/mL, about 1100 ng/mL to about 1950 ng/mL, about 1150 ng/mL to about 1950 ng/mL, about 1200 ng/mL to about 1950 ng/mL, about 1250 ng/mL to about 1950 ng/mL, about 1300 ng/mL to about 1950 ng/mL, about 1350 ng/mL to about 1950 ng/mL, about 1400 ng/mL to about 1950 ng/mL, about 900 ng/mL to about 1900 ng/mL, about 950 ng/mL to about 1900 ng/mL, about 1000 ng/mL to about 1900 ng/mL, about 1050 ng/mL to about 1900 ng/mL, about 1100 ng/mL to about 1900 ng/mL, about 1150 ng/mL to about 1900 ng/mL, about 1200 ng/mL to about 1900 ng/mL, about 1250 ng/mL to about 1900 ng/mL, about 1300 ng/mL to about 1900 ng/mL, about 1350 ng/mL to about 1900 ng/mL, about 900 ng/mL to about 1850 ng/mL, about 950 ng/mL to about 1850 ng/mL, about 1000 ng/mL to about 1850 ng/mL, about 1050 ng/mL to about 1850 ng/mL, about 1100 ng/mL to about 1850 ng/mL, about 1150 ng/mL to about 1850 ng/mL, about 1200 ng/mL to about 1850 ng/mL, about 1250 ng/mL to about 1850 ng/mL, about 1300 ng/mL to about 1850 ng/mL, about 900 ng/mL to about 1800 ng/mL, about 950 ng/mL to about 1800 ng/mL, about 1000 ng/mL to about 1800 ng/mL, about 1050 ng/mL to about 1800 ng/mL, about 1100 ng/mL to about 1800 ng/mL, about 1150 ng/mL to about 1800 ng/mL, about 1200 ng/mL to about 1800 ng/mL, about 1250 ng/mL to about 1800 ng/mL, about 900 ng/mL to about 1750 ng/mL, about 950 ng/mL to about 1750 ng/mL, about 1000 ng/mL to about 1750 ng/mL, about 1050 ng/mL to about 1750 ng/mL, about 1100 ng/mL to about 1750 ng/mL, about 1150 ng/mL to about 1750 ng/mL, about 1200 ng/mL to about 1750 ng/mL, about 900 ng/mL to about 1700 ng/mL, about 950 ng/mL to about 1700 ng/mL, about 1000 ng/mL to about 1700 ng/mL, about 1050 ng/mL to about 1700 ng/mL, about 1100 ng/mL to about 1700 ng/mL, about 1150 ng/mL to about 1700 ng/mL, about 900 ng/mL to about 1650 ng/mL, about 950 ng/mL to about 1650 ng/mL, about 1000 ng/mL to about 1650 ng/mL, about 1050 ng/mL to about 1650 ng/mL, about 1100 ng/mL to about 1650 ng/mL, about 900 ng/mL to about 1600 ng/mL, about 950 ng/mL to about 1600 ng/mL, about 1000 ng/mL to about 1600 ng/mL, about 1050 ng/mL to about 1600 ng/mL, about 900 ng/mL to about 1550 ng/mL, about 950 ng/mL to about 1550 ng/mL, about 1000 ng/mL to about 1550 ng/mL, about 900 ng/mL to about 1500 ng/mL, about 950 ng/mL to about 1500 ng/mL, or about 900 ng/mL to about 1500 ng/mL.

In some aspects of the disclosure, the dosing cycle results in an $AUC_{0-14}$ of about 2200 ng•day/mL to about 5600 ng•day/mL, about 2500 ng•day/mL to about 5600 ng•day/mL, about 2750 ng•day/mL to about 5600 ng•day/mL, about 3000 ng•day/mL to about 5600 ng•day/mL, about 3250 ng•day/mL to about 5600 ng•day/mL, about 3500 ng•day/mL to about 5600 ng•day/mL, about 3750 ng•day/mL to about 5600 ng•day/mL, about 4000 ng•day/mL to about 5600 ng•day/mL, about 4250 ng•day/mL to about 5600 ng•day/mL, about 2200 ng•day/mL to about 5500 ng•day/mL, about 2500 ng•day/mL to about 5500 ng•day/mL, about 2750 ng•day/mL to about 5500 ng•day/mL, about 3000 ng•day/mL to about 5500 ng•day/mL, about 3250 ng•day/mL to about 5500 ng•day/mL, about 3500 ng•day/mL to about 5500 ng•day/mL, about 3750 ng•day/mL to about 5500 ng•day/mL, about 4000 ng•day/mL to about 5500 ng•day/mL, about 2200 ng•day/mL to about 5400 ng•day/mL, about 2500 ng•day/mL to about 5400 ng•day/mL, about 2750 ng•day/mL to about 5400 ng•day/mL, about 3000 ng•day/mL to about 5400 ng•day/mL, about 3250 ng•day/mL to about 5400 ng•day/mL, about 3500 ng•day/mL to about 5400 ng•day/mL, about 3750 ng•day/mL to about 5400 ng•day/mL, about 2200 ng•day/mL to about 5300 ng•day/mL, about 2500 ng•day/mL to about 5300 ng•day/mL, about 2750 ng•day/mL to about 5300 ng•day/mL, about 3000 ng•day/mL to about 5300 ng•day/mL, about 3250 ng•day/mL to about 5300 ng•day/mL, about 3500 ng•day/mL to about 5300 ng•day/mL, about 2200 ng•day/mL to about 5200 ng•day/mL, about 2500 ng•day/mL to about 5200 ng•day/mL, about 2750 ng•day/mL to about 5200 ng•day/mL, about 3000 ng•day/mL to about 5200 ng•day/mL, about 3250 ng•day/mL to about 5200 ng•day/mL, about 2200 ng•day/mL to about 5100 ng•day/mL, about 2500 ng•day/mL to about 5100 ng•day/mL, about 2750 ng•day/mL to about 5100 ng•day/mL, about 3000 ng•day/mL to about 5100 ng•day/mL, about 2200 ng•day/mL to about 5000 ng•day/mL, about 2500 ng•day/mL to about 5000 ng•day/mL, about 2750 ng•day/mL to about 5000 ng•day/mL, about 2200 ng•day/mL to about 4900 ng•day/mL, about 2500 ng•day/mL to about 4900 ng•day/mL, about 2750 ng•day/mL to about 4900 ng•day/mL, about 3000 ng•day/mL to about 4900 ng•day/mL, about 3250 ng•day/mL to about 4900 ng•day/mL, about 3500 ng•day/mL to about 4900 ng•day/mL, about 3750 ng•day/mL to about 4900 ng•day/mL, about 4000 ng•day/mL to about 4900 ng•day/mL, about 4250 ng•day/mL to about 4900 ng•day/mL, about 2200 ng•day/mL to about 4800 ng•day/mL, about 2500 ng•day/mL to about 4800 ng•day/mL, about 2750 ng•day/mL to about 4800 ng•day/mL, about 3000 ng•day/mL to about 4800 ng•day/mL, about 3250 ng•day/mL to about 4800 ng•day/mL, about 3500 ng•day/mL to about 4800 ng•day/mL, about 3750 ng•day/mL to about 4800 ng•day/mL, about 4000 ng•day/mL to about 4800 ng•day/mL, about 4250 ng•day/mL to about 4800 ng•day/mL, about 2200 ng•day/mL to about 4700 ng•day/mL, about 2500 ng•day/mL to about 4700 ng•day/mL, about 2750 ng•day/mL to about 4700 ng•day/mL, about 3000 ng•day/mL to about 4700 ng•day/mL, about 3250 ng•day/mL to about 4700 ng•day/mL, about 3500 ng•day/mL to about 4700 ng•day/mL, about 3750 ng•day/mL to about 4700 ng•day/mL, about 4000 ng•day/mL to about 4700 ng•day/mL, about 4250 ng•day/mL to about 4700 ng•day/mL, about 2200 ng•day/mL to about 4600 ng•day/mL, about 2500 ng•day/mL to about 4600 ng•day/mL, about 2750 ng•day/mL to about 4600 ng•day/mL, about 3000 ng•day/mL to about 4600 ng•day/mL, about 3250 ng•day/mL to about 4600 ng•day/mL, about 3500 ng•day/mL to about 4600 ng•day/mL, about 3750 ng•day/mL to about 4600 ng•day/mL, about 4000 ng•day/mL to about 4600 ng•day/mL, about 4250 ng•day/mL to about 4600 ng•day/mL, about 2200 ng•day/mL to about 4500 ng•day/mL, about 2500 ng•day/mL to about 4500 ng•day/mL, about 2750 ng•day/mL to about 4500 ng•day/mL, about 3000 ng•day/mL to about 4500 ng•day/mL, about 3250 ng•day/mL to about 4500 ng•day/mL, about 3500 ng•day/mL to about 4500 ng•day/mL, about 3750 ng•day/mL to about 4500 ng•day/mL, about 4000 ng•day/mL to about 4500 ng•day/mL, or about 4250 ng•day/mL to about 4500 ng•day/mL.

In yet another example, provided herein is an IL-22 Fc fusion protein for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1) and one or more further doses of the IL-22 Fc fusion protein, wherein the dosing cycle results in a $C_{max}$ of the IL-22 Fc fusion protein of about 1850 ng/mL or lower (e.g., about 1850 ng/mL or lower, about 1800 ng/mL or lower, about 1750 ng/mL or lower, about 1700 ng/mL or lower, about 1650 ng/mL or lower, about 1600 ng/mL or lower, about 1550 ng/mL or lower, about 1500 ng/mL or lower, about 1450 ng/mL or lower, about 1400 ng/mL or lower, about 1350 ng/mL or lower, about 1300 ng/mL or lower, about 1250 ng/mL or lower, about 1200 ng/mL or lower, about 1150 ng/mL or lower, about 1100 ng/mL or lower, about 1050 ng/mL or lower, about 1000 ng/mL or lower, abour 950 ng/mL or lower, or about 900 ng/mL or lower) and/or an an $AUC_{0-14}$ of about 4500 ng•day/mL or lower (e.g., about 4500 ng•day/mL or lower, about 4450 ng•day/mL or lower, about 4400 ng•day/mL or lower, about 4350 ng•day/mL or lower, about 4300 ng•day/mL or lower, about 4250 ng•day/mL or lower, about 4200 ng•day/mL or lower, about 4150 ng•day/mL or lower, about 4100 ng•day/mL or lower, about 4050 ng•day/mL or lower, about 4000 ng•day/mL or lower, about 3950 ng•day/mL or lower, about 3900 ng•day/mL or lower, about 3850 ng•day/mL or lower, about 3800 ng•day/mL or lower, about 3750 ng•day/mL or lower, about 3700 ng•day/mL or lower, about 3650 ng•day/mL or lower, about 3600 ng•day/mL or lower, about 3550 ng•day/mL or lower, about 3500 ng•day/mL or lower, about 3450 ng•day/mL or lower, about 3400 ng•day/mL or lower, about 3350 ng•day/mL or lower, about 3300 ng•day/mL or lower, about 3250 ng•day/mL or lower, about 3200 ng•day/mL or lower, about 3100 ng•day/mL or lower, about 3050 ng•day/mL or lower, about 3000 ng•day/mL or lower, about 2950 ng•day/mL or lower, about 2900 ng•day/mL or lower, about 2850 ng•day/mL or lower, about 2800 ng•day/mL or lower, about 2750 ng•day/mL or lower, about 2700 ng•day/mL or lower, about 2650 ng•day/mL or lower, about 2600 ng•day/mL or lower, about 2550 ng•day/mL or lower, about 2500 ng•day/mL or lower, about 2450 ng•day/mL or lower, about 2400 ng•day/mL or lower, about 2350 ng•day/mL or lower, about 2300 ng•day/mL or lower, about 2250 ng•day/mL or lower, or about 2200 ng•day/mL or lower).

In some aspects of the disclosure, the dosing cycle results in a $C_{max}$ of the IL-22 Fc fusion protein of about 900 ng/mL to about 1850 ng/mL, about 950 ng/mL to about 1850 ng/mL, about 1000 ng/mL to about 1850 ng/mL, about 1050 ng/mL to about 1850 ng/mL, about 1100 ng/mL to about 1850 ng/mL, about 1150 ng/mL to about 1850 ng/mL, about 1200 ng/mL to about 1850 ng/mL, about 1250 ng/mL to about 1850 ng/mL, about 1300 ng/mL to about 1850 ng/mL, about 1350 ng/mL to about 1850 ng/mL, about 1400 ng/mL to about 1850 ng/mL, about 1450 ng/mL to about 1850 ng/mL, about 1500 ng/mL to about 1850 ng/mL, about 1550 ng/mL to about 1850 ng/mL, about 1600 ng/mL to about 1850 ng/mL, about 1650 ng/mL to about 1850 ng/mL, about 1700 ng/mL to about 1850 ng/mL, about 1750 ng/mL to about 1850 ng/mL, about 1800 ng/mL to about 1850 ng/mL, about 900 ng/mL to about 1800 ng/mL, about 950 ng/mL to about 1800 ng/mL, about 1000 ng/mL to about 1800 ng/mL, about 1050 ng/mL to about 1800 ng/mL, about 1100 ng/mL to about 1800 ng/mL, about 1150 ng/mL to about 1800 ng/mL, about 1200 ng/mL to about 1800 ng/mL, about 1250 ng/mL to about 1800 ng/mL, about 1300 ng/mL to about 1800 ng/mL, about 1350 ng/mL to about 1800 ng/mL, about 1400 ng/mL to about 1800 ng/mL, about 1450 ng/mL to about 1800 ng/mL, about 1500 ng/mL to about 1800 ng/mL, about 1550 ng/mL to about 1800 ng/mL, about 1600 ng/mL to about 1800 ng/mL, about 1650 ng/mL to about 1800 ng/mL, about 1700 ng/mL to about 1800 ng/mL, about 1750 ng/mL to about 1800 ng/mL, about 900 ng/mL to about 1750 ng/mL, about 950 ng/mL to about 1750 ng/mL, about 1000 ng/mL to about 1750 ng/mL, about 1050 ng/mL to about 1750 ng/mL, about 1100 ng/mL to about 1750 ng/mL, about 1150 ng/mL to about 1750 ng/mL, about 1200 ng/mL to about 1750 ng/mL, about 1250 ng/mL to about 1750 ng/mL, about 1300 ng/mL to about 1750 ng/mL, about 1350 ng/mL to about 1750 ng/mL, about 1400 ng/mL to about 1750 ng/mL, about 1450 ng/mL to about 1750 ng/mL, about 1500 ng/mL to about 1750 ng/mL, about 1550 ng/mL to about 1750 ng/mL, about 1600 ng/mL to about 1750 ng/mL, about 1650 ng/mL to about 1750 ng/mL, about 1700 ng/mL to about 1750 ng/mL, about 900 ng/mL to about 1700 ng/mL, about 950 ng/mL to about 1700 ng/mL, about 1000 ng/mL to about 1700 ng/mL, about 1050 ng/mL to about 1700 ng/mL, about 1100 ng/mL to about 1700 ng/mL, about 1150 ng/mL to about 1700 ng/mL, about 1200 ng/mL to about 1700 ng/mL, about 1250 ng/mL to about 1700 ng/mL, about 1300 ng/mL to about 1700 ng/mL, about 1350 ng/mL to about 1700 ng/mL, about 1400 ng/mL to about 1700 ng/mL, about 1450 ng/mL to about 1700 ng/mL, about 1500 ng/mL to about 1700 ng/mL, about 1550 ng/mL to about 1700 ng/mL, about 1600 ng/mL to about 1700 ng/mL, about 1650 ng/mL to about 1700 ng/mL, about 900 ng/mL to about 1650 ng/mL, about 950 ng/mL to about 1650 ng/mL, about 1000 ng/mL to about 1650 ng/mL, about 1050 ng/mL to about 1650 ng/mL, about 1100 ng/mL to about 1650 ng/mL, about 1150 ng/mL to about 1650 ng/mL, about 1200 ng/mL to about 1650 ng/mL, about 1250 ng/mL to about 1650 ng/mL, about 1300 ng/mL to about 1650 ng/mL, about 1350 ng/mL to about 1650 ng/mL, about 1400 ng/mL to about 1650 ng/mL, about 1450 ng/mL to about 1650 ng/mL, about 1500 ng/mL to about 1650 ng/mL, about 1550 ng/mL to about 1650 ng/mL, about 1600 ng/mL to about 1650 ng/mL, about 900 ng/mL to about 1600 ng/mL, about 950 ng/mL to about 1600 ng/mL, about 1000 ng/mL to about 1600 ng/mL, about 1050 ng/mL to about 1600 ng/mL, about 1100 ng/mL to about 1600 ng/mL, about 1150 ng/mL to about 1600 ng/mL, about 1200 ng/mL to about 1600 ng/mL, about 1250 ng/mL to about 1600 ng/mL, about 1300 ng/mL to about 1600 ng/mL, about 1350 ng/mL to about 1600 ng/mL, about 1400 ng/mL to about 1600 ng/mL, about 1450 ng/mL to about 1600 ng/mL, about 1500 ng/mL to about 1600 ng/mL, about 1550 ng/mL to about 1600 ng/mL, about 900 ng/mL to about 1550 ng/mL, about 950 ng/mL to about 1550 ng/mL, about 1000 ng/mL to about 1550 ng/mL, about 1050 ng/mL to about 1550 ng/mL, about 1100 ng/mL to about 1550 ng/mL, about 1150 ng/mL to about 1550 ng/mL, about 1200 ng/mL to about 1550 ng/mL, about 1250 ng/mL to about 1550 ng/mL, about 1300 ng/mL to about 1550 ng/mL, about 1350 ng/mL to about 1550 ng/mL, about 1400 ng/mL to about 1550 ng/mL, about 1450 ng/mL to about 1550 ng/mL, about 1500 ng/mL to about 1550 ng/mL, about 900 ng/mL to about 1500 ng/mL, about 950 ng/mL to about 1500 ng/mL, about 1000 ng/mL to about 1500 ng/mL, about 1050 ng/mL to about 1500 ng/mL, about 1100 ng/mL to about 1500 ng/mL, about 1150 ng/mL to about 1500 ng/mL, about 1200 ng/mL to about 1500 ng/mL, about 1250 ng/mL to about 1500 ng/mL, about 1300 ng/mL to about 1500 ng/mL, about 1350 ng/mL to about 1500 ng/mL, about 1400 ng/mL to about 1500 ng/mL, about 1450 ng/mL to about 1500 ng/mL, about 900 ng/mL to about 1450 ng/mL, about 950 ng/mL to about 1450 ng/mL, about 1000 ng/mL to about 1450 ng/mL, about 1050 ng/mL to about 1450 ng/mL, about 1100 ng/mL to about 1450 ng/mL, about 1150 ng/mL to about 1450 ng/mL, about 1200 ng/mL to about 1450 ng/mL, about 1250 ng/mL to about 1450 ng/mL, about 1300 ng/mL to about 1450 ng/mL, about 1350 ng/mL to about 1450 ng/mL, about 1400 ng/mL to about 1450 ng/mL, about 900 ng/mL to about 1400 ng/mL, about 950 ng/mL to about 1400 ng/mL, about 1000 ng/mL to about 1400 ng/mL, about 1050 ng/mL to about 1400 ng/mL, about 1100 ng/mL to about 1400 ng/mL, about 1150 ng/mL to about 1400 ng/mL, about 1200 ng/mL to about 1400 ng/mL, about 1250 ng/mL to about 1400 ng/mL, about 1300 ng/mL to about 1400 ng/mL, about 1350 ng/mL to about 1400 ng/mL, about 900 ng/mL to about 1350 ng/mL, about 950 ng/mL to about 1350 ng/mL, about 1000 ng/mL to about 1350 ng/mL, about 1050 ng/mL to about 1350 ng/mL, about 1100 ng/mL to about 1350 ng/mL, about 1150 ng/mL to about 1350 ng/mL, about 1200 ng/mL to about 1350 ng/mL, about 1250 ng/mL to about 1350 ng/mL, about 1300 ng/mL to about 1350 ng/mL, about 900 ng/mL to about 1300 ng/mL, about 950 ng/mL to about 1300 ng/mL, about 1000 ng/mL to about 1300 ng/mL, about 1050 ng/mL to about 1300 ng/mL, about 1100 ng/mL to about 1300 ng/mL, about 1150 ng/mL to about 1300 ng/mL, about 1200 ng/mL to about 1300 ng/mL, about 1250 ng/mL to about 1300 ng/mL, about 900 ng/mL to about 1250 ng/mL, about 950 ng/mL to about 1250 ng/mL, about 1000 ng/mL to about 1250 ng/mL, about 1050 ng/mL to about 1250 ng/mL, about 1100 ng/mL to about 1250 ng/mL, about 1150 ng/mL to about 1250 ng/mL, about 1200 ng/mL to about 1250 ng/mL, about 900 ng/mL to about 1200 ng/mL, about 950 ng/mL to about 1200 ng/mL, about 1000 ng/mL to about 1200 ng/mL, about 1050 ng/mL to about 1200 ng/mL, about 1100 ng/mL to about 1200 ng/mL, about 1150 ng/mL to about 1200 ng/mL, about 900 ng/mL to about 1150 ng/mL, about 950 ng/mL to about 1150 ng/mL, about 1000 ng/mL to about 1150 ng/mL, about 1050 ng/mL to about 1150 ng/mL, about 1100 ng/mL to about 1150 ng/mL, about 900 ng/mL to about 1100 ng/mL, about 950 ng/mL to about 1100 ng/mL, about 1000 ng/mL to about 1100 ng/mL, about 1050 ng/mL to about 1100 ng/mL, about 900 ng/mL to about 1050 ng/mL, about 950 ng/mL to about 1050 ng/mL, about 1000 ng/mL to about 1050 ng/mL, about 900 ng/mL to about 1000 ng/mL, about 950 ng/mL to about 1000 ng/mL, or about 900 ng/mL to about 950 ng/mL.

In some aspects of the disclosure, the dosing cycle results in an $AUC_{0-14}$ of about 2200 ng·day/mL to about 4500 ng·day/mL, about 2500 ng·day/mL to about 4500 ng·day/mL, about 2750 ng·day/mL to about 4500 ng·day/mL, about 3000 ng·day/mL to about 4500 ng·day/mL, about 3250 ng·day/mL to about 4500 ng·day/mL, about 3500 ng·day/mL to about 4500 ng·day/mL, about 3750 ng·day/mL to about 4500 ng·day/mL, about 4000 ng·day/mL to about 4500 ng·day/mL, about 4250 ng·day/mL to about 4500 ng·day/mL, about 2200 ng·day/mL to about 4250 ng·day/mL, about 2500 ng·day/mL to about 4250 ng·day/mL, about 2750 ng·day/mL to about 4250 ng·day/mL, about 3000 ng·day/mL to about 4250 ng·day/mL, about 3250 ng·day/mL to about 4250 ng·day/mL, about 3500 ng·day/mL to about 4250 ng·day/mL, about 3750 ng·day/mL to about 4250 ng·day/mL, about 4000 ng·day/mL to about 4250 ng·day/mL, about 2200 ng·day/mL to about 4000 ng·day/mL, about 2500 ng·day/mL to about 4000 ng·day/mL, about 2750 ng·day/mL to about 4000 ng·day/mL, about 3000 ng·day/mL to about 4000 ng·day/mL, about 3250 ng·day/mL to about 4000 ng·day/mL, about 3500 ng·day/mL to about 4000 ng·day/mL, about 3750 ng·day/mL to about 4000 ng·day/mL, about 2200 ng·day/mL to about 3750 ng·day/mL, about 2500 ng·day/mL to about 3750 ng·day/mL, about 2750 ng·day/mL to about 3750 ng·day/mL, about 3000 ng·day/mL to about 3750 ng·day/mL, about 3250 ng·day/mL to about 3750 ng·day/mL, about 3500 ng·day/mL to about 3750 ng·day/mL, about 2200 ng·day/mL to about 3500 ng·day/mL, about 2500 ng·day/mL to about 3500 ng·day/mL, about 2750 ng·day/mL to about 3500 ng·day/mL, about 3000 ng·day/mL to about 3500 ng·day/mL, about 3250 ng·day/mL to about 3500 ng·day/mL, about 2200 ng·day/mL to about 3250 ng·day/mL, about 2500 ng·day/mL to about 3250 ng·day/mL, about 2750 ng·day/mL to about 3250 ng·day/mL, about 3000 ng·day/mL to about 3250 ng·day/mL, about 2200 ng·day/mL to about 3000 ng·day/mL, about 2500 ng·day/mL to about 3000 ng·day/mL, about 2750 ng·day/mL to about 3000 ng·day/mL, about 2200 ng·day/mL to about 2750 ng•day/mL, about 2500 ng•day/mL to about 2750 ng•day/mL, or about 2200 ng•day/mL to about 2500 ng•day/mL.

The dosing cycle can result in a $C_{max}$ of the IL-22 Fc fusion protein of greater than about 100 ng/mL. For example, in some aspects, the dosing cycle results in a $C_{max}$ of the IL-22 Fc fusion protein of between about 100 ng/mL and about 2350 ng/mL. In some aspects, the dosing cycle results in a $C_{max}$ of the IL-22 Fc fusion protein of between about 100 ng/mL and about 1850 ng/mL. In some embodiments, the dosing cycle results in a $C_{max}$ of the IL-22 Fc fusion protein of between about 100 ng/mL and about 1810 ng/mL.

The dosing cycle can result in an $AUC_{0-14}$ of the IL-22 Fc fusion protein of greater than about 1200 ng•day/mL. For example, in some aspects, the dosing cycle results in an $AUC_{0-14}$ of the IL-22 Fc fusion protein of between about 1200 ng•day/mL and about 5600 ng•day/mL. In some embodiments, the dosing cycle results in an $AUC_{0-14}$ of the IL-22 Fc fusion protein of between about 1200 ng•day/mL and about 4500 ng•day/mL. In some aspects, the dosing cycle results in an $AUC_{0-14}$ of the IL-22 Fc fusion protein of between about 1200 ng•day/mL and about 4310 ng•day/mL.

The $C_{max}$ and/or the $AUC_{0-14}$ may be determined in any suitable compartment or biological sample. In some aspects, the $C_{max}$ and/or the $AUC_{0-14}$ may be determined in serum. In some aspects, the $C_{max}$ may be determined in serum. In some aspects, the $AUC_{0-14}$ may be determined in serum.

In some aspects of the disclosure, the dose(s) are administered to the subject every week (q1w), every two weeks (q2w), every three weeks (q3w), every four weeks, (q4w), every five weeks (q5w), every six weeks (q6w), every seven weeks (q7w), every eight weeks (q8w), every nine weeks (q9w), every ten weeks (q10w), every 12 weeks (q12w), every fourteen weeks (q14w), every sixteen weeks (q16w), every eighteen weeks (q18w), or every twenty weeks (q20w). For example, in some aspects, the doses are administered to the subject every week (q1w), every two weeks (q2w), every four weeks (q4w), or every six weeks (q6w). In some aspects, the doses are administered to the subject every week (q1w), every two weeks (q2w), or every four weeks (q4w). In particular aspects, the doses are administered to the subject every two weeks (q2w).

For example, in some aspects, the dosing cycle includes about one to about twenty doses, about one to about nineteen doses, about one to about eighteen doses, about one to about seventeen doses, about one to about sixteen doses, about one to about fifteen doses, about one to about fourteen doses, about one to about thirteen doses, about one to about twelve doses, about one to about eleven doses, about one to about ten doses, about one to about nine doses, about one to about eight doses, about one to about seven doses, about one to about six doses, about one to about five doses, about one to about four doses, about one to about three doses, about one to about two doses, about two to about twenty doses, about two to about nineteen doses, about two to about eighteen doses, about two to about seventeen doses, about two to about sixteen doses, about two to about fifteen doses, about two to about fourteen doses, about two to about thirteen doses, about two to about twelve doses, about two to about eleven doses, about two to about ten doses, about two to about nine doses, about two to about eight doses, about two to about seven doses, about two to about six doses, about two to about five doses, about two to about four doses, about two to about three doses, about three to about twenty doses, about three to about nineteen doses, about three to about eighteen doses, about three to about seventeen doses, about three to about sixteen doses, about three to about fifteen doses, about three to about fourteen doses, about three to about thirteen doses, about three to about twelve doses, about three to about eleven doses, about three to about ten doses, about three to about nine doses, about three to about eight doses, about three to about seven doses, about three to about six doses, about three to about five doses, about three to about four doses, about four to about twenty doses, about four to about nineteen doses, about four to about eighteen doses, about four to about seventeen doses, about four to about sixteen doses, about four to about fifteen doses, about four to about fourteen doses, about four to about thirteen doses, about four to about twelve doses, about four to about eleven doses, about four to about ten doses, about four to about nine doses, about four to about eight doses, about four to about seven doses, about four to about six doses, about four to about five doses, about five to about twenty doses, about five to about nineteen doses, about five to about eighteen doses, about five to about seventeen doses, about five to about sixteen doses, about five to about fifteen doses, about five to about fourteen doses, about five to about thirteen doses, about five to about twelve doses, about five to about eleven doses, about five to about ten doses, about five to about nine doses, about five to about eight doses, about five to about seven doses, about five to about six doses, about six to about twenty doses, about six to about nineteen doses, about six to about eighteen doses, about six to about seventeen doses, about six to about sixteen doses, about six to about fifteen doses, about six to about fourteen doses, about six to about thirteen doses, about six to about twelve doses, about six to about eleven doses, about six to about ten doses, about six to about nine doses, about six to about eight doses, about six to about seven doses, about seven to about twenty doses, about seven to about nineteen doses, about seven to about eighteen doses, about seven to about seventeen doses, about seven to about sixteen doses, about seven to about fifteen doses, about seven to about fourteen doses, about seven to about thirteen doses, about seven to about twelve doses, about seven to about eleven doses, about seven to about ten doses, about seven to about nine doses, about seven to about eight doses, about eight to about twenty doses, about eight to about nineteen doses, about eight to about eighteen doses, about eight to about seventeen doses, about eight to about sixteen doses, about eight to about fifteen doses, about eight to about fourteen doses, about eight to about thirteen doses, about eight to about twelve doses, about eight to about eleven doses, about eight to about ten doses, about eight to about nine doses, about nine to about twenty doses, about nine to about nineteen doses, about nine to about eighteen doses, about nine to about seventeen doses, about nine to about sixteen doses, about nine to about fifteen doses, about nine to about fourteen doses, about nine to about thirteen doses, about nine to about twelve doses, about nine to about eleven doses, about nine to about ten doses, about ten to about twenty doses, about ten to about nineteen doses, about ten to about eighteen doses, about ten to about seventeen doses, about ten to about sixteen doses, about ten to about fifteen doses, about ten to about fourteen doses, about ten to about thirteen doses, about ten to about twelve doses, about ten to about eleven doses, about eleven to about twenty doses, about eleven to about nineteen doses, about eleven to about eighteen doses, about eleven to about seventeen doses, about eleven to about sixteen doses, about eleven to about fifteen doses, about eleven to about fourteen doses, about eleven to about thirteen doses, about eleven to about twelve doses, about twelve to about twenty doses, about twelve to about nineteen doses, about twelve to about eighteen doses, about twelve to about seventeen doses, about twelve to about sixteen doses, about twelve to about fifteen doses, about twelve to about fourteen doses, about twelve to about thirteen doses, about thirteen to about twenty doses, about thirteen to about nineteen doses, about thirteen to about eighteen doses, about thirteen to about seventeen doses, about thirteen to about sixteen doses, about thirteen to about fifteen doses, about thirteen to about fourteen doses, about fourteen to about twenty doses, about fourteen to about nineteen doses, about fourteen to about eighteen doses, about fourteen to about seventeen doses, about fourteen to about sixteen doses, about fourteen to about fifteen doses, about fifteen to about twenty doses, about fifteen to about nineteen doses, about fifteen to about eighteen doses, about fifteen to about seventeen doses, about fifteen to about sixteen doses, about sixteen to about twenty doses, about sixteen to about nineteen doses, about sixteen to about eighteen doses, about sixteen to about seventeen doses, about seventeen to about twenty doses, about seventeen to about nineteen doses, about seventeen to about eighteen doses, about eighteen to about twenty doses, about eighteen to about nineteen doses, or about nineteen to about twenty doses.

In some aspects, one total dose, two total doses, three total doses, four total doses, five total doses, six total doses, seven total doses, eight total doses, nine total doses, ten total doses, eleven total doses, twelve total doses, thirteen total doses, fourteen total doses, fifteen total doses, sixteen total doses, seventeen total doses, eighteen total doses, nineteen total doses, twenty total doses, or more total doses of the IL-22 Fc fusion protein (e.g., as described herein, e.g., an IL-22 Fc fusion protein comprising the amino acid sequence set forth in SEQ ID NO: 8) are administered to the subject.

For example, in some aspects, one total dose, two total doses, three total doses, four total doses, five total doses, or six total doses of the IL-22 Fc fusion protein (e.g., as described herein, e.g., an IL-22 Fc fusion protein comprising the amino acid sequence set forth in SEQ ID NO: 8) are administered to the subject.

In some aspects, no more than two total doses, no more than three total doses, no more than four total doses, no more than five total doses, no more than six total doses, no more than seven total doses, no more than eight total doses, no more than nine total doses, no more than ten total doses, no more than eleven total doses, no more than twelve total doses, no more than thirteen total doses, no more than fourteen total doses, no more than fifteen total doses, no more than sixteen total doses, no more than seventeen total doses, no more than eighteen total doses, no more than nineteen total doses, or no more than twenty total doses of the IL-22 Fc fusion protein (e.g., as described herein, e.g., an IL-22 Fc fusion protein comprising the amino acid sequence set forth in SEQ ID NO: 8) are administered to the subject.

For example, in some aspects, no more than two total doses, no more than three total doses, no more than four total doses, no more than five total doses, or no more than six total doses of the IL-22 Fc fusion protein (e.g., as described herein, e.g., an IL-22 Fc fusion protein comprising the amino acid sequence set forth in SEQ ID NO: 8) are administered to the subject. In some particular embodiments, no more than six total doses of the IL-22 Fc fusion protein (e.g., as described herein, e.g., an IL-22 Fc fusion protein comprising the amino acid sequence set forth in SEQ ID NO: 8) are administered to the subject.

In some aspects, the one or more further doses comprise at least a second dose (C1D2).

In some aspects, the one or more further doses comprise at least a C1D2 and a third dose (C1D3).

In some aspects, the one or more further doses comprise at least a C1D2, a C1D3, and a fourth dose (C1D4).

In some aspects, the one or more further doses comprise at least a C1D2, a C1D3, a C1D4, and a fifth dose (C1D5).

In some aspects, the one or more further doses comprise at least a C1D2, a C1D3, a C1D4, a C1D5, and a sixth dose (C1D6).

In some aspects, the dosing cycle comprises the C1D1, a C1D2, a C1D3, a C1D4, a C1D5, and a C1D6.

In some aspects, the dosing cycle comprises the C1D1, a C1D2, a C1D3, a C1D4, a C1D5, a C1D6, and a C1D7.

In some aspects, the dosing cycle comprises the C1D1, a C1D2, a C1D3, a C1D4, a C1D5, a C1D6, a C1D7, and a C1D8.

As one example, in some aspects, the dosing regimen comprises the C1D1, a second dose (C1D2), a third dose (C1D3), a fourth dose (C1D4), a fifth dose (C1D5), and a sixth dose (C1D6) of the IL-22 Fc fusion protein.

In one example, provided herein is an IL-22 Fc fusion protein for use in preventing acute GVHD in a subject,

wherein the IL-22 Fc fusion protein is for administration to a subject in need thereof in a dosing regimen comprising a dosing cycle, wherein the dosing cycle comprises two total doses of the IL-22 Fc fusion protein that are administered to the subject concurrently with or after allo-HSCT.

In a further example, provided herein is an IL-22 Fc fusion protein for use in reducing the risk of developing chronic GVHD in a subject, wherein the IL-22 Fc fusion protein is for administration to a subject in need thereof in a dosing regimen comprising a dosing cycle, wherein the dosing cycle comprises two total doses of the IL-22 Fc fusion protein that are administered to the subject concurrently with or after allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises two total doses of the IL-22 Fc fusion protein that are administered to the subject concurrently with or after allo-HSCT.

In another example, provided herein is an IL-22 Fc fusion protein for use in preventing acute GVHD in a subject, wherein the IL-22 Fc fusion protein is for administration to a subject in need thereof in a dosing regimen comprising a dosing cycle, wherein the dosing cycle comprises three total doses of the IL-22 Fc fusion protein that are administered to the subject concurrently with or after allo-HSCT.

In a further example, provided herein is an IL-22 Fc fusion protein for use in reducing the risk of developing chronic GVHD in a subject, wherein the IL-22 Fc fusion protein is for administration to a subject in need thereof in a dosing regimen comprising a dosing cycle, wherein the dosing cycle comprises three total doses of the IL-22 Fc fusion protein that are administered to the subject concurrently with or after allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises three total doses of the IL-22 Fc fusion protein that are administered to the subject concurrently with or after allo-HSCT.

In another example, provided herein is an IL-22 Fc fusion protein for use in preventing acute GVHD in a subject, wherein the IL-22 Fc fusion protein is for administration to a subject in need thereof in a dosing regimen comprising a dosing cycle, wherein the dosing cycle comprises four total doses of the IL-22 Fc fusion protein that are administered to the subject concurrently with or after allo-HSCT.

In a further example, provided herein is an IL-22 Fc fusion protein for use in reducing the risk of developing chronic GVHD in a subject, wherein the IL-22 Fc fusion protein is for administration to a subject in need thereof in a dosing regimen comprising a dosing cycle, wherein the dosing cycle comprises four total doses of the IL-22 Fc fusion protein that are administered to the subject concurrently with or after allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises four total doses of the IL-22 Fc fusion protein that are administered to the subject concurrently with or after allo-HSCT.

In another example, provided herein is an IL-22 Fc fusion protein for use in preventing acute GVHD in a subject, wherein the IL-22 Fc fusion protein is for administration to a subject in need thereof in a dosing regimen comprising a dosing cycle, wherein the dosing cycle comprises five total doses of the IL-22 Fc fusion protein that are administered to the subject concurrently with or after allo-HSCT.

In a further example, provided herein is an IL-22 Fc fusion protein for use in reducing the risk of developing chronic GVHD in a subject, wherein the IL-22 Fc fusion protein is for administration to a subject in need thereof in a dosing regimen comprising a dosing cycle, wherein the dosing cycle comprises five total doses of the IL-22 Fc fusion protein that are administered to the subject concurrently with or after allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises five total doses of the IL-22 Fc fusion protein that are administered to the subject concurrently with or after allo-HSCT.

In another example, provided herein is an IL-22 Fc fusion protein for use in preventing acute GVHD in a subject, wherein the IL-22 Fc fusion protein is for administration to a subject in need thereof in a dosing regimen comprising a dosing cycle, wherein the dosing cycle comprises six total doses of the IL-22 Fc fusion protein that are administered to the subject concurrently with or after allo-HSCT.

In a further example, provided herein is an IL-22 Fc fusion protein for use in reducing the risk of developing chronic GVHD in a subject, wherein the IL-22 Fc fusion protein is for administration to a subject in need thereof in a dosing regimen comprising a dosing cycle, wherein the dosing cycle comprises six total doses of the IL-22 Fc fusion protein that are administered to the subject concurrently with or after allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises six total doses of the IL-22 Fc fusion protein that are administered to the subject concurrently with or after allo-HSCT.

Any suitable amount of the IL-22 Fc fusion protein may be administered to the subject in each dose. For example, in some aspects, each dose (e.g., the C1D1, the C1D2, the C1D3, the C1D4, the C1D5, and/or the C1D6) is between about 1 $\mu$g/kg to about 200 $\mu$g/kg of the IL-22 Fc fusion protein (e.g., about 60 $\mu$g/kg). In some aspects, each dose (e.g., the C1D1, the C1D2, the C1D3, the C1D4, the C1D5, and/or the C1D6) is between about 1 $\mu$g/kg to about 200 $\mu$g/kg of the IL-22 Fc fusion protein. For example, in some aspects, each dose in the dosing cycle is about 30 $\mu$g/kg to about 120 $\mu$g/kg, about 30 $\mu$g/kg to about 90 $\mu$g/kg, about 60 $\mu$g/kg to about 120 $\mu$g/kg, or about 60 $\mu$g/kg to about 90 $\mu$g/kg. In some aspects, each dose is about 30 $\mu$g/kg. In some embodiments, each dose is about 60 $\mu$g/kg. In some aspects, each dose is about 90 $\mu$g/kg. In some aspects, each dose is about 120 $\mu$g/kg.

For example, in some particular aspects, the C1D1, the C1D2, the C1D3, the C1D4, the C1D5, and/or the C1D6 are each between about 30 $\mu$g/kg to about 120 $\mu$g/kg. In some aspects, the C1D1, the C1D2, the C1D3, the C1D4, the C1D5, and the C1D6 are each about 30 $\mu$g/kg. In some aspects, the C1D1, the C1D2, the C1D3, the C1D4, the C1D5, and the C1D6 are each about 60 $\mu$g/kg. In some aspects, the C1D1, the C1D2, the C1D3, the C1D4, the C1D5, and the C1D6 are each about 90 $\mu$g/kg. In some aspects, the C1D1, the C1D2, the C1D3, the C1D4, the C1D5, and the C1D6 are each about 120 $\mu$g/kg.

For example, in some aspects, a total of about 1 $\mu$g/kg to about 2000 $\mu$g/kg of the IL-22 Fc fusion protein is administered to the subject in the dosing cycle, e.g., about 60 $\mu$g/kg.

For example, in some aspects, a total of about 1 $\mu$g/kg to about 2000 $\mu$g/kg of the IL-22 Fc fusion protein is administered to the subject in the dosing cycle.

In some aspects, a total of about 180 $\mu$g/kg to about 540 $\mu$g/kg of the IL-22 Fc fusion protein is administered to the subject in the dosing cycle.

In some aspects, the length of the dosing cycle is between about 1 week and about 30 weeks, e.g., about 1 week, about 2 weeks, about 3 weeks, about 4 weeks, about 5 weeks, about 6 weeks, about 7 weeks, about 8 weeks, about 9 weeks, about 10 weeks, about 11 weeks, about 12 weeks, about 13 weeks, about 14 weeks, about 15 weeks, about 16 weeks, about 17 weeks, about 18 weeks, about 19 weeks, about 20 weeks, about 21 weeks, about 22 weeks, about 23 weeks, about 24 weeks, about 25 weeks, about 26 weeks, about 27 weeks, about 28 weeks, about 29 weeks, or about 30 weeks. In some embodiments, the length of the dosing cycle is about 71 days.

For example, in some aspects, the length of the dosing cycle is between about 1 week and about 30 weeks, between about 1 week about 25 weeks, between about 1 week and about 20 weeks, between about 1 week and about 15 weeks, between about 1 week and about 11 weeks, between about 1 week and about 10 weeks, between about 1 week and about 9 weeks, between about 1 week and about 8 weeks, between about 1 week and about 7 weeks, between about 1 week and about 6 weeks, between about 1 week and about 5 weeks, between about 1 week and about 4 weeks, between about 1 week and about 3 weeks, between about 1 week and about 2 weeks, about 2 weeks and about 30 weeks, between about 2 weeks about 25 weeks, between about 2 weeks and about 20 weeks, between about 2 weeks and about 15 weeks, between about 2 weeks and about 11 weeks, between about 2 weeks and about 10 weeks, between about 2 weeks and about 9 weeks, between about 2 weeks and about 8 weeks, between about 2 weeks and about 7 weeks, between about 2 weeks and about 6 weeks, between about 2 weeks and about 5 weeks, between about 2 weeks and about 4 weeks, between about 2 weeks and about 3 weeks, between about 3 weeks and about 30 weeks, between about 3 weeks about 25 weeks, between about 3 weeks and about 20 weeks, between about 3 weeks and about 15 weeks, between about 3 weeks and about 11 weeks, between about 3 weeks and about 10 weeks, between about 3 weeks and about 9 weeks, between about 3 weeks and about 8 weeks, between about 3 weeks and about 7 weeks, between about 3 weeks and about 6 weeks, between about 3 weeks and about 5 weeks, between about 3 weeks and about 4 weeks, between about 4 weeks and about 30 weeks, between about 4 weeks about 25 weeks, between about 4 weeks and about 20 weeks, between about 4 weeks and about 15 weeks, between about 4 weeks and about 11 weeks, between about 4 weeks and about 10 weeks, between about 4 weeks and about 9 weeks, between about 4 weeks and about 8 weeks, between about 4 weeks and about 7 weeks, between about 4 weeks and about 6 weeks, between about 4 weeks and about 5 weeks, between about 5 weeks and about 30 weeks, between about 5 weeks about 25 weeks, between about 5 weeks and about 20 weeks, between about 5 weeks and about 15 weeks, between about 5 weeks and about 11 weeks, between about 5 weeks and about 10 weeks, between about 5 weeks and about 9 weeks, between about 5 weeks and about 8 weeks, between about 5 weeks and about 7 weeks, between about 5 weeks and about 6 weeks, between about 6 weeks and about 30 weeks, between about 6 weeks about 25 weeks, between about 6 weeks and about 20 weeks, between about 6 weeks and about 15 weeks, between about 6 weeks and about 11 weeks, between about 6 weeks and about 10 weeks, between about 6 weeks and about 9 weeks, between about 6 weeks and about 8 weeks, between about 6 weeks and about 7 weeks, between about 7 weeks and about 30 weeks, between about 7 weeks about 25 weeks, between about 7 weeks and about 20 weeks, between about 7 weeks and about 15 weeks, between about 7 weeks and about 11 weeks, between about 7 weeks and about 10 weeks, between about 7 weeks and about 9 weeks, between about 7 weeks and about 8 weeks, between about 8 weeks and about 30 weeks, between about 8 weeks about 25 weeks, between about 8 weeks and about 20 weeks, between about 8 weeks and about 15 weeks, between about 8 weeks and about 11 weeks, between about 8 weeks and about 10 weeks, between about 8 weeks and about 9 weeks, between about 9 weeks and about 30 weeks, between about 9 weeks about 25 weeks, between about 9 weeks and about 20 weeks, between about 9 weeks and about 15 weeks, between about 9 weeks and about 11 weeks, between about 9 weeks and about 10 weeks, between about 10 weeks and about 30 weeks, between about 10 weeks about 25 weeks, between about 10 weeks and about 20 weeks, between about 10 weeks and about 15 weeks, between about 10 weeks and about 11 weeks, between about 11 weeks and about 30 weeks, between about 11 weeks about 25 weeks, between about 11 weeks and about 20 weeks, between about 11 weeks and about 15 weeks, between about 12 weeks and about 30 weeks, between about 12 weeks about 25 weeks, between about 12 weeks and about 20 weeks, between about 12 weeks and about 15 weeks, between about 13 weeks and about 30 weeks, between about 13 weeks about 25 weeks, between about 13 weeks and about 20 weeks, between about 13 weeks and about 15 weeks, between about 14 weeks and about 30 weeks, between about 14 weeks about 25 weeks, between about 14 weeks and about 20 weeks, between about 14 weeks and about 15 weeks, between about 15 weeks and about 30 weeks, between about 15 weeks about 25 weeks, between about 15 weeks and about 20 weeks, between about 20 weeks and about 30 weeks, between about 20 weeks about 25 weeks, or between about 25 weeks and about 30 weeks. In some aspects, the length of the dosing cycle is between 5 weeks and 15 weeks. In some aspects, the length of the dosing cycle is between 8 weeks and 12 weeks. In particular aspects, the length of the dosing cycle is about 8 weeks.

In other particular aspects, the length of the dosing cycle is about 10 weeks. In yet other particular aspects, the length of the dosing cycle is about 11 weeks. In yet other particular aspects, the length of the dosing cycle is about 12 weeks. In yet other particular aspects, the length of the dosing cycle is about 13 weeks. In yet other particular aspects, the length of the dosing cycle is about 14 weeks. In yet other particular embodiments, the length of the dosing cycle is about 15 weeks.

For example, in some aspects, the dosing cycle comprises 2 doses.

In another example, in some aspects, the dosing cycle comprises 3 doses.

In yet another example, in someaspects, the dosing cycle comprises 4 doses.

In a further example, in some aspects, the dosing cycle comprises 5 doses.

In a further example still, in some aspects, the dosing cycle comprises 6 doses.

For example, in some embodiments, a total of about 1 μg/kg to about 2000 μg/kg of the IL-22 Fc fusion protein is administered to the subject in the dosing cycle

For example, in some aspects, a total of about 1 μg/kg to about 2000 μg/kg of the IL-22 Fc fusion protein is administered to the subject in the dosing cycle.

In some aspects, a total of about 180 μg/kg to about 540 μg/kg of the IL-22 Fc fusion protein is administered to the subject in the dosing cycle.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject in need thereof, wherein the IL-22 Fc fusion protein is administered to the subject in a dosing regimen comprising a dosing cycle, wherein the dosing cycle comprises one total dose of the IL-22 Fc fusion protein, wherein the dose dose is about 60 μg/kg, and wherein the dose is administered to the subject q1w, q2w, q3w, or q4w, preferably q2w, after allo-HSCT.

I

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject in need thereof, wherein the IL-22 Fc fusion protein is administered to the subject in a dosing regimen comprising a dosing cycle, wherein the dosing cycle comprises two total doses of the IL-22 Fc fusion protein, wherein each dose is about 60 μg/kg, and wherein the doses are administered to the subject q1w, q2w, q3w, or q4w, preferably q2w, after allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject in need thereof, wherein the IL-22 Fc fusion protein is administered to the subject in a dosing regimen comprising a dosing cycle, wherein the dosing cycle comprises three total doses of the IL-22 Fc fusion protein, wherein each dose is about 60 μg/kg, and wherein the doses are administered to the subject q1w, q2w, q3w, or q4w, preferably q2w, after allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject in need thereof, wherein the IL-22 Fc fusion protein is administered to the subject in a dosing regimen comprising a dosing cycle, wherein the dosing cycle comprises four total doses of the IL-22 Fc fusion protein, wherein each dose is about 60 μg/kg, and wherein the doses are administered to the subject q1w, q2w, q3w, or q4w, preferably q2w, after allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject in need thereof, wherein the IL-22 Fc fusion protein is administered to the subject in a dosing regimen comprising a dosing cycle, wherein the dosing cycle comprises five total doses of the IL-22 Fc fusion protein, wherein each dose is about 60 μg/kg, and wherein the doses are administered to the subject q1w, q2w, q3w, or q4w, preferably q2w, after allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject in need thereof, wherein the IL-22 Fc fusion protein is administered to the subject in a dosing regimen comprising a dosing cycle, wherein the dosing cycle comprises six total doses of the IL-22 Fc fusion protein, wherein each dose is about 60 μg/kg, and wherein the doses are administered to the subject q1w, q2w, q3w, or q4w, preferably q2w, after allo-HSCT.

In another example, provided herein is an IL-22 Fc fusion protein (e.g., as described herein, e.g., an IL-22 Fc fusion protein comprising SEQ ID NO: 8) for use in preventing acute GVHD, reducing the risk of developing chronic GVHD, or reducing the risk of corticosteroid-refractory acute GVHD in a subject wherein the IL-22 Fc fusion protein is administered to the subject in a dosing regimen comprising a dosing cycle, wherein the dosing cycle comprises up to and no more than six total doses of the IL-22 Fc fusion protein, wherein the dosing cycle comprises a first dose (C1D1) administered prior to (e.g., 1 day, 2 days, 3 days, 4 days, 5 days, 6 days, 1 week, 2 weeks or 3 weeks, prior to) allo-HSCT, and one or more further doses administered before, concurrently with, or after allo-HSCT, wherein each dose is about 60 μg/kg, and wherein the doses are administered to the subject q1w, q2w, q3w, or q4w, preferably q2w. In some aspects, a second dose (C1D2) is administered prior to (e.g., 1 day, 2 days, 3 days, 4 days, 5 days, 6 days, 1 week, 2 weeks or 3 weeks, prior to) or concurrently with allo-HSCT, and subsequent doses are administered after allo-HSCT. In some embodiments, the second dose (C1D2) is administered prior to allo-HSCT, and a third dose (C1D3) is administered prior to or concurrently with allo-HSCT, and subsequent doses are administered after allo-HSCT. In some aspects, the second dose (C1D2) and any subsequent doses are each administered after allo-HSCT.

For example, in some aspects, the dosing cycle comprises two total doses. For example, in some embodiments, the dosing cycle may include a first dose (C1D1) and a second dose (C1D2) administered prior to allo-HSCT. In some aspects, the dosing cycle may include a first dose (C1D1) administered prior to allo-HSCT and a second dose (C1D2) administered concurrently with allo-HSCT. In some aspects, the dosing cycle may include a first dose (C1D1) administered prior to allo-HSCT and a second dose (C1D2) administered after allo-HSCT. In some embodiments, the dosing cycle may include a first dose (C1D1) administered concurrently with allo-HSCT and a second dose (C1D2) administered after allo-HSCT. In some aspects, the dosing cycle may include a first dose (C1D1) administered after allo-HSCT and a second dose (C1D2) administered after allo-HSCT.

In another example, in some aspects, the dosing cycle comprises three total doses. For example, in some aspects, the dosing cycle may include a first dose (C1D1), a second dose (C1D2), and a third dose (C1D3) administered prior to allo-HSCT. In some aspects, the dosing cycle may include a first dose (C1D1) administered prior to allo-HSCT, a second dose (C1D2) administered prior to allo-HSCT, and a third dose (C1D3) administered concurrently with allo-HSCT. In some embodiments, the dosing cycle may include a first dose (C1D1) administered prior to allo-HSCT, a second dose (C1D2) administered prior to allo-HSCT, and a third dose (C1D3) administered after allo-HSCT. In some aspects, the dosing cycle may include a first dose (C1D1) administered prior to allo-HSCT, a second dose (C1D2) administered concurrently with allo-HSCT, and a third dose (C1D3) administered after allo-HSCT. In some aspects, the dosing cycle may include a first dose (C1D1) administered prior to allo-HSCT, a second dose (C1D2) administered after allo-HSCT, and a third dose (C1D3) administered after allo-HSCT. In some aspects, the dosing cycle may include a first dose (C1D1) administered concurrently with allo-HSCT, a second dose (C1D2) administered after allo-HSCT, and a third dose (C1D3) administered after allo-HSCT. In some aspects, the dosing cycle may include a first dose (C1D1) administered after allo-HSCT, a second dose (C1D2) administered after allo-HSCT, and a third dose (C1D3) administered after allo-HSCT.

In yet another example, in some aspects, the dosing cycle comprises four total doses. For example, in some aspects, the dosing cycle may include a first dose (C1D1), a second dose (C1D2), a third dose (C1D3), and a fourth dose (C1D4) administered prior to allo-HSCT. In some embodiments, the dosing cycle may include a first dose (C1D1) administered prior to allo-HSCT, a second dose (C1D2) administered prior to allo-HSCT, a third dose administered prior to allo-HSCT, and a fourth dose (C1D4) administered concurrently with allo-HSCT. In some aspects, the dosing cycle may include a first dose (C1D1) administered prior to allo-HSCT, a second dose (C1D2) administered prior to allo-HSCT, a third dose administered prior to allo-HSCT, and a fourth dose (C1D4) administered after allo-HSCT. In some aspects, the dosing cycle may include a first dose (C1D1) administered prior to allo-HSCT, a second dose (C1D2) administered prior to allo-HSCT, a third dose administered concurrently with allo-HSCT, and a fourth dose (C1D4) administered after allo-HSCT. In some aspects, the dosing cycle may include a first dose (C1D1) administered prior to allo-HSCT, a second dose (C1D2) administered prior to allo-HSCT, a third dose administered after allo-HSCT, and a fourth dose (C1D4) administered after allo-HSCT. In some aspects, the dosing cycle may include a first dose (C1D1) administered prior to allo-HSCT, a second dose (C1D2) administered concurrently with allo-HSCT, a third dose administered after allo-HSCT, and a fourth dose (C1D4) administered after allo-HSCT. In some aspects, the dosing cycle may include a first dose (C1D1) administered prior to allo-HSCT, a second dose (C1D2) administered after allo-HSCT, a third dose administered after allo-HSCT, and a fourth dose (C1D4) administered after allo-HSCT. In some aspects, the dosing cycle may include a first dose (C1D1) administered concurrently with allo-HSCT, a second dose (C1D2) administered after allo-HSCT, a third dose administered after allo-HSCT, and a fourth dose (C1D4) administered after allo-HSCT. In some aspects, the dosing cycle may include a first dose (C1D1) administered after allo-HSCT, a second dose (C1D2) administered after allo-HSCT, a third dose administered after allo-HSCT, and a fourth dose (C1D4) administered after allo-HSCT.

In a further example, in some aspects, the dosing cycle comprises five total doses. For example, in some aspects, the dosing cycle may include a first dose (C1D1), a second dose (C1D2), a third dose (C1D3), a fourth dose (C1D4), and a fifth dose (C1D5) administered prior to allo-HSCT. In some aspects, the dosing cycle may include a first dose (C1D1) administered prior to allo-HSCT, a second dose (C1D2) administered prior to allo-HSCT, a third dose (C1D3) administered prior to allo-HSCT, a fourth dose (C1D4) administered prior to allo-HSCT, and a fifth dose (C1D5) administered concurrently with allo-HSCT. In some aspects, the dosing cycle may include a first dose (C1D1) administered prior to allo-HSCT, a second dose (C1D2) administered prior to allo-HSCT, a third dose (C1D3) administered prior to allo-HSCT, a fourth dose (C1D4) administered prior to allo-HSCT, and a fifth dose (C1D5) administered after allo-HSCT. In some aspects, the dosing cycle may include a first dose (C1D1) administered prior to allo-HSCT, a second dose (C1D2) administered prior to allo-HSCT, a third dose (C1D3) administered prior to allo-HSCT, a fourth dose (C1D4) administered concurrently with allo-HSCT, and a fifth dose (C1D5) administered after allo-HSCT. In some aspects, the dosing cycle may include a first dose (C1D1) administered prior to allo-HSCT, a second dose (C1D2) administered prior to allo-HSCT, a third dose (C1D3) administered prior to allo-HSCT, a fourth dose (C1D4) administered after allo-HSCT, and a fifth dose (C1D5) administered after allo-HSCT. In some aspects, the dosing cycle may include a first dose (C1D1) administered prior to allo-HSCT, a second dose (C1D2) administered prior to allo-HSCT, a third dose (C1D3) administered concurrently with allo-HSCT, a fourth dose (C1D4) administered after allo-HSCT, and a fifth dose (C1D5) administered after allo-HSCT. In some aspects, the dosing cycle may include a first dose (C1D1) administered prior to allo-HSCT, a second dose (C1D2) administered prior to allo-HSCT, a third dose (C1D3) administered concurrently with allo-HSCT, a fourth dose (C1D4) administered after allo-HSCT, and a fifth dose (C1D5) administered after allo-HSCT. In some embodiments, the dosing cycle may include a first dose (C1D1) administered prior to allo-HSCT, a second dose (C1D2) administered prior to allo-HSCT, a third dose (C1D3) administered after allo-HSCT, a fourth dose (C1D4) administered concurrently after allo-HSCT, and a fifth dose (C1D5) administered after allo-HSCT. In some aspects, the dosing cycle may include a first dose (C1D1) administered prior to allo-HSCT, a second dose (C1D2) administered concurrently with allo-HSCT, a third dose (C1D3) administered after allo-HSCT, a fourth dose (C1D4) administered after allo-HSCT, and a fifth dose (C1D5) administered after allo-HSCT. In some aspects, the dosing cycle may include a first dose (C1D1) administered prior to allo-HSCT, a second dose (C1D2) administered after allo-HSCT, a third dose (C1D3) administered after allo-HSCT, a fourth dose (C1D4) administered after allo-HSCT, and a fifth dose (C1D5) administered after allo-HSCT. In some embodiments, the dosing cycle may include a first dose (C1D1) administered concurrently with allo-HSCT, a second dose (C1D2) administered after allo-HSCT, a third dose (C1D3) administered after allo-HSCT, a fourth dose (C1D4) administered after allo-HSCT, and a fifth dose (C1D5) administered after allo-HSCT. In some aspects, the dosing cycle may include a first dose (C1D1) administered after allo-HSCT, a second dose (C1D2) administered after allo-HSCT, a third dose (C1D3) administered after allo-HSCT, a fourth dose (C1D4) administered after allo-HSCT, and a fifth dose (C1D5) administered after allo-HSCT.

In a further example still, in some aspects, the dosing cycle comprises six total doses.

For example, in some aspects, the dosing cycle may include a first dose (C1D1), a second dose (C1D2), a third dose (C1D3), a fourth dose (C1D4), a fifth dose (C1D5), and a sixth dose (C1D6) administered prior to allo-HSCT. In some aspects, the dosing cycle may include a first dose (C1D1) administered prior to allo-HSCT, a second dose (C1D2) administered prior to allo-HSCT, a third dose (C1D3) administered prior to allo-HSCT, a fourth dose (C1D4) administered prior to allo-HSCT, a fifth dose (C1D5) administered prior to allo-HSCT, and a sixth dose (C1D6) administered concurrently with allo-HSCT. In some aspects, the dosing cycle may include a first dose (C1D1) administered prior to allo-HSCT, a second dose (C1D2) administered prior to allo-HSCT, a third dose (C1D3) administered prior to allo-HSCT, a fourth dose (C1D4) administered prior to allo-HSCT, a fifth dose (C1D5) administered prior to allo-HSCT, and a sixth dose (C1D6) administered after allo-HSCT. In some aspects, the dosing cycle may include a first dose (C1D1) administered prior to allo-HSCT, a second dose (C1D2) administered prior to allo-HSCT, a third dose (C1D3) administered prior to allo-HSCT, a fourth dose (C1D4) administered prior to allo-HSCT, a fifth dose (C1D5) administered concurrently with allo-HSCT, and a sixth dose (C1D6) administered after allo-HSCT. In some embodiments, the dosing cycle may include a first dose (C1D1) administered prior to allo-HSCT, a second dose (C1D2) administered prior to allo-HSCT, a third dose (C1D3) administered prior to allo-HSCT, a fourth dose (C1D4) administered prior to allo-HSCT, a fifth dose (C1D5) administered after allo-HSCT, and a sixth dose (C1D6) administered after allo-HSCT. In some aspects, the dosing cycle may include a first dose (C1D1) administered prior to allo-HSCT, a second dose (C1D2) administered prior to allo-HSCT, a third dose (C1D3) administered prior to allo-HSCT, a fourth dose (C1D4) administered concurrently with allo-HSCT, a fifth dose (C1D5) administered after allo-HSCT, and a sixth dose (C1D6) administered after allo-HSCT. In some aspects, the dosing cycle may include a first dose (C1D1) administered prior to allo-HSCT, a second dose (C1D2) administered prior to allo-HSCT, a third dose (C1D3) administered prior to allo-HSCT, a fourth dose (C1D4) administered after allo-HSCT, a fifth dose (C1D5) administered after allo-HSCT, and a sixth dose (C1D6) administered after allo-HSCT. In some aspects, the dosing cycle may include a first dose (C1D1) administered prior to allo-HSCT, a second dose (C1D2) administered prior to allo-HSCT, a third dose (C1D3) administered concurrently with allo-HSCT, a fourth dose (C1D4) administered after allo-HSCT, a fifth dose (C1D5) administered after allo-HSCT, and a sixth dose (C1D6) administered after allo-HSCT. In some embodiments, the dosing cycle may include a first dose (C1D1) administered prior to allo-HSCT, a second dose (C1D2) administered prior to allo-HSCT, a third dose (C1D3) administered after allo-HSCT, a fourth dose (C1D4) administered after allo-HSCT, a fifth dose (C1D5) administered after allo-HSCT, and a sixth dose (C1D6) administered after allo-HSCT. In some aspects, the dosing cycle may include a first dose (C1D1) administered prior to allo-HSCT, a second dose (C1D2) administered concurrently with allo-HSCT, a third dose (C1D3) administered after allo-HSCT, a fourth dose (C1D4) administered after allo-HSCT, a fifth dose (C1D5) administered after allo-HSCT, and a sixth dose (C1D6) administered after allo-HSCT. In some aspects, the dosing cycle may include a first dose (C1D1) administered prior to allo-HSCT, a second dose (C1D2) administered after allo-HSCT, a third dose (C1D3) administered after allo-HSCT, a fourth dose (C1D4) administered after allo-HSCT, a fifth dose (C1D5) administered after allo-HSCT, and a sixth dose (C1D6) administered after allo-HSCT. In some aspects, the dosing cycle may include a first dose (C1D1) administered concurrently with allo-HSCT, a second dose (C1D2) administered after allo-HSCT, a third dose (C1D3) administered after allo-HSCT, a fourth dose (C1D4) administered after allo-HSCT, a fifth dose (C1D5) administered after allo-HSCT, and a sixth dose (C1D6) administered after allo-HSCT. In some aspects, the dosing cycle may include a first dose (C1D1) administered after allo-HSCT, a second dose (C1D2) administered after allo-HSCT, a third dose (C1D3) administered after allo-HSCT, a fourth dose (C1D4) administered after allo-HSCT, a fifth dose (C1D5) administered after allo-HSCT, and a sixth dose (C1D6) administered after allo-HSCT.

In some aspects, the C1D1 is administered to the subject prior to allo-HSCT. In some aspects, the C1D1 is administered to the subject 1 to 3 days prior to allo-HSCT. In some embodiments, the C1D1 is administered to the subject 1 day prior to allo-HSCT. In some aspects, the one or more further doses comprise at least a second dose (C1D2). In some aspects, the one or more further doses comprise at least a C1D2 and a third dose (C1D3). In some aspects, the one or more further doses comprise at least a C1D2, a C1D3, and a fourth dose (C1D4). In some aspects, the one or more further doses comprise at least a C1D2, a C1D3, a C1D4, and a fifth dose (C1D5). In some aspects, the dosing cycle comprises the C1D1, a C1D2, a C1D3, a C1D4, a C1D5, and a sixth dose (C1D6) of the IL-22 Fc fusion protein. In some aspects, the doses are administered to the subject q2w. In some embodiments, the dosing cycle has a length of about 70 (t3) days. In someaspects, the dosing cycle has a length of about 70 days. In some aspects, the dosing cycle consists of a C1D1, a C1D2, a C1D3, a C1D4, a C1D5, and a C1D6, and wherein the C1D1 is administered to the subject 1 day prior to allo-HSCT, the C1D2 is administered to the subject 13 days after allo-HSCT, the C1D3 is administered to the subject 27 days after allo-HSCT, the C1D4 is administered to the subject 41 days after allo-HSCT, the C1D5 is administered to the subject 55 days after allo-HSCT, and the C1D6 is administered to the subject 69 days after allo-HSCT.

In another example, provided herein is an IL-22 Fc fusion protein (e.g., as described herein, e.g., an IL-22 Fc fusion protein comprising SEQ ID NO: 8) for use in preventing acute GVHD, reducing the risk of developing chronic GVHD, or reducing the risk of corticosteroid-refractory acute GVHD in a subject comprising administering to a subject in need thereof an IL-22 Fc fusion protein in a dosing regimen comprising a dosing cycle, wherein the dosing cycle comprises up to and no more than seven total doses of the IL-22 Fc fusion protein, wherein the dosing cycle comprises a first dose (C1D1) administered prior to (e.g., 1 day, 2 days, 3 days, 4 days, 5 days, 6 days, 1 week, 2 weeks or 3 weeks, prior to) allo-HSCT, and one or more further doses administered before, concurrently with, or after allo-HSCT, wherein each dose is about 60 $\mu$g/kg, and wherein the doses are administered to the subject q1w, q2w, q3w, or q4w, preferably q2w. In some aspects the second dose C1 D2 is administered prior to or concurrently with allo-HSCT, and subsequent doses are administered after allo-HSCT. In some aspects the second dose C1D2 is administered prior to allo-HSCT, and the third dose is administered prior to or concurrently with allo-HSCT, and subsequent doses are administered after allo-HSCT. In some aspects the second dose C1D2 and any subsequent doses are each administered after allo-HSCT.

I

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject comprising administering to a subject in need thereof an IL-22 Fc fusion protein in a dosing regimen comprising a dosing cycle, wherein the dosing cycle comprises up to and no more than eight total doses of the IL-22 Fc fusion protein, wherein the dosing cycle comprises a first dose (C1 D1) administered prior to (e.g., 1 day, 2 days, 3 days, 4 days, 5 days, 6 days, 1 week, 2 weeks or 3 weeks, prior to) allo-HSCT, and one or more further doses administered before, concurrently with, or after allo-HSCT, wherein each dose is about 60 $\mu$g/kg, and wherein the doses are administered to the subject q1w, q2w, q3w, or q4w, preferably q2w. In some aspects the second dose C1 D2 is administered prior to or concurrently with allo-HSCT, and subsequent doses are administered after allo-HSCT. In some aspects the second dose C1 D2 is administered prior to allo-HSCT, and the third dose is administered prior to or concurrently with allo-HSCT, and subsequent doses are administered after allo-HSCT. In some aspects the second dose C1D2 and any subsequent doses are each administered after allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises up to and no more than six doses of the IL-22 Fc fusion protein, wherein each dose is 30 $\mu$g/kg, and wherein the doses are administered to the subject q1w, q2w ,q3w, or q4w.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises one total dose of the IL-22 Fc fusion protein of about 30 $\mu$g/kg.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises one total dose of the IL-22 Fc fusion protein of about 60 $\mu$g/kg.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises one total dose of the IL-22 Fc fusion protein of about 90 $\mu$g/kg.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises one total dose of the IL-22 Fc fusion protein of about 120 $\mu$g/kg.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a C1D1, and at least one further dose, wherein the dosing cycle comprises up to and no more than six doses of the IL-22 Fc fusion protein, wherein each dose is 30 $\mu$g/kg, and wherein the doses are administered to the subject q1w, q2w ,q3w, or q4w.

For example, in some aspects, the dosing cycle comprises two total doses.

In another example, in some aspects, the dosing cycle comprises three total doses.

In yet another example, in some aspects, the dosing cycle comprises four total doses.

In a further example, in some aspects, the dosing cycle comprises five total doses.

In a further example still, in some aspects, the dosing cycle comprises six total doses.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises two total doses of the IL-22 Fc fusion protein, wherein each dose is about 30 $\mu$g/kg.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises three total doses of the IL-22 Fc fusion protein, wherein each dose is about 30 μg/kg.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises four total doses of the IL-22 Fc fusion protein, wherein each dose is about 30 μg/kg.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises five total doses of the IL-22 Fc fusion protein, wherein each dose is about 30 μg/kg.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises six total doses of the IL-22 Fc fusion protein, wherein each dose is about 30 μg/kg.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises two total doses of the IL-22 Fc fusion protein, wherein each dose is about 60 μg/kg.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises three total doses of the IL-22 Fc fusion protein, wherein each dose is about 60 μg/kg.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises four total doses of the IL-22 Fc fusion protein, wherein each dose is about 60 μg/kg.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises five total doses of the IL-22 Fc fusion protein, wherein each dose is about 60 μg/kg.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises six total doses of the IL-22 Fc fusion protein, wherein each dose is about 60 μg/kg.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises two total doses of the IL-22 Fc fusion protein, wherein each dose is about 90

μg/kg.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises three total doses of the IL-22 Fc fusion protein, wherein each dose is about 90 μg/kg.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises four total doses of the IL-22 Fc fusion protein, wherein each dose is about 90 μg/kg.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises five total doses of the IL-22 Fc fusion protein, wherein each dose is about 90 μg/kg.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises six total doses of the IL-22 Fc fusion protein, wherein each dose is about 90 μg/kg.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises two total doses of the IL-22 Fc fusion protein, wherein each dose is about 120 μg/kg.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises three total doses of the IL-22 Fc fusion protein, wherein each dose is about 120 μg/kg.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises four total doses of the IL-22 Fc fusion protein, wherein each dose is about 120 μg/kg.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises five total doses of the IL-22 Fc fusion protein, wherein each dose is about 120 μg/kg.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises six total doses of the IL-22 Fc fusion protein, wherein each dose is about 120 μg/kg.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises two total doses of the IL-22 Fc fusion protein, wherein each dose is about 30 μg/kg, and wherein the doses are administered to the subject q1w, q2w, q3w, or q4w.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises three total doses of the IL-22 Fc fusion protein, wherein each dose is about 30 μg/kg, and wherein the doses are administered to the subject q1w, q2w, q3w, or q4w.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises four total doses of the IL-22 Fc fusion protein, wherein each dose is about 30 μg/kg, and wherein the doses are administered to the subject q1w, q2w, q3w, or q4w.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises five total doses of the IL-22 Fc fusion protein, wherein each dose is about 30 μg/kg, and wherein the doses are administered to the subject q1w, q2w, q3w, or q4w.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises six total doses of the IL-22 Fc fusion protein, wherein each dose is about 30 μg/kg, and wherein the doses are administered to the subject q1w, q2w, q3w, or q4w.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises two total doses of the IL-22 Fc fusion protein, wherein each dose is about 60 μg/kg, and wherein the doses are administered to the subject q1w, q2w, q3w, or q4w.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises three total doses of the IL-22 Fc fusion protein, wherein each dose is about 60 μg/kg, and wherein the doses are administered to the subject q1w, q2w, q3w, or q4w.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises four total doses of the IL-22 Fc fusion protein, wherein each dose is about 60 μg/kg, and wherein the doses are administered to the subject q1w, q2w, q3w, or q4w.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises five total doses of the IL-22 Fc fusion protein, wherein each dose is about 60 μg/kg, and wherein the doses are administered to the subject q1w, q2w, q3w, or q4w.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises six total doses of the IL-22 Fc fusion protein, wherein each dose is about 60 μg/kg, and wherein the doses are administered to the subject q1w, q2w, q3w, or q4w.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises two total doses of the IL-22 Fc fusion protein, wherein each dose is about 90 μg/kg, and wherein the doses are administered to the subject q1w, q2w, q3w, or q4w.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises three total doses of the IL-22 Fc fusion protein, wherein each dose is about 90 μg/kg, and wherein the doses are administered to the subject q1w, q2w, q3w, or q4w.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises four total doses of the IL-22 Fc fusion protein, wherein each dose is about 90 μg/kg, and wherein the doses are administered to the subject q1w, q2w, q3w, or q4w.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises five total doses of the IL-22 Fc fusion protein, wherein each dose is about 90 μg/kg, and wherein the doses are administered to the subject q1w, q2w, q3w, or q4w.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises six total doses of the IL-22 Fc fusion protein, wherein each dose is about 90 μg/kg, and wherein the doses are administered to the subject q1w, q2w, q3w, or q4w.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises two total doses of the IL-22 Fc fusion protein, wherein each dose is about 120 μg/kg, and wherein the doses are administered to the subject q1w, q2w, q3w, or q4w.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises three total doses of the IL-22 Fc fusion protein, wherein each dose is about 120 μg/kg, and wherein the doses are administered to the subject q1w, q2w, q3w, or q4w.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises four total doses of the IL-22 Fc fusion protein, wherein each dose is about 120 μg/kg, and wherein the doses are administered to the subject q1w, q2w, q3w, or q4w.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises five total doses of the IL-22 Fc fusion protein, wherein each dose is about 120 μg/kg, and wherein the doses are administered to the subject q1w, q2w, q3w, or q4w.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises six total doses of the IL-22 Fc fusion protein, wherein each dose is about 120 µg/kg, and wherein the doses are administered to the subject q1w, q2w, q3w, or q4w.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject comprising administering to a subject in need thereof an IL-22 Fc fusion protein in a dosing regimen comprising a dosing cycle, wherein the dosing cycle comprises up to and no more than eight total doses of the IL-22 Fc fusion protein, wherein the dosing cycle comprises a first dose (C1 D1) administered prior to (e.g., 1 day, 2 days, 3 days, 4 days, 5 days, 6 days, 1 week, 2 weeks or 3 weeks, prior to) allo-HSCT, and one or more further doses administered before, concurrently with, or after allo-HSCT, wherein each dose is about 30 µg/kg, and wherein the doses are administered to the subject q1w, q2w, q3w, or q4w, preferably q2w or q4w. In some aspects, a second dose (C1D2) is administered prior to or concurrently with allo-HSCT, and subsequent doses are administered after allo-HSCT. In some aspects, the second dose (C1 D2) is administered prior to allo-HSCT, and a third dose (C1 D3) is administered prior to or concurrently with allo-HSCT, and subsequent doses are administered after allo-HSCT. In some aspects, the second dose (C1 D2) and any subsequent doses are each administered after allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD, reducing the risk of developing chronic GVHD, or reducing the risk of corticosteroid-refractory acute GVHD in a subject comprising administering to a subject in need thereof an IL-22 Fc fusion protein in a dosing regimen comprising a dosing cycle, wherein the dosing cycle comprises up to and no more than eight total doses of the IL-22 Fc fusion protein, wherein the dosing cycle comprises a first dose (C1D1) administered prior to (e.g., 1 day, 2 days, 3 days, 4 days, 5 days, 6 days, 1 week, 2 weeks or 3 weeks, prior to) allo-HSCT, and one or more further doses administered before, concurrently with, or after allo-HSCT, wherein each dose is about 60 µg/kg, and wherein the doses are administered to the subject q1w, q2w, q3w, or q4w, preferably q2w. In some aspects, the second dose (C1D2) is administered prior to or concurrently with allo-HSCT, and subsequent doses are administered after allo-HSCT. In some aspects, the second dose (C1D2) is administered prior to allo-HSCT, and a third dose (C1D3) is administered prior to or concurrently with allo-HSCT, and subsequent doses are administered after allo-HSCT. In some aspects, the second dose (C1D2) and any subsequent doses are each administered after allo-HSCT.

In some aspects, the C1 D1 is administered to the subject 1 to 3 days prior to allo-HSCT. In some aspects, the C1D1 is administered to the subject 1 day prior to allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject comprising administering to a subject in need thereof an IL-22 Fc fusion protein in a dosing regimen comprising a dosing cycle, wherein the dosing cycle comprises up to and no more than six total doses of the IL-22 Fc fusion protein, wherein the dosing cycle comprises a first dose (C1D1) administered 1 day prior to allo-HSCT, and one or more further doses administered before, concurrently with, or after allo-HSCT, wherein each dose is about 30 µg/kg, and wherein the doses are administered to the subject q2w. In some aspects, a second dose (C1D2) is administered prior to or concurrently with allo-HSCT, and subsequent doses are administered after allo-HSCT. In some embodiments, the second dose (C1D2) is administered prior to allo-HSCT, and a third dose (C1D3) is administered prior to or concurrently with allo-HSCT, and subsequent doses are administered after allo-HSCT. In some aspects, the second dose (C1D2) and any subsequent doses are each administered after allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject comprising administering to a subject in need thereof an IL-22 Fc fusion protein in a dosing regimen comprising a dosing cycle, wherein the dosing cycle comprises up to and no more than six total doses of the IL-22 Fc fusion protein, wherein the dosing cycle comprises a first dose (C1D1) administered 1 day prior to allo-HSCT, and one or more further doses administered before, concurrently with, or after allo-HSCT, wherein each dose is about 30 µg/kg, and wherein the doses are administered to the subject q4w. In some aspects, a second dose (C1D2) is administered prior to or concurrently with allo-HSCT, and subsequent doses are administered after allo-HSCT. In some aspects, the second dose (C1D2) is administered prior to allo-HSCT, and a third dose (C1D3) is administered prior to or concurrently with allo-HSCT, and subsequent doses are administered after allo-HSCT. In some aspects, the second dose (C1D2) and any subsequent doses are each administered after allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject comprising administering to a subject in need thereof an IL-22 Fc fusion protein in a dosing regimen comprising a dosing cycle, wherein the dosing cycle comprises up to and no more than six total doses of the IL-22 Fc fusion protein, wherein the dosing cycle comprises a first dose (C1D1) administered 1 day prior to allo-HSCT, and one or more further doses administered before, concurrently with, or after allo-HSCT, wherein each dose is about 60 µg/kg, and wherein the doses are administered to the subject q2w. In some aspects, a second dose (C1D2) is administered prior to or concurrently with allo-HSCT, and subsequent doses are administered after allo-HSCT. In some aspects, the second dose (C1D2) is administered prior to allo-HSCT, and a third dose (C1D3) is administered prior to or concurrently with allo-HSCT, and subsequent doses are administered after allo-HSCT. In some aspects, the second dose (C1D2) and any subsequent doses are each administered after allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject comprising administering to a subject in need thereof an IL-22 Fc fusion protein in a dosing regimen comprising a dosing cycle, wherein the dosing cycle comprises up to and no more than six total doses of the IL-22 Fc fusion protein, wherein the dosing cycle comprises a first dose (C1D1) administered 1 day prior to allo-HSCT, and one or more further doses administered before, concurrently with, or after allo-HSCT, wherein each dose is about 90 μg/kg, and wherein the doses are administered to the subject q2w. In some aspects, the second dose (C1D2) is administered prior to or concurrently with allo-HSCT, and subsequent doses are administered after allo-HSCT. In some aspects, the second dose (C1D2) is administered prior to allo-HSCT, and a third dose (C1D3) is administered prior to or concurrently with allo-HSCT, and subsequent doses are administered after allo-HSCT. In some aspects the second dose C1D2 and any subsequent doses are each administered after allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject comprising administering to a subject in need thereof an IL-22 Fc fusion protein in a dosing regimen comprising a dosing cycle, wherein the dosing cycle comprises up to and no more than six total doses of the IL-22 Fc fusion protein, wherein the dosing cycle comprises a first dose (C1D1) administered 1 day prior to allo-HSCT, and one or more further doses administered before, concurrently with, or after allo-HSCT, wherein each dose is about 120 μg/kg, and wherein the doses are administered to the subject q2w. In some aspects, a second dose (C1D2) is administered prior to or concurrently with allo-HSCT, and subsequent doses are administered after allo-HSCT. In some aspects, the second dose (C1D2) is administered prior to allo-HSCT, and a third dose (C1D3) is administered prior to or concurrently with allo-HSCT, and subsequent doses are administered after allo-HSCT. In some aspects, the second dose (C1D2) and any subsequent doses are each administered after allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject comprising administering to a subject in need thereof an IL-22 Fc fusion protein in a dosing regimen comprising a dosing cycle, wherein the dosing cycle comprises up to and no more than six total doses of the IL-22 Fc fusion protein, wherein the dosing cycle comprises a first dose (C1D1) administered 1 day prior to allo-HSCT, and one or more further doses administered before, concurrently with, or after allo-HSCT, wherein each dose is about 30 μg/kg, and wherein the doses are administered to the subject q2w. In some aspects, a second dose (C1D2) is administered prior to or concurrently with allo-HSCT, and subsequent doses are administered after allo-HSCT. In some aspects, the second dose (C1D2) is administered prior to allo-HSCT, and a third dose (C1D3) is administered prior to or concurrently with allo-HSCT, and subsequent doses are administered after allo-HSCT. In some aspects, the second dose (C1D2) and any subsequent doses are each administered after allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject comprising administering to a subject in need thereof an IL-22 Fc fusion protein in a dosing regimen comprising a dosing cycle, wherein the dosing cycle comprises up to and no more than six total doses of the IL-22 Fc fusion protein, wherein the dosing cycle comprises a first dose (C1D1) administered 1 day prior to allo-HSCT, and one or more further doses administered before, concurrently with, or after allo-HSCT, wherein each dose is about 30 μg/kg, and wherein the doses are administered to the subject q4w. In some aspects, a second dose (C1D2) is administered prior to or concurrently with allo-HSCT, and subsequent doses are administered after allo-HSCT. In some aspects, the second dose (C1D2) is administered prior to allo-HSCT, and a third dose (C1D3) is administered prior to or concurrently with allo-HSCT, and subsequent doses are administered after allo-HSCT. In some aspects, the second dose (C1D2) and any subsequent doses are each administered after allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject comprising administering to a subject in need thereof an IL-22 Fc fusion protein in a dosing regimen comprising a dosing cycle, wherein the dosing cycle comprises up to and no more than six total doses of the IL-22 Fc fusion protein, wherein the dosing cycle comprises a first dose (C1D1) administered 1 day prior to allo-HSCT, and one or more further doses administered before, concurrently with, or after allo-HSCT, wherein each dose is about 60 μg/kg, and wherein the doses are administered to the subject q2w. In some aspects, a second dose (C1D2) is administered prior to or concurrently with allo-HSCT, and subsequent doses are administered after allo-HSCT. In some aspects, the second dose (C1D2) is administered prior to allo-HSCT, and a third dose (C1D3) is administered prior to or concurrently with allo-HSCT, and subsequent doses are administered after allo-HSCT. In some aspects, the second dose (C1D2) and any subsequent doses are each administered after allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject comprising administering to a subject in need thereof an IL-22 Fc fusion protein in a dosing regimen comprising a dosing cycle, wherein the dosing cycle comprises up to and no more than six total doses of the IL-22 Fc fusion protein, wherein the dosing cycle comprises a first dose (C1D1) administered 1 day prior to allo-HSCT, and one or more further doses administered before, concurrently with, or after allo-HSCT, wherein each dose is about 90 $\mu$g/kg, and wherein the doses are administered to the subject q2w. In some aspects, a second dose (C1D2) is administered prior to or concurrently with allo-HSCT, and subsequent doses are administered after allo-HSCT. In some aspects, the second dose (C1D2) is administered prior to allo-HSCT, and a third dose (C1D3) is administered prior to or concurrently with allo-HSCT, and subsequent doses are administered after allo-HSCT. In some aspects, the second dose (C1D2) and any subsequent doses are each administered after allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject comprising administering to a subject in need thereof an IL-22 Fc fusion protein in a dosing regimen comprising a dosing cycle, wherein the dosing cycle comprises up to and no more than six total doses of the IL-22 Fc fusion protein, wherein the dosing cycle comprises a first dose (C1D1) administered 1 day prior to allo-HSCT, and one or more further doses administered before, concurrently with, or after allo-HSCT, wherein each dose is about 120 $\mu$g/kg, and wherein the doses are administered to the subject q2w. In some aspects, a second dose (C1D2) is administered prior to or concurrently with allo-HSCT, and subsequent doses are administered after allo-HSCT. In some aspects, the second dose (C1D2) is administered prior to allo-HSCT, and a third dose (C1D3) is administered prior to or concurrently with allo-HSCT, and subsequent doses are administered after allo-HSCT. In some aspects, the second dose (C1D2) and any subsequent doses are each administered after allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject comprising administering to a subject in need thereof an IL-22 Fc fusion protein in a dosing regimen comprising a dosing cycle, wherein the dosing cycle comprises up to and no more than six total doses of the IL-22 Fc fusion protein, wherein the dosing cycle comprises a first dose (C1D1) administered 1 day prior to allo-HSCT, and one or more further doses administered before, concurrently with, or after allo-HSCT, wherein each dose is about 30 $\mu$g/kg, and wherein the doses are administered to the subject q2w. In some aspects, a second dose (C1D2) is administered prior to or concurrently with allo-HSCT, and subsequent doses are administered after allo-HSCT. In some aspects, the second dose (C1D2) is administered prior to allo-HSCT, and a third dose (C1D3) is administered prior to or concurrently with allo-HSCT, and subsequent doses are administered after allo-HSCT. In some aspects, the second dose (C1D2) and any subsequent doses are each administered after allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in reducing the reducing the risk of corticosteroid-refractory acute GVHD in a subject comprising administering to a subject in need thereof an IL-22 Fc fusion protein in a dosing regimen comprising a dosing cycle, wherein the dosing cycle comprises up to and no more than six total doses of the IL-22 Fc fusion protein, wherein the dosing cycle comprises a first dose (C1D1) administered 1 day prior to allo-HSCT, and one or more further doses administered before, concurrently with, or after allo-HSCT, wherein each dose is about 30 $\mu$g/kg, and wherein the doses are administered to the subject q4w. In some aspects, a second dose (C1D2) is administered prior to or concurrently with allo-HSCT, and subsequent doses are administered after allo-HSCT. In some aspects, the second dose (C1D2) is administered prior to allo-HSCT, and a third dose (C1D3) is administered prior to or concurrently with allo-HSCT, and subsequent doses are administered after allo-HSCT. In some aspects, the second dose (C1D2) and any subsequent doses are each administered after allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject comprising administering to a subject in need thereof an IL-22 Fc fusion protein in a dosing regimen comprising a dosing cycle, wherein the dosing cycle comprises up to and no more than six total doses of the IL-22 Fc fusion protein, wherein the dosing cycle comprises a first dose (C1D1) administered 1 day prior to allo-HSCT, and one or more further doses administered before, concurrently with, or after allo-HSCT, wherein each dose is about 60 $\mu$g/kg, and wherein the doses are administered to the subject q2w. In some aspects, a second dose (C1D2) is administered prior to or concurrently with allo-HSCT, and subsequent doses are administered after allo-HSCT. In some aspects, the second dose (C1D2) is administered prior to allo-HSCT, and a third dose (C1D3) is administered prior to or concurrently with allo-HSCT, and subsequent doses are administered after allo-HSCT. In some aspects, the second dose (C1D2) and any subsequent doses are each administered after allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject comprising administering to a subject in need thereof an IL-22 Fc fusion protein in a dosing regimen comprising a dosing cycle, wherein the dosing cycle comprises up to and no more than six total doses of the IL-22 Fc fusion protein, wherein the dosing cycle comprises a first dose (C1D1) administered 1 day prior to allo-HSCT, and one or more further doses administered before, concurrently with, or after allo-HSCT, wherein each dose is about 90 µg/kg, and wherein the doses are administered to the subject q2w. In some aspects, a second dose (C1D2) is administered prior to or concurrently with allo-HSCT, and subsequent doses are administered after allo-HSCT. In some aspects, the second dose (C1D2) is administered prior to allo-HSCT, and a third dose (C1D3) is administered prior to or concurrently with allo-HSCT, and subsequent doses are administered after allo-HSCT. In some aspects, the second dose (C1D2) and any subsequent doses are each administered after allo-HSCT.

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject comprising administering to a subject in need thereof an IL-22 Fc fusion protein in a dosing regimen comprising a dosing cycle, wherein the dosing cycle comprises up to and no more than six total doses of the IL-22 Fc fusion protein, wherein the dosing cycle comprises a first dose (C1D1) administered 1 day prior to allo-HSCT, and one or more further doses administered before, concurrently with, or after allo-HSCT, wherein each dose is about 120 µg/kg, and wherein the doses are administered to the subject q2w. In some aspects, a second dose (C1D2) is administered prior to or concurrently with allo-HSCT, and subsequent doses are administered after allo-HSCT. In some aspects, the second dose (C1D2) is administered prior to allo-HSCT, and a third dose (C1D3) is administered prior to or concurrently with allo-HSCT, and subsequent doses are administered after allo-HSCT. In some aspects, the second dose (C1D2) and any subsequent doses are each administered after allo-HSCT.

In some examples of any of the preceding aspects, the dosing cycle comprises two total doses.

In some examples of any of the preceding aspects, the dosing cycle comprises three total doses.

In some examples of any of the preceding aspects, the dosing cycle comprises four total doses.

In some examples of any of the preceding aspects, the dosing cycle comprises five total doses.

In some examples of any of the preceding aspects, the dosing cycle comprises six total doses. In some aspects, the one or more further doses comprise at least a second dose (C1D2). In some aspects, the dosing cycle comprises the C1D1, a C1D2, and a third dose (C1D3). In some aspects, the one or more further doses comprise at least a C1D2 and a third dose (C1D3). In some aspects, the one or more further doses comprise at least a C1D2, a C1D3, and a fourth dose (C1D4). In some aspects, the one or more further doses comprise at least a C1D2, a C1D3, a C1D4, and a fifth dose (C1D5). In some aspects, the dosing cycle comprises the C1D1, a C1D2, a C1D3, a C1D4, a C1D5, and a sixth dose (C1D6) of the IL-22 Fc fusion protein.

In some aspects, the doses are administered to the subject q2w. In some aspects, the dosing cycle has a length of about 70 (t3) days. In some aspects, the dosing cycle has a length of about 70 days. In some embodiments, the dosing cycle consists of a C1D1, a C1D2, a C1D3, a C1D4, a C1D5, and a C1D6, and wherein the C1D1 is administered to the subject 1 day prior to allo-HSCT, the C1D2 is administered to the subject 13 days after allo-HSCT, the C1D3 is administered to the subject 27 days after allo-HSCT, the C1D4 is administered to the subject 41 days after allo-HSCT, the C1D5 is administered to the subject 55 days after allo-HSCT, and the C1D6 is administered to the subject 69 days after allo-HSCT.

In other aspects, the doses are administered to the subject q4w. In some aspects, the dosing cycle has a length of about 55 (t3) days. In some aspects, the dosing cycle has a length of about 55 days. In some embodiments, the dosing cycle consists of a C1D1, a C1D2, and a C1D3, and wherein the C1D1 is administered to the subject 1 day prior to allo-HSCT, the C1D2 is administered to the subject 27 days after allo-HSCT, and the C1D3 is administered to the subject 55 days after allo-HSCT.

In some aspects, the C1D1 is administered to the subject after allo-HSCT. In some aspects, the C1D1 is administered to the subject 1 to 3 days after allo-HSCT. In some aspects, the C1D1 is administered to the subject within 2 days of allo-HSCT. In some aspects, the C1D1 is administered to the subject one day after allo-HSCT.

In another example, provided herein is an IL-22 Fc fusion protein for use in preventing acute GVHD in a subject, wherein the IL-22 Fc fusion protein is for administration to a subject in need thereof in a dosing regimen comprising a dosing cycle, wherein the dosing cycle comprises up to and no more than six total doses of the IL-22 Fc fusion protein, wherein the dosing cycle comprises a first dose (C1D1) and one or more further doses of the IL-22 Fc fusion protein, wherein each dose is between about 30 µg/kg and 90 µg/kg, and wherein the doses are administered to the subject q1w, q2w, q3w, or q4w, preferably q2w.

In another example, provided herein is an IL-22 Fc fusion protein for use in preventing acute GVHD in a subject, wherein the IL-22 Fc fusion protein is for administration to a subject in need thereof in a dosing regimen comprising a dosing cycle, wherein the dosing cycle comprises two total doses of the IL-22 Fc fusion protein, wherein each dose is between about 30 µg/kg and 90 µg/kg, and wherein the doses are administered to the subject q1w, q2w, q3w, or q4w, preferably q2w.

In another example, provided herein is an IL-22 Fc fusion protein for use in preventing acute GVHD in a subject,

wherein the IL-22 Fc fusion protein is for administration to a subject in need thereof in a dosing regimen comprising a dosing cycle, wherein the dosing cycle comprises three total doses of the IL-22 Fc fusion protein, wherein each dose is between about 30 μg/kg and 90 μg/kg, and wherein the doses are administered to the subject q1w, q2w, q3w, or q4w, preferably q2w.

In another example, provided herein is an IL-22 Fc fusion protein for use in preventing acute GVHD in a subject, wherein the IL-22 Fc fusion protein is for administration to a subject in need thereof in a dosing regimen comprising a dosing cycle, wherein the dosing cycle comprises four total doses of the IL-22 Fc fusion protein, wherein each dose is between about 30 μg/kg and 90 μg/kg, and wherein the doses are administered to the subject q1w, q2w, q3w, or q4w, preferably q2w.

In another example, provided herein is an IL-22 Fc fusion protein for use in preventing acute GVHD in a subject, wherein the IL-22 Fc fusion protein is for administration to a subject in need thereof in a dosing regimen comprising a dosing cycle, wherein the dosing cycle comprises five total doses of the IL-22 Fc fusion protein, wherein each dose is between about 30 μg/kg and 90 μg/kg, and wherein the doses are administered to the subject q1w, q2w, q3w, or q4w, preferably q2w.

In another example, provided herein is an IL-22 Fc fusion protein for use in preventing acute GVHD in a subject, wherein the IL-22 Fc fusion protein is for administration to a subject in need thereof in a dosing regimen comprising a dosing cycle, wherein the dosing cycle comprises six total doses of the IL-22 Fc fusion protein, wherein each dose is between about 30 μg/kg and 90 μg/kg, and wherein the doses are administered to the subject q1w, q2w, q3w, or q4w, preferably q2w.

In another example, provided herein is an IL-22 Fc fusion protein for use in preventing acute GVHD in a subject, wherein the IL-22 Fc fusion protein is for administration to a subject in need thereof in a dosing regimen comprising a dosing cycle, wherein the dosing cycle comprises up to and no more than six total doses of the IL-22 Fc fusion protein, wherein the dosing cycle comprises a first dose (C1D1) and one or more further doses of the IL-22 Fc fusion protein, wherein each dose is between about 30 μg/kg and 120 μg/kg, about 30 μg/kg and 90 μg/kg, about 30 μg/kg and 60 μg/kg, about 60 μg/kg and 90 μg/kg, or about 60 μg/kg and 120 μg/kg, wherein the doses are administered to the subject q1w, q2w, q3w, or q4w, preferably q2w.

In another example, provided herein is an IL-22 Fc fusion protein for use in preventing acute GVHD in a subject, wherein the IL-22 Fc fusion protein is for administration to a subject in need thereof in a dosing regimen comprising a dosing cycle, wherein the dosing cycle comprises two total doses of the IL-22 Fc fusion protein, wherein each dose is between about 30 μg/kg and 120 μg/kg, about 30 μg/kg and 90 μg/kg, about 30 μg/kg and 60 μg/kg, about 60 μg/kg and 90 μg/kg, or about 60 μg/kg and 120 μg/kg, wherein the doses are administered to the subject q1w, q2w, q3w, or q4w, preferably q2w.

In another example, provided herein is an IL-22 Fc fusion protein for use in preventing acute GVHD in a subject, wherein the IL-22 Fc fusion protein is for administration to a subject in need thereof in a dosing regimen comprising a dosing cycle, wherein the dosing cycle comprises three total doses of the IL-22 Fc fusion protein, wherein each dose is between about 30 μg/kg and 120 μg/kg, about 30 μg/kg and 90 μg/kg, about 30 μg/kg and 60 μg/kg, about 60 μg/kg and 90 μg/kg, or about 60 μg/kg and 120 μg/kg, wherein the doses are administered to the subject q1w, q2w, q3w, or q4w, preferably q2w.

In another example, provided herein is an IL-22 Fc fusion protein for use in preventing acute GVHD in a subject, wherein the IL-22 Fc fusion protein is for administration to a subject in need thereof in a dosing regimen comprising a dosing cycle, wherein the dosing cycle comprises four total doses of the IL-22 Fc fusion protein, wherein each dose is between about 30 μg/kg and 120 μg/kg, about 30 μg/kg and 90 μg/kg, about 30 μg/kg and 60 μg/kg, about 60 μg/kg and 90 μg/kg, or about 60 μg/kg and 120 μg/kg, wherein the doses are administered to the subject q1w, q2w, q3w, or q4w, preferably q2w.

In another example, provided herein is an IL-22 Fc fusion protein for use in preventing acute GVHD in a subject, wherein the IL-22 Fc fusion protein is for administration to a subject in need thereof in a dosing regimen comprising a dosing cycle, wherein the dosing cycle comprises five total doses of the IL-22 Fc fusion protein, wherein each dose is between about 30 μg/kg and 120 μg/kg, about 30 μg/kg and 90 μg/kg, about 30 μg/kg and 60 μg/kg, about 60 μg/kg and 90 μg/kg, or about 60 μg/kg and 120 μg/kg, wherein the doses are administered to the subject q1w, q2w, q3w, or q4w, preferably q2w.

In another example, provided herein is an IL-22 Fc fusion protein for use in preventing acute GVHD in a subject, wherein the IL-22 Fc fusion protein is for administration to a subject in need thereof in a dosing regimen comprising a dosing cycle, wherein the dosing cycle comprises six total doses of the IL-22 Fc fusion protein, wherein each dose is between about 30 μg/kg and 120 μg/kg, about 30 μg/kg and 90 μg/kg, about 30 μg/kg and 60 μg/kg, about 60 μg/kg and 90 μg/kg, or about 60 μg/kg and 120 μg/kg, wherein the doses are administered to the subject q1w, q2w, q3w, or q4w, preferably q2w.

In another example, provided herein is an IL-22 Fc fusion protein for use in preventing acute GVHD in a subject, wherein the IL-22 Fc fusion protein is for administration to a subject in need thereof in a dosing regimen comprising a dosing cycle, wherein the dosing cycle consists of a first dose (C1D1), a second dose (C1D2), a third dose (C1D3), a fourth dose (C1D4), a fifth dose (C1 D5), and a sixth dose (C1D6) of the IL-22 Fc fusion protein, wherein the C1D1, the C1D2, the C1D3, the C1D4, the C1D5, and the C1D6 are each between about 30 μg/kg and 120 μg/kg, about 30 μg/kg and 90 μg/kg, about 30 μg/kg and 60 μg/kg, about 60 μg/kg and 90 μg/kg, or about 60 μg/kg and 120 μg/kg, wherein the doses are administered to the subject q1w, q2w, q3w, or q4w, preferably q2w

In another example, provided herein is an IL-22 Fc fusion protein for use in preventing acute GVHD in a subject, wherein the IL-22 Fc fusion protein is for administration to a subject in need thereof in a dosing regimen comprising a dosing cycle, wherein the dosing cycle comprises up to and no more than six total doses of the IL-22 Fc fusion protein, wherein the dosing cycle comprises a first dose (C1D1) and one or more further doses of the IL-22 Fc fusion protein, wherein each dose is about 60 μg/kg, and wherein the doses are administered to the subject q1w, q2w, q3w, or q4w, preferably q2w.

In another example, provided herein is an IL-22 Fc fusion protein for use in preventing acute GVHD in a subject, wherein the IL-22 Fc fusion protein is for administration to a subject in need thereof in a dosing regimen comprising a dosing cycle, wherein the dosing cycle consists of a first dose (C1D1), a second dose (C1D2), a third dose (C1D3), a fourth dose (C1D4), a fifth dose (C1 D5), and a sixth dose (C1D6) of the IL-22 Fc fusion protein, wherein the C1D1, the C1D2, the C1D3, the C1D4, the C1D5, and the C1D6 are each about 60 μg/kg, wherein the doses are administered to the subject q1w, q2w, q3w, or q4w, preferably q2w.

In another example, provided herein is an IL-22 Fc fusion protein for use in reducing the risk of developing chronic GVHD in a subject, wherein the IL-22 Fc fusion protein is for administration to a subject in need thereof in a dosing regimen comprising a dosing cycle, wherein the dosing cycle comprises up to and no more than six total doses of the IL-22 Fc fusion protein, wherein the dosing cycle comprises a first dose (C1 D1) and one or more further doses of the IL-22 Fc fusion protein, wherein each dose is between about 30 μg/kg and 120 μg/kg, about 30 μg/kg and 90 μg/kg, about 30 μg/kg and 60 μg/kg, about 60 μg/kg and 90 μg/kg, or about 60 μg/kg and 120 μg/kg, and wherein the doses are administered to the subject q1w, q2w, q3w, or q4w, preferably q2w.

In another example, provided herein is an IL-22 Fc fusion protein for use in reducing the risk of developing chronic GVHD in a subject, wherein the IL-22 Fc fusion protein is for administration to a subject in need thereof in a dosing regimen comprising a dosing cycle, wherein the dosing cycle comprises two total doses of the IL-22 Fc fusion protein, wherein each dose is between about 30 μg/kg and 120 μg/kg, about 30 μg/kg and 90 μg/kg, about 30 μg/kg and 60 μg/kg, about 60 μg/kg and 90 μg/kg, or about 60 μg/kg and 120 μg/kg, wherein the doses are administered to the subject q1w, q2w, q3w, or q4w, preferably q2w.

In another example, provided herein is an IL-22 Fc fusion protein for use in reducing the risk of developing chronic GVHD in a subject, wherein the IL-22 Fc fusion protein is for administration to a subject in need thereof in a dosing regimen comprising a dosing cycle, wherein the dosing cycle comprises three total doses of the IL-22 Fc fusion protein, wherein each dose is between about 30 μg/kg and 120 μg/kg, about 30 μg/kg and 90 μg/kg, about 30 μg/kg and 60 μg/kg, about 60 μg/kg and 90 μg/kg, or about 60 μg/kg and 120 μg/kg, wherein the doses are administered to the subject q1w, q2w, q3w, or q4w, preferably q2w.

In another example, provided herein is an IL-22 Fc fusion protein for use in reducing the risk of developing chronic GVHD in a subject, wherein the IL-22 Fc fusion protein is for administration to a subject in need thereof in a dosing regimen comprising a dosing cycle, wherein the dosing cycle comprises four total doses of the IL-22 Fc fusion protein, wherein each dose is between about 30 μg/kg and 120 μg/kg, about 30 μg/kg and 90 μg/kg, about 30 μg/kg and 60 μg/kg, about 60 μg/kg and 90 μg/kg, or about 60 μg/kg and 120 μg/kg, wherein the doses are administered to the subject q1w, q2w, q3w, or q4w, preferably q2w.

In another example, provided herein is an IL-22 Fc fusion protein for use in reducing the risk of developing chronic GVHD in a subject, wherein the IL-22 Fc fusion protein is for administration to a subject in need thereof in a dosing regimen comprising a dosing cycle, wherein the dosing cycle comprises five total doses of the IL-22 Fc fusion protein, wherein each dose is between about 30 μg/kg and 120 μg/kg, about 30 μg/kg and 90 μg/kg, about 30 μg/kg and 60 μg/kg, about 60 μg/kg and 90 μg/kg, or about 60 μg/kg and 120 μg/kg, wherein the doses are administered to the subject q1w, q2w, q3w, or q4w, preferably q2w.

In another example, provided herein is an IL-22 Fc fusion protein for use in reducing the risk of developing chronic GVHD in a subject, wherein the IL-22 Fc fusion protein is for administration to a subject in need thereof in a dosing regimen comprising a dosing cycle, wherein the dosing cycle comprises six total doses of the IL-22 Fc fusion protein, wherein each dose is between about 30 μg/kg and 120 μg/kg, about 30 μg/kg and 90 μg/kg, about 30 μg/kg and 60 μg/kg, about 60 μg/kg and 90 μg/kg, or about 60 μg/kg and 120 μg/kg, wherein the doses are administered to the subject q1w, q2w, q3w, or q4w, preferably q2w.

In another example, provided herein is an IL-22 Fc fusion protein for use in reducing the risk of developing chronic GVHD in a subject, wherein the IL-22 Fc fusion protein is for administration to a subject in need thereof in a dosing regimen comprising a dosing cycle, wherein the dosing cycle consists of a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between about 30 μg/kg and 120 μg/kg, about 30 μg/kg and 90 μg/kg, about 30 μg/kg and 60 μg/kg, about 60 μg/kg and 90 μg/kg, or about 60 μg/kg and 120 μg/kg , and wherein the doses are administered to the subject q1w, q2w, q3w, or q4w, preferably q2w.

In another example, provided herein is an IL-22 Fc fusion protein for use in reducing the risk of developing chronic GVHD in a subject, wherein the IL-22 Fc fusion protein is for administration to a subject in need thereof in a dosing regimen comprising a dosing cycle, wherein the dosing cycle comprises up to and no more than six total doses of the IL-22 Fc fusion protein, wherein the dosing cycle comprises a first dose (C1 D1) and one or more further doses of the IL-22 Fc fusion protein, wherein each dose is about 60 μg/kg, and wherein the doses are administered to the subject q1w, q2w, q3w, or q4w, preferably q2w.

In another example, provided herein is an IL-22 Fc fusion protein for use in reducing the risk of developing chronic GVHD in a subject, wherein the IL-22 Fc fusion protein is for administration to a subject in need thereof in a dosing regimen comprising a dosing cycle, wherein the dosing cycle consists of a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the doses are administered to the subject q1w, q2w, q3w, or q4w, preferably q2w.

In another example, provided herein is an IL-22 Fc fusion protein for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises up to and no more than six total doses of the IL-22 Fc fusion protein, wherein the dosing cycle comprises a first dose (C1 D1) and one or more further doses of the IL-22 Fc fusion protein, wherein each dose is between about 30 μg/kg and 120 μg/kg, about 30 μg/kg and 90 μg/kg, about 30 μg/kg and 60 μg/kg, about 60 μg/kg and 90 μg/kg, or about 60 μg/kg and 120 μg/kg , wherein the doses are administered to the subject q1w, q2w, q3w, or q4w, preferably q2w.

In another example, provided herein is an IL-22 Fc fusion protein for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises up to and no more than eight total doses of the IL-22 Fc fusion protein, wherein the dosing cycle comprises a first dose (C1 D1) and one or more further doses of the IL-22 Fc fusion protein, wherein each dose is between about 30 μg/kg and 120 μg/kg, about 30 μg/kg and 90 μg/kg, about 30 μg/kg and 60 μg/kg, about 60 μg/kg and 90 μg/kg, or about 60 μg/kg and 120 μg/kg, wherein the doses are administered to the subject q1w, q2w, q3w, or q4w, preferably q2w concurrently with or after allo-HSCT.

In another example, provided herein is an IL-22 Fc fusion protein for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle consists of a first dose (C1 D1), a second dose (C1D2), a third dose (C1 D3), a fourth dose (C1D4), a fifth dose (C1D5), and a sixth dose (C1D6) of the IL-22 Fc fusion protein, wherein the C1D1, the C1D2, the C1D3, the C1 D4, the C1D5, and the C1D6 are each between about 30 μg/kg and 120 μg/kg, about 30 μg/kg and 90 μg/kg, about 30 μg/kg and 60 μg/kg, about 60 μg/kg and 90 μg/kg, or about 60 μg/kg and 120 μg/kg , wherein the doses are administered to the subject q1w, q2w, q3w, or q4w, preferably q2w

In another example, provided herein is an IL-22 Fc fusion protein for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises two total doses of the IL-22 Fc fusion protein, wherein each dose is between about 30 μg/kg and 120 μg/kg, about 30 μg/kg and 90 μg/kg, about 30 μg/kg and 60 μg/kg, about 60 μg/kg and 90 μg/kg, or about 60 μg/kg and 120 μg/kg, wherein the doses are administered to the subject q1w, q2w, q3w, or q4w, preferably q2w.

In another example, provided herein is an IL-22 Fc fusion protein for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises three total doses of the IL-22 Fc fusion protein, wherein each dose is between about 30 μg/kg and 120 μg/kg, about 30 μg/kg and 90 μg/kg, about 30 μg/kg and 60 μg/kg, about 60 μg/kg and 90 μg/kg, or about 60 μg/kg and 120 μg/kg, wherein the doses are administered to the subject q1w, q2w, q3w, or q4w, preferably q2w.

In another example, provided herein is an IL-22 Fc fusion protein for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises four total doses of the IL-22 Fc fusion protein, wherein each dose is between about 30 μg/kg and 120 μg/kg, about 30 μg/kg and 90 μg/kg, about 30 μg/kg and 60 μg/kg, about 60 μg/kg and 90 μg/kg, or about 60 μg/kg and 120 μg/kg, wherein the doses are administered to the subject q1w, q2w, q3w, or q4w, preferably q2w.

In another example, provided herein is an IL-22 Fc fusion protein for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises five total doses of the IL-22 Fc fusion protein, wherein each dose is between about 30 μg/kg and 120 μg/kg, about 30 μg/kg and 90 μg/kg, about 30 μg/kg

and 60 µg/kg, about 60 µg/kg and 90 µg/kg, or about 60 µg/kg and 120 µg/kg, wherein the doses are administered to the subject q1w, q2w, q3w, or q4w, preferably q2w.

In another example, provided herein is an IL-22 Fc fusion protein for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises six total doses of the IL-22 Fc fusion protein, wherein each dose is between about 30 µg/kg and 120 µg/kg, about 30 µg/kg and 90 µg/kg, about 30 µg/kg and 60 µg/kg, about 60 µg/kg and 90 µg/kg, or about 60 µg/kg and 120 µg/kg, wherein the doses are administered to the subject q1w, q2w, q3w, or q4w, preferably q2w.

In another example, provided herein is an IL-22 Fc fusion protein for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises up to and no more than six total doses of the IL-22 Fc fusion protein, wherein the dosing cycle comprises a first dose (C1 D1) and one or more further doses of the IL-22 Fc fusion protein, wherein each dose is about 60 µg/kg, and wherein the doses are administered to the subject q1w, q2w, q3w, or q4w, preferably q2w.

In another example, provided herein is an IL-22 Fc fusion protein for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle consists of a first dose (C1 D1), a second dose (C1D2), a third dose (C1 D3), a fourth dose (C1D4), a fifth dose (C1D5), and a sixth dose (C1D6) of the IL-22 Fc fusion protein, wherein the C1D1, the C1D2, the C1D3, the C1 D4, the C1D5, and the C1D6 are each about 60 µg/kg, wherein the doses are administered to the subject q1w, q2w, q3w, or q4w, preferably q2w.

In some aspects, the one or more further doses comprise at least a second dose (C1 D2).

In some aspects, the one or more further doses comprise at least a C1D2 and a third dose (C1D3).

In some aspects, the one or more further doses comprise at least a C1D2, a C1D3, and a fourth dose (C1D4).

In some aspects, the one or more further doses comprise at least a C1D2, a C1D3, a C1 D4, and a fifth dose (C1D5).

In some aspects, the one or more further doses comprise at least a C1D2, a C1D3, a C1 D4, a C1D5, and a sixth dose (C1D6).

In some aspects, the dosing cycle comprises the C1D1, a C1D2, a C1D3, a C1D4, a C1D5, and a C1D6.

In some aspects, the dosing cycle comprises the C1D1, a C1D2, a C1D3, a C1D4, a C1D5, a C1D6, and a C1D7.

In some aspects, the dosing cycle comprises the C1D1, a C1D2, a C1D3, a C1D4, a C1D5, a C1D6, a C1D7, and a C1D8.

In some aspects, the C1D1 may be administered to the subject prior to allogeneic hematopoietic stem cell transplantation (allo-HSCT). The first dose of the dosing cycle may be administered at any suitable time prior to the allo-HSCT. For example, the first dose of the dosing cycle may be administered about 0.5 days, about 1 day, about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 21 days, about 22 days, about 23 days, about 24 days, about 25 days, about 26 days, about 27 days, about 28 days, about 29 days, about 30 days, about 31 days, about 2 months, about 3 months, about 4 months, about 5 months, about 6 months, about 7 months, about 8 months, about 9 months, about 10 months, about 11 months, or about a year prior to allo-HSCT. In some aspects, the first dose of the dosing cycle is administered to the subject about 3 (t2) days prior to allo-HSCT. In other aspects, the first dose of the dosing cycle is administered to the subject about 1 (t2) days prior to allo-HSCT. In some aspects, the C1D1 may be administered up to 1 week prior to allo-HSCT, up to 2 weeks prior to allo-HSCT, up to 3 weeks prior to allo-HSCT, up to 4 weeks prior to allo-HSCT, up to 5 weeks prior to allo-HSCT, up to 6 weeks prior to allo-HSCT, up to 7 weeks prior to allo-HSCT, or up to 8 weeks prior to allo-HSCT.

In another example, provided herein is an IL-22 Fc fusion protein for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises two total doses of the IL-22 Fc fusion protein, wherein each dose is about 60 µg/kg, wherein the doses are administered to the subject q1w, q2w, q3w, or q4w, preferably q2w.

In another example, provided herein is an IL-22 Fc fusion protein for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises three total doses of the IL-22 Fc fusion protein, wherein each dose is about 60 µg/kg, wherein the doses are administered to the subject q1w, q2w, q3w, or q4w, preferably q2w.

In another example, provided herein is an IL-22 Fc fusion protein for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises four total doses of the IL-22 Fc fusion protein, wherein each dose is about 60 μg/kg, wherein the doses are administered to the subject q1w, q2w, q3w, or q4w, preferably q2w.

In another example, provided herein is an IL-22 Fc fusion protein for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises five total doses of the IL-22 Fc fusion protein, wherein each dose is about 60 μg/kg, wherein the doses are administered to the subject q1w, q2w, q3w, or q4w, preferably q2w.

In another example, provided herein is an IL-22 Fc fusion protein for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises six total doses of the IL-22 Fc fusion protein, wherein each dose is about 60 μg/kg, wherein the doses are administered to the subject q1w, q2w, q3w, or q4w, preferably q2w.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), and a third dose (C1D3) of the IL-22 Fc fusion protein, wherein the C1D1, the C1D2, and the C1D3 are each between about 30 μg/kg and 120 μg/kg, wherein the C1D1 is administered to the subject 1 day prior to allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), and a third dose (C1D3) of the IL-22 Fc fusion protein, wherein the C1D1, the C1D2, and the C1D3 are each between about 30 μg/kg and 120 μg/kg, wherein the C1D1 is administered to the subject 1 day prior to allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), and a third dose (C1D3) of the IL-22 Fc fusion protein, wherein the C1D1, the C1D2, and the C1D3 are each between about 30 μg/kg and 120 μg/kg, wherein the C1D1 is administered to the subject 1 day prior to allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), and a third dose (C1D3) of the IL-22 Fc fusion protein, wherein the C1D1, the C1D2, and the C1D3 are each between about 30 μg/kg and 120 μg/kg, wherein the C1D1 is administered to the subject 1 day prior to allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), and a third dose (C1D3) of the IL-22 Fc fusion protein, wherein the C1D1, the C1D2, and the C1D3 are each between about 30 μg/kg and 120 μg/kg, wherein the C1D1 is administered to the subject 2 days prior to allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), and a third dose (C1D3) of the IL-22 Fc fusion protein, wherein the C1D1, the C1D2, and the C1D3 are each between about 30 μg/kg and 120 μg/kg, wherein the C1D1 is administered to the subject 2 days prior to allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), and a third dose (C1D3) of the IL-22 Fc fusion protein, wherein the C1D1, the C1D2, and the C1D3 are each between about 30 $\mu$g/kg and 120 $\mu$g/kg, wherein the C1D1 is administered to the subject 2 days prior to allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), and a third dose (C1D3) of the IL-22 Fc fusion protein, wherein the C1D1, the C1D2, and the C1D3 are each between about 30 $\mu$g/kg and 120 $\mu$g/kg, wherein the C1D1 is administered to the subject 2 days prior to allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), and a third dose (C1D3) of the IL-22 Fc fusion protein, wherein the C1D1, the C1D2, and the C1D3 are each between about 30 $\mu$g/kg and 120 $\mu$g/kg, wherein the C1D1 is administered to the subject 3 days prior to allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), and a third dose (C1D3) of the IL-22 Fc fusion protein, wherein the C1D1, the C1D2, and the C1D3 are each between about 30 $\mu$g/kg and 120 $\mu$g/kg, wherein the C1D1 is administered to the subject 3 days prior to allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8 or 10) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), and a third dose (C1D3) of the IL-22 Fc fusion protein, wherein the C1D1, the C1D2, and the C1D3 are each between about 30 $\mu$g/kg and 120 $\mu$g/kg, wherein the C1D1 is administered to the subject 3 days prior to allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), and a third dose (C1D3) of the IL-22 Fc fusion protein, wherein the C1D1, the C1D2, and the C1D3 are each between about 30 $\mu$g/kg and 120 $\mu$g/kg, wherein the C1D1 is administered to the subject 3 days prior to allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), and a third dose (C1D3) of the IL-22 Fc fusion protein, wherein the C1D1, the C1D2, and the C1D3 are each about 30 $\mu$g/kg, wherein the C1D1 is administered to the subject 1 day prior to allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), and a third dose (C1D3) of the IL-22 Fc fusion protein, wherein the C1D1, the C1D2, and the C1D3 are each about 60 $\mu$g/kg, wherein the C1D1 is administered to the subject 1 day prior to allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), and a third dose (C1D3) of the IL-22 Fc fusion protein, wherein the C1D1, the C1D2, and the C1D3 are each about 60 $\mu$g/kg, wherein the C1D1 is administered to the subject 1 day prior to allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), and a third dose (C1D3) of the IL-22 Fc fusion protein, wherein the C1D1, the C1D2, and the C1D3 are each about 60 μg/kg, wherein the C1D1 is administered to the subject 1 day prior to allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), and a third dose (C1D3) of the IL-22 Fc fusion protein, wherein the C1D1, the C1D2, and the C1D3 are each between about 60 μg/kg, wherein the C1D1 is administered to the subject 1 day prior to allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), and a third dose (C1D3) of the IL-22 Fc fusion protein, wherein the C1D1, the C1D2, and the C1D3 are each about 60 μg/kg, wherein the C1D1 is administered to the subject 2 days prior to allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), and a third dose (C1D3) of the IL-22 Fc fusion protein, wherein the C1D1, the C1D2, and the C1D3 are each about 60 μg/kg, wherein the C1D1 is administered to the subject 2 days prior to allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), and a third dose (C1D3) of the IL-22 Fc fusion protein, wherein the C1D1, the C1D2, and the C1D3 are each about 60 μg/kg, wherein the C1D1 is administered to the subject 2 days prior to allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), and a third dose (C1D3) of the IL-22 Fc fusion protein, wherein the C1D1, the C1D2, and the C1D3 are each about 60 μg/kg, wherein the C1D1 is administered to the subject 2 days prior to allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), and a third dose (C1D3) of the IL-22 Fc fusion protein, wherein the C1D1, the C1D2, and the C1D3 are each about 60 μg/kg, wherein the C1D1 is administered to the subject 3 days prior to allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), and a third dose (C1D3) of the IL-22 Fc fusion protein, wherein the C1D1, the C1D2, and the C1D3 are each about 60 μg/kg, wherein the C1D1 is administered to the subject 3 days prior to allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), and a third dose (C1D3) of the IL-22 Fc fusion protein, wherein the C1D1, the C1D2, and the C1D3 are each about 60 μg/kg, wherein the C1D1 is administered to the subject 3 days prior to allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), and a third dose (C1D3) of the IL-22 Fc fusion protein, wherein the C1D1, the C1D2, and the C1D3 are each about 60 μg/kg, wherein the C1D1 is administered to the subject 3 days prior to allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between about 30 μg/kg and 120 μg/kg, wherein the C1D1 is administered to the subject 1 day prior to allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between about 30 μg/kg and 120 μg/kg, wherein the C1D1 is administered to the subject 1 day prior to allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between about 30 μg/kg and 120 μg/kg, wherein the C1D1 is administered to the subject 1 day prior to allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between about 30 μg/kg and 120 μg/kg, wherein the C1D1 is administered to the subject 1 day prior to allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between about 30 μg/kg and 120 μg/kg, wherein the C1D1 is administered to the subject 2 days prior to allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between about 30 µg/kg and 120 µg/kg, wherein the C1D1 is administered to the subject 2 days prior to allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between about 30 µg/kg and 120 µg/kg, wherein the C1D1 is administered to the subject 2 days prior to allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between about 30 µg/kg and 120 µg/kg, wherein the C1D1 is administered to the subject 2 days prior to allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between about 30 µg/kg and 120 µg/kg, wherein the C1D1 is administered to the subject 3 days prior to allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between about 30 µg/kg and 120 µg/kg, wherein the C1D1 is administered to the subject 3 days prior to allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between about 30 µg/kg and 120 µg/kg, wherein the C1D1 is administered to the subject 3 days prior to allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between about 30 µg/kg and 120 µg/kg, wherein the C1D1 is administered to the subject 3 days prior to allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each about 60 µg/kg, wherein the C1D1 is administered to the subject 1 day prior to allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each about 60 μg/kg, wherein the C1D1 is administered to the subject 1 day prior to allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each about 60 μg/kg, wherein the C1D1 is administered to the subject 1 day prior to allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between about 60 μg/kg, wherein the C1D1 is administered to the subject 1 day prior to allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each about 60 μg/kg, wherein the C1D1 is administered to the subject 2 days prior to allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each about 60 μg/kg, wherein the C1D1 is administered to the subject 2 days prior to allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each about 60 μg/kg, wherein the C1D1 is administered to the subject 2 days prior to allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each about 60 μg/kg, wherein the C1D1 is administered to the subject 2 days prior to allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each about 60 μg/kg, wherein the C1D1 is administered to the subject 3 days prior to allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each about 60 µg/kg, wherein the C1D1 is administered to the subject 3 days prior to allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each about 60 µg/kg, wherein the C1D1 is administered to the subject 3 days prior to allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each about 60 µg/kg, wherein the C1D1 is administered to the subject 3 days prior to allo-HSCT, and wherein the doses are administered to the subject every 4 weeks (q4w).

The C1D1 may be administered to the subject concurrently with or after allo-HSCT.

In some examples, the C1D1 may be administered to the subject concurrently with allo-HSCT.

In other examples, the C1D1 may be administered to the subject after allo-HSCT.

The C1D1 may be administered to the subject any suitable amount of time after allo-HSCT. For example, the C1D1 may be administered to the subject 0.5 days, 1 day, 2 days, 3 days, 4 days, 5 days, 6 days, 7 days, 8 days, 9 days 10 days, 11 days, 12 days, 13 days, 14 days, 15 days, 16 days, 17 days, 18 days, 19 days, 20 days, 21 days, 22 days, 23 days, 24 days, 25 days, 26 days, 27 days, 28 days, 29 days, 30 days, 31 days, or longer, after HSCT. The C1D1 may be administered to the subject 1 month, 2 months, 3 months, 4 months, 5 months, 6 months, seven months, eight months, nine months, ten months, eleven months, twelve months, thirteen months, fourteen months, fifteen months, sixteen months, seventeen months, eighteen months, nineteen months, twenty months, twenty-one months, twenty-two months, twenty-three months, twenty-four months, or longer, after allo-HSCT. In some embodiments, the C1D1 may be administered to the subject 1 to 14 days, 1 to 13 days, 1 to 12 days, 1 to 11 days, 1 to 10 days, 1 to 9 days, 1 to 8 days, 1 to 7 days, 1 to 6 days, 1 to 5 days, 1 to 4 days, 1 to 3 days, 1 to 2 days, 2 to 14 days, 2 to 13 days, 2 to 12 days, 2 to 11 days, 2 to 10 days, 2 to 9 days, 2 to 8 days, 2 to 7 days, 2 to 6 days, 2 to 5 days, 2 to 4 days, 2 to 3 days, 3 to 14 days, 3 to 13 days, 3 to 12 days, 3 to 11 days, 3 to 10 days, 3 to 9 days, 3 to 8 days, 3 to 7 days, 3 to 6 days, 3 to 5 days, 3 to 4 days, 4 to 14 days, 4 to 13 days, 4 to 12 days, 4 to 11 days, 4 to 10 days, 4 to 9 days, 4 to 8 days, 4 to 7 days, 4 to 6 days, 4 to 5 days, 5 to 14 days, 5 to 13 days, 5 to 12 days, 5 to 11 days, 5 to 10 days, 5 to 9 days, 5 to 8 days, 5 to 7 days, 5 to 6 days, 6 to 14 days, 6 to 13 days, 6 to 12 days, 6 to 11 days, 6 to 10 days, 6 to 9 days, 6 to 8 days, 6 to 7 days, 7 to 14 days, 7 to 13 days, 7 to 12 days, 7 to 11 days, 7 to 10 days, 7 to 9 days, 7 to 8 days, 8 to 14 days, 8 to 13 days, 8 to 12 days, 8 to 11 days, 8 to 10 days, 8 to 9 days, 9 to 14 days, 9 to 13 days, 9 to 12 days, 9 to 11 days, 9 to 10 days, 10 to 14 days, 10 to 13 days, 10 to 12 days, 10 to 11 days, 11 to 14 days, 11 to 13 days, 11 to 12 days, 12 to 14 days, 12 to 13 days, or 13 to 14 days after allo-HSCT.

For example, in particular aspects, the C1D1 may be administered to the subject 1 to 3 days (e.g., 1, 2, or 3 days) after allo-HSCT. In some particular aspects, the C1D1 is administered to the subject within 2 days of allo-HSCT. In some particular aspects, the C1D1 is administered to the subject 1 day after allo-HSCT. In other particular aspects, the C1D1 is administered to the subject 2 days after allo-HSCT. In yet other particular aspects, the C1D1 is administered to the subject 3 days after allo-HSCT.

In some aspects, the GVHD is acute GVHD. In some aspecs, the acute GVHD is corticosteroid-refractory acute GVHD. In other aspects, the GVHD is chronic GVHD.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 µg/kg and 120 µg/kg, wherein the C1D1 is administered to the subject 1 day after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the C1D1 is administered to the subject 1 day after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the C1D1 is administered to the subject 1 day after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the C1D1 is administered to the subject 1 day after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the C1D1 is administered to the subject 2 days after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the C1D1 is administered to the subject 2 days after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the C1D1 is administered to the subject 2 days after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the C1D1 is administered to the subject 2 days after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 $\mu$g/kg and 120 $\mu$g/kg, wherein the C1D1 is administered to the subject 3 days after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 $\mu$g/kg and 120 $\mu$g/kg, wherein the C1D1 is administered to the subject 3 days after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 $\mu$g/kg and 120 $\mu$g/kg, wherein the C1D1 is administered to the subject 3 days after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 $\mu$g/kg and 120 $\mu$g/kg, wherein the C1D1 is administered to the subject 3 days after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 $\mu$g/kg, wherein the C1D1 is administered to the subject 1 day after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 $\mu$g/kg, wherein the C1D1 is administered to the subject 1 day after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 $\mu$g/kg, wherein the C1D1 is administered to the subject 1 day after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 $\mu$g/kg, wherein the C1D1 is administered to the subject 1 day after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 $\mu$g/kg, wherein the C1D1 is administered to the subject 2 days after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 $\mu$g/kg, wherein the C1D1 is administered to the subject 2 days after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 $\mu$g/kg, wherein the C1D1 is administered to the subject 2 days after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 $\mu$g/kg, wherein the C1D1 is administered to the subject 2 days after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 $\mu$g/kg, wherein the C1D1 is administered to the subject 3 days after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 $\mu$g/kg, wherein the C1D1 is administered to the subject 3 days after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 $\mu$g/kg, wherein the C1D1 is administered to the subject 3 days after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 $\mu$g/kg, wherein the C1D1 is administered to the subject 3 days after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 µg/kg and 120 µg/kg, wherein the C1D1 is administered to the subject 1 day after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 µg/kg and 120 µg/kg, wherein the C1D1 is administered to the subject 1 day after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 µg/kg and 120 µg/kg, wherein the C1D1 is administered to the subject 1 day after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 µg/kg and 120 µg/kg, wherein the C1D1 is administered to the subject 1 day after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 µg/kg and 120 µg/kg, wherein the C1D1 is administered to the subject 2 days after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 µg/kg and 120 µg/kg, wherein the C1D1 is administered to the subject 2 days after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 µg/kg and 120 µg/kg, wherein the C1D1 is administered to the subject 2 days after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 µg/kg and

120 μg/kg, wherein the C1D1 is administered to the subject 2 days after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the C1D1 is administered to the subject 3 days after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the C1D1 is administered to the subject 3 days after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the C1D1 is administered to the subject 3 days after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the C1D1 is administered to the subject 3 days after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the C1D1 is administered to the subject 1 day after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the C1D1 is administered to the subject 1 day after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the C1D1 is administered to the subject 1 day after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 $\mu$g/kg, wherein the C1D1 is administered to the subject 1 day after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 $\mu$g/kg, wherein the C1D1 is administered to the subject 2 days after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 $\mu$g/kg, wherein the C1D1 is administered to the subject 2 days after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 $\mu$g/kg, wherein the C1D1 is administered to the subject 2 days after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 $\mu$g/kg, wherein the C1D1 is administered to the subject 2 days after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 $\mu$g/kg, wherein the C1D1 is administered to the subject 3 days after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 $\mu$g/kg, wherein the C1D1 is administered to the subject 3 days after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1 D3, the C1D4, the C1D5, and the C1D6 are each 60 $\mu$g/kg, wherein the C1D1 is administered to the subject 3 days after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 $\mu$g/kg, wherein the C1D1 is administered to the subject 3 days after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 $\mu$g/kg and 120 $\mu$g/kg, wherein the C1D1 is administered to the subject 1 day after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 $\mu$g/kg and 120 $\mu$g/kg, wherein the C1D1 is administered to the subject 1 day after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 $\mu$g/kg and 120 $\mu$g/kg, wherein the C1D1 is administered to the subject 1 day after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 $\mu$g/kg and 120 $\mu$g/kg, wherein the C1D1 is administered to the subject 1 day after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 $\mu$g/kg and 120 $\mu$g/kg, wherein the C1D1 is administered to the subject 2 days after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the C1D1 is administered to the subject 2 days after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the C1D1 is administered to the subject 2 days after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the C1D1 is administered to the subject 2 days after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the C1D1 is administered to the subject 3 days after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the C1D1 is administered to the subject 3 days after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the C1D1 is administered to the subject 3 days after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the C1D1 is administered to the subject 3 days after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the

C1D1 is administered to the subject 1 day after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the C1D1 is administered to the subject 1 day after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the C1D1 is administered to the subject 1 day after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the C1D1 is administered to the subject 1 day after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the C1D1 is administered to the subject 2 days after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the C1D1 is administered to the subject 2 days after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the C1D1 is administered to the subject 2 days after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the C1D1 is administered to the subject 2 days after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the C1D1 is administered to the subject 3 days after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the C1D1 is administered to the subject 3 days after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the C1D1 is administered to the subject 3 days after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the C1D1 is administered to the subject 3 days after allo-HSCT, and wherein the doses are administered to the subject every 4 weeks (q4w).

Any suitable subject may receive the dosing regimens described herein. In some aspects, the allo-HSCT is HLA-matched related HSCT, HLA-matched unrelated HSCT, or single-antigen HLA-mismatched unrelated HSCT. In some aspects, the allo-HSCT is from peripheral blood or bone marrow stem cells. In some aspects, the subject has been diagnosed with acute myeloid leukemia (AML) in first complete remission, optionally with no circulating blasts and less than about 5% blasts in the bone marrow. In some aspects, the subject has been diagnosed with high-risk myelodysplastic syndrome (MDS), optionally with no circulating blasts and less than about 10% blasts in the bone marrow.

Any suitable conditioning regimen may be used prior to allo-HSCT. In some aspects, the subject has received a myeloablative conditioning regimen. In other aspects, the subject has received a non-myeloablative conditioning regimen.

The IL-22 Fc fusion protein may include an IL-22 polypeptide linked to an Fc region by a linker. In some aspectts, the IL-22 polypeptide is glycosylated and/or the Fc region is not glycosylated. In some aspects: (i) the amino acid residue at position 297 as in the EU index of the Fc region is Gly or Ala; and/or (ii) the amino acid residue at position 299 as in the EU index of the Fc region is Ala, Gly, or Val. In some aspects, the Fc region is an IgG1 region or an IgG4 region. In some aspects, the Fc region is an IgG4 Fc region. I In some embodiments, the IL-22 Fc fusion protein comprises or consists of the amino acid sequence of SEQ ID NO:8.

In some aspects, the IL-22 Fc fusion protein is a dimeric IL-22 Fc fusion protein.

In other aspects, the IL-22 Fc fusion protein is a monomeric IL-22 Fc fusion protein.

In some aspects, the IL-22 polypeptide is a human IL-22 polypeptide.

In some aspects, the IL-22 Fc fusion protein binds to IL-22 receptor. In some aspects, the IL-22 receptor is human IL-22 receptor.

In some aspects, the IL-22 Fc fusion protein may be included in a pharmaceutical composition, e.g., a pharmaceutical composition comprising an IL-22 Fc fusion protein (e.g., as described herein, e.g., an IL-22 Fc fusion protein comprising the amino acid sequence set forth in SEQ ID NO: 8) and a pharmaceutically acceptable carrier.

In some aspects, the pharmaceutical composition may have an average sialic acid content of 8 moles of sialic acid per mole of the IL-22 Fc fusion protein. In other aspects of any of the preceding methods, the pharmaceutical composition may have an average sialic acid content of 9 moles of sialic acid per mole of the IL-22 Fc fusion protein. In some aspects, the sialic acid comprises N-acetylneuraminic acid (NANA). In some aspects, the pharmaceutical composition has an average NGNA content of less than 1 mole of NGNA per mole of the IL-22 Fc fusion protein.

In any of the preceding uses, the pharmaceutical composition may be a liquid composition.

In some aspects, (i) the IL-22 Fc fusion protein may have a maximum observed concentration ($C_{max}$) of about 8,000 ng/mL to about 19,000 ng; (ii) the IL-22 Fc fusion protein may have an area under the serum concentration-time curve from time 0 to the last measureable time point ($AUC_{last}$) of about 7,000 day·ng/mL to about 25,000 day·ng/mL; and/or (iii) the IL-22 Fc fusion protein may have a clearance (CL) of about 40 mL/kg/day to about 140 mL/kg/day. In some aspects, the $C_{max}$, $AUC_{last}$, and/or CL is assessed following intravenous administration of about 1,000 $\mu$g/kg of the IL-22 Fc fusion protein to a CD1 mouse.

In some aspects, the IL-22 polypeptide may comprise N-glycans having monoantennary, biantennary, triantennary, and/or tetraantennary structure. In some embodiments: (i) about 0.1% to about 2% of the N-glycans have monoantennary structure; (ii) about 10% to about 25% of the N-glycans have biantennary structure; (iii) about 25% to about 40% of the N-glycans have triantennary structure; and/or (iv) about 30% to about 51% of the N-glycans have tetraantennary structure. In some embodiments: (i) 0.1% to 2% of the N-glycans have monoantennary structure; (ii) 10% to 25% of the N-glycans have biantennary structure; (iii) 25% to 40% of the N-glycans have triantennary structure; and/or (iv) 30% to 51% of the N-glycans have tetraantennary structure.

In some aspects, the IL-22 Fc fusion protein may comprise N-glycans comprising zero, one, two, three, or four galactose moieties. In some aspects: (i) about 9% to about 32% of the N-glycans comprise zero galactose moieties; (ii) about 10% to about 20% of the N-glycans comprise one galactose moiety; (iii) about 8% to about 25% of the N-glycans comprise two galactose moieties; (iv) about 12% to about 25% of the N-glycans comprise three galactose moieties; and/or (v) about 12% to about 30% of the N-glycans comprise four galactose moieties. In some aspects: (i) 9% to 32% of the N-glycans comprise zero galactose moieties; (ii) 10% to 20% of the N-glycans comprise one galactose moiety; (iii) 8% to 25% of the N-glycans comprise two galactose moieties; (iv) 12% to 25% of the N-glycans comprise three galactose moieties; and/or (v) 12% to 30% of the N-glycans comprise four galactose moieties.

In some aspects, the IL-22 Fc fusion protein may comprise N-glycans comprising zero, one, two, three, or four sialic acid moieties. In some aspects: (i) about 12% to about 35% of the N-glycans comprise zero sialic acid moieties; (ii) about 10% to about 30% of the N-glycans comprise one sialic acid moiety; (iii) about 10% to about 30% of the N-glycans comprise two sialic acid moieties; (iv) about 10% to about 30% of the N-glycans comprise three sialic acid moieties; and/or (v) about 1% to about 20% of the N-glycans comprise four sialic acid moieties. In some embodiments: (i) 12% to 35% of the N-glycans comprise zero sialic acid moieties; (ii) 10% to 30% of the N-glycans comprise one sialic acid moiety; (iii) 10% to 30% of the N-glycans comprise two sialic acid moieties; (iv) 10% to 30% of the N-glycans comprise three sialic acid moieties; and/or (v) 1% to 20% of the N-glycans comprise four sialic acid moieties.

In some aspects, (i) the IL-22 polypeptide may comprise about 0% to about 10% N-glycans comprising a terminal mannose moiety; and/or (ii) the IL-22 polypeptide may comprise about 30% to about 55% N-glycans comprising a terminal N-acetylglucosamine (GlcNAc) moiety. In some aspects, (i) the IL-22 polypeptide comprises 0% to 10% N-glycans comprising a terminal mannose moiety; and/or (ii) the IL-22 polypeptide comprises 30% to 55% N-glycans comprising a terminal GlcNAc moiety. In some aspects, the IL-22 polypeptide comprises 0% to 10% N-glycans comprising a terminal mannose moiety. In some aspects, the IL-22 polypeptide comprises 30% to 55% N-glycans comprising a terminal GlcNAc moiety.

In some aspects, the N-glycans may comprise one, two, three, or four terminal GlcNAc moieties. In some aspects: (i) about 1% to about 20% of the N-glycans comprise one terminal GlcNAc moiety; (ii) about 1% to about 20% of the N-glycans comprise two terminal GlcNAc moieties; (iii) about 5% to about 25% of the N-glycans comprise three terminal GlcNAc moieties; and/or (iv) about 0% to about 15% of the N-glycans comprise four terminal GlcNAc moieties. In some aspects: (i) 1% to 20% of the N-glycans comprise one terminal GlcNAc moiety; (ii) 1% to 20% of the N-glycans comprise two terminal GlcNAc moieties; (iii) 5% to 25% of the N-glycans comprise three terminal GlcNAc moieties; and/or (iv) 0% to 15% of the N-glycans comprise four terminal GlcNAc moieties.

In some aspects, (i) the IL-22 polypeptide may comprise about 20% to about 45% N-glycans comprising a terminal galactose (Gal) moiety; and/or (ii) the N-glycans comprise one, two, or three terminal Gal moieties. In some aspects, (i) the IL-22 polypeptide comprises 20% to 45% N-glycans comprising a terminal Gal moiety; and/or (ii) the N-glycans comprise one, two, or three terminal Gal moieties.

In some aspects, (i) about 15% to about 30% of the N-glycans may comprise one terminal Gal moiety; (ii) about 1% to about 15% of the N-glycans may comprise two terminal Gal moieties; and/or (iii) about 0.1% to about 6% of the N-glycans may comprise three terminal Gal moieties. In some aspects: (i) 15% to 30% of the N-glycans comprise one terminal Gal moiety; (ii) 1% to 15% of the N-glycans comprise two terminal Gal moieties; and/or (iii) 0.1% to 6% of the N-glycans comprise three terminal Gal moieties.

In some aspects, (i) the IL-22 polypeptide may comprise N-glycans comprises galactose N-acetylglucosamine (LacNAc) repeats; (ii) the IL-22 polypeptide may comprise N-glycans comprising fucosylated N-glycans; and/or (iii) the IL-22 polypeptide may comprise N-glycans comprising afucosylated N-glycans.

Any suitable concentration of the IL-22 Fc fusion protein may be used. For example, in some aspects, the concentration of the IL-22 Fc fusion protein is about 0.5 mg/mL to about 20 mg/mL. In some aspects, the concentration of the IL-22 Fc fusion protein is about 0.5 mg/mL to about 5 mg/mL. In some aspects, the concentration of the IL-22 Fc fusion protein is about 1 mg/mL. In some aspects, the concentration of the IL-22 Fc fusion protein is about 8 mg/mL to about 12 mg/mL. In some aspects, the concentration of the IL-22 Fc fusion protein is about 10 mg/mL.

The IL-22 Fc fusion proteins or compositions thereof can be administered alone (i.e., as a monotherapy) or in combination with an additional GVHD therapy, including, for example, immunosuppressive agents (e.g., cyclosporine, mycophenolate mofetil (MMF), or tacrolimus), mTOR inhibitors (e.g., sirolimus or everolimus)), chemotherapy agents (e.g., imatinib, pentostatin, methotrexate, or thalidomide), TNF antagonists (e.g., etanercept), steroids (e.g., prednisolone, methylprednisolone, topical steroids, or steroid eye drops), light treatment (e.g., extracorporeal photopheresis), hydroxychloroquine, anti-fibrotic agents (e.g., halofuginone), monoclonal antibodies (e.g., alemtuzumab, infliximab, or rituximab), or combinations thereof. In some aspects, the additional GVHD therapy is an immunosuppressive agent (e.g., cyclosporine or tacrolimus). In some aspects, the IL-22 Fc fusion protein is administered in combination with an additional therapeutic agent. In some embodiments, the additional GVHD therapy is standard of care for acute GVHD prophylaxis (e.g., calcineurin (CN) inhibitor (e.g., cyclosporine or tacrolimus) + methotrexate or mycophenolate mofetil (MMF)). Any suitable standard of care aGVHD prophylaxis may be used (see, e.g., Gatza et al. Int. J. Hematol. Oncol. 4(3):113-126, 2015). A person of skill in the art will be able to select a suitable standard of care as appropriate.

For example, in some aspects, the IL-22 Fc fusion protein is administered in combination with an additional GVHD therapy selected from an immunosuppressive agent, a chemotherapy agent, a TNF antagonist, a steroid, light treatment, hydroxychloroquine, an anti-fibrotic agent, a monoclonal antibody, or a combination thereof. In some aspects, the additional GVHD therapy is an immunosuppressive agent. In some embodiments, the immunosuppressive agent is a calcineurin inhibitor. In some aspects, the calcineurin inhibitor is cyclosporine or tacrolimus. In some aspects, the IL-22 Fc fusion protein is administered in combination with standard of care. In some aspects, the standard of care for acute GVHD prophylaxis is cyclosporine or tacrolimus in combination with methotrexate.

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the IL-22 Fc fusion protein is administered in combination with acute GVHD standard of care, wherein the C1D1 is administered to the subject 1 day after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the IL-22 Fc fusion protein is administered in combination with acute GVHD standard of care, wherein the C1D1 is administered to the subject 1 day after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the IL-22 Fc fusion protein is administered in combination with acute GVHD standard of care, wherein the C1D1 is administered to the subject 1 day after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the IL-22 Fc fusion protein is administered in combination with acute GVHD standard of care, wherein the C1D1 is administered to the subject 1 day after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 $\mu$g/kg and 120 $\mu$g/kg, wherein the IL-22 Fc fusion protein is administered in combination with acute GVHD standard of care, wherein the C1D1 is administered to the subject 2 days after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 $\mu$g/kg and 120 $\mu$g/kg, wherein the IL-22 Fc fusion protein is administered in combination with acute GVHD standard of care, wherein the C1D1 is administered to the subject 2 days after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 $\mu$g/kg and 120 $\mu$g/kg, wherein the IL-22 Fc fusion protein is administered in combination with acute GVHD standard of care, wherein the C1D1 is administered to the subject 2 days after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 $\mu$g/kg and 120 $\mu$g/kg, wherein the IL-22 Fc fusion protein is administered in combination with acute GVHD standard of care, wherein the C1D1 is administered to the subject 2 days after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 $\mu$g/kg and 120 $\mu$g/kg, wherein the IL-22 Fc fusion protein is administered in combination with acute GVHD standard of care, wherein the C1D1 is administered to the subject 3 days after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 $\mu$g/kg and 120 $\mu$g/kg, wherein the IL-22 Fc fusion protein is administered in combination with acute GVHD standard of care, wherein the C1D1 is administered to the subject 3 days after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 $\mu$g/kg and 120 $\mu$g/kg, wherein the IL-22 Fc fusion protein is administered in combination with acute GVHD standard of care, wherein the C1D1 is administered to the subject 3 days after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 µg/kg and 120 µg/kg, wherein the IL-22 Fc fusion protein is administered in combination with acute GVHD standard of care, wherein the C1D1 is administered to the subject 3 days after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 µg/kg, wherein the IL-22 Fc fusion protein is for administration in combination with acute GVHD standard of care, wherein the C1D1 is administered to the subject 1 day after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 µg/kg, wherein the IL-22 Fc fusion protein is for administration in combination with acute GVHD standard of care, wherein the C1D1 is administered to the subject 1 day after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 µg/kg, wherein the IL-22 Fc fusion protein is for administration in combination with acute GVHD standard of care, wherein the C1D1 is administered to the subject 1 day after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 µg/kg, wherein the IL-22 Fc fusion protein is for administration in combination with acute GVHD standard of care, wherein the C1D1 is administered to the subject 1 day after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 µg/kg, wherein the IL-22 Fc fusion protein is for administration in combination with acute GVHD standard of care, wherein the C1D1 is administered to the subject 2 days after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 µg/kg, wherein the IL-22 Fc fusion protein is for administration in combination with acute GVHD standard of care, wherein the C1D1 is administered to the subject 2 days after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 µg/kg, wherein the IL-22 Fc fusion protein is for administration in combination with acute GVHD standard of care, wherein the C1D1 is administered to the subject 2 days after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the IL-22 Fc fusion protein is for administration in combination with acute GVHD standard of care, wherein the C1D1 is administered to the subject 2 days after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the IL-22 Fc fusion protein is for administration in combination with acute GVHD standard of care, wherein the C1D1 is administered to the subject 3 days after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the IL-22 Fc fusion protein is for administration in combination with acute GVHD standard of care, wherein the C1D1 is administered to the subject 3 days after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the IL-22 Fc fusion protein is for administration in combination with acute GVHD standard of care, wherein the C1D1 is administered to the subject 3 days after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in preventing acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the IL-22 Fc fusion protein is for administration in combination with acute GVHD standard of care, wherein the C1D1 is administered to the subject 3 days after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the IL-22 Fc fusion protein is administered in combination with acute GVHD standard of care, wherein the C1D1 is administered to the subject 1 day after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the IL-22 Fc fusion protein is administered in combination with acute GVHD standard of care, wherein the C1D1 is administered to the subject 1 day after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 $\mu$g/kg and 120 $\mu$g/kg, wherein the IL-22 Fc fusion protein is administered in combination with acute GVHD standard of care, wherein the C1D1 is administered to the subject 1 day after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 $\mu$g/kg and 120 $\mu$g/kg, wherein the IL-22 Fc fusion protein is administered in combination with acute GVHD standard of care, wherein the C1D1 is administered to the subject 1 day after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 $\mu$g/kg and 120 $\mu$g/kg, wherein the IL-22 Fc fusion protein is administered in combination with acute GVHD standard of care, wherein the C1D1 is administered to the subject 2 days after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 $\mu$g/kg and 120 $\mu$g/kg, wherein the IL-22 Fc fusion protein is administered in combination with acute GVHD standard of care, wherein the C1D1 is administered to the subject 2 days after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 $\mu$g/kg and 120 $\mu$g/kg, wherein the IL-22 Fc fusion protein is administered in combination with acute GVHD standard of care, wherein the C1D1 is administered to the subject 2 days after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 $\mu$g/kg and 120 $\mu$g/kg, wherein the IL-22 Fc fusion protein is administered in combination with acute GVHD standard of care, wherein the C1D1 is administered to the subject 2 days after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the IL-22 Fc fusion protein is administered in combination with acute GVHD standard of care, wherein the C1D1 is administered to the subject 3 days after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the IL-22 Fc fusion protein is administered in combination with acute GVHD standard of care, wherein the C1D1 is administered to the subject 3 days after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the IL-22 Fc fusion protein is administered in combination with acute GVHD standard of care, wherein the C1D1 is administered to the subject 3 days after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the IL-22 Fc fusion protein is administered in combination with acute GVHD standard of care, wherein the C1D1 is administered to the subject 3 days after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the IL-22 Fc fusion protein is for administration in combination with acute GVHD standard of care, wherein the C1D1 is administered to the subject 1 day after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the IL-22 Fc fusion protein is for administration in combination with acute GVHD standard of care, wherein the C1D1 is administered to the subject 1 day after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the

IL-22 Fc fusion protein is for administration in combination with acute GVHD standard of care, wherein the C1D1 is administered to the subject 1 day after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 $\mu$g/kg, wherein the IL-22 Fc fusion protein is for administration in combination with acute GVHD standard of care, wherein the C1D1 is administered to the subject 1 day after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 $\mu$g/kg, wherein the IL-22 Fc fusion protein is for administration in combination with acute GVHD standard of care, wherein the C1D1 is administered to the subject 2 days after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 $\mu$g/kg, wherein the IL-22 Fc fusion protein is for administration in combination with acute GVHD standard of care, wherein the C1D1 is administered to the subject 2 days after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 $\mu$g/kg, wherein the IL-22 Fc fusion protein is for administration in combination with acute GVHD standard of care, wherein the C1D1 is administered to the subject 2 days after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 $\mu$g/kg, wherein the IL-22 Fc fusion protein is for administration in combination with acute GVHD standard of care, wherein the C1D1 is administered to the subject 2 days after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 $\mu$g/kg, wherein the IL-22 Fc fusion protein is for administration in combination with acute GVHD standard of care, wherein the C1D1 is administered to the subject 3 days after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the IL-22 Fc fusion protein is for administration in combination with acute GVHD standard of care, wherein the C1D1 is administered to the subject 3 days after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the IL-22 Fc fusion protein is for administration in combination with acute GVHD standard of care, wherein the C1D1 is administered to the subject 3 days after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of developing chronic GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 μg/kg, wherein the IL-22 Fc fusion protein is for administration in combination with acute GVHD standard of care, wherein the C1D1 is administered to the subject 3 days after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the IL-22 Fc fusion protein is administered in combination with acute GVHD standard of care, wherein the C1D1 is administered to the subject 1 day after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the IL-22 Fc fusion protein is administered in combination with acute GVHD standard of care, wherein the C1D1 is administered to the subject 1 day after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 μg/kg and 120 μg/kg, wherein the IL-22 Fc fusion protein is administered in combination with acute GVHD standard of care, wherein the C1D1 is administered to the subject 1 day after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 µg/kg and 120 µg/kg, wherein the IL-22 Fc fusion protein is administered in combination with acute GVHD standard of care, wherein the C1D1 is administered to the subject 1 day after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 µg/kg and 120 µg/kg, wherein the IL-22 Fc fusion protein is administered in combination with acute GVHD standard of care, wherein the C1D1 is administered to the subject 2 days after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 µg/kg and 120 µg/kg, wherein the IL-22 Fc fusion protein is administered in combination with acute GVHD standard of care, wherein the C1D1 is administered to the subject 2 days after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 µg/kg and 120 µg/kg, wherein the IL-22 Fc fusion protein is administered in combination with acute GVHD standard of care, wherein the C1D1 is administered to the subject 2 days after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 µg/kg and 120 µg/kg, wherein the IL-22 Fc fusion protein is administered in combination with acute GVHD standard of care, wherein the C1D1 is administered to the subject 2 days after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 µg/kg and 120 µg/kg, wherein the IL-22 Fc fusion protein is administered in combination with acute GVHD standard of care, wherein the C1D1 is administered to the subject 3 days after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 µg/kg and 120 µg/kg, wherein the IL-22 Fc fusion protein is administered in combination with acute GVHD standard of care, wherein the C1D1 is administered to the subject 3 days after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 µg/kg and 120 µg/kg, wherein the IL-22 Fc fusion protein is administered in combination with acute GVHD standard of care, wherein the C1D1 is administered to the subject 3 days after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each between 30 µg/kg and 120 µg/kg, wherein the IL-22 Fc fusion protein is administered in combination with acute GVHD standard of care, wherein the C1D1 is administered to the subject 3 days after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 µg/kg, wherein the IL-22 Fc fusion protein is for administration in combination with acute GVHD standard of care, wherein the C1D1 is administered to the subject 1 day after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 µg/kg, wherein the IL-22 Fc fusion protein is for administration in combination with acute GVHD standard of care, wherein the C1D1 is administered to the subject 1 day after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 µg/kg, wherein the IL-22 Fc fusion protein is for administration in combination with acute GVHD standard of care, wherein the C1D1 is administered to the subject 1 day after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 µg/kg, wherein the IL-22 Fc fusion protein is for administration in combination with acute GVHD standard of care, wherein the C1D1 is administered to the subject 1 day after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 µg/kg, wherein the IL-22 Fc fusion protein is for administration in combination with acute GVHD standard of care, wherein the C1D1 is administered to the subject 2 days after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 µg/kg, wherein the IL-22 Fc fusion protein is for administration in combination with acute GVHD standard of care, wherein the C1D1 is administered to the subject 2 days after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 µg/kg, wherein the IL-22 Fc fusion protein is for administration in combination with acute GVHD standard of care, wherein the C1D1 is administered to the subject 2 days after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 µg/kg, wherein the IL-22 Fc fusion protein is for administration in combination with acute GVHD standard of care, wherein the C1D1 is administered to the subject 2 days after allo-HSCT, and wherein the doses are administered to the subject every 4 weeks (q4w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 µg/kg, wherein the IL-22 Fc fusion protein is for administration in combination with acute GVHD standard of care, wherein the C1D1 is administered to the subject 3 days after allo-HSCT, and wherein the doses are administered to the subject every week (q1w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 µg/kg, wherein the IL-22 Fc fusion protein is for administration in combination with acute GVHD standard of care, wherein the C1D1 is administered to the subject 3 days after allo-HSCT, and wherein the doses are administered to the subject every 2 weeks (q2w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 µg/kg, wherein the IL-22 Fc fusion protein is for administration in combination with acute GVHD standard of care, wherein the C1D1 is administered to the subject 3 days after allo-HSCT, and wherein the doses are administered to the subject every 3 weeks (q3w).

In another example, provided herein is an IL-22 Fc fusion protein (e.g., as described herein, e.g., an IL-22 Fc fusion protein comprising the amino acid sequence set forth in SEQ ID NO: 8) for use in reducing the risk of corticosteroid-refractory acute GVHD in a subject, wherein the IL-22 Fc fusion protein is for administration to a subject in need thereof in a dosing regimen comprising a dosing cycle, wherein the dosing cycle comprises a first dose (C1D1), a second dose (C1D2), a third dose (C1D3), a fourth dose (C1D4), a fifth dose (C1D5), and a sixth dose (C1D6) of the IL-22 Fc fusion protein, wherein the C1D1, the C1D2, the C1D3, the C1D4, the C1D5, and the C1D6 are each 60 µg/kg, wherein the IL-22 Fc fusion protein is for administration in combination with acute GVHD standard of care, wherein the C1D1 is administered to the subject 3 days after allo-HSCT, and wherein the doses are administered to the subject every 4 weeks (q4w).

In any of the preceding uses, , the acute GVHD standard of care can be an immunosuppressive agent. In some aspects, the acute GVHD standard of care is a calcineurin (CN) inhibitor (e.g., cyclosporine or tacrolimus) in combination with methotrexate or mycophenolate mofetil (MMF)). In some aspects, the acute GVHD standard of care is cyclosporine or tacrolimus in combination with methotrexate. In some aspects, the acute GVHD standard of care is cyclosporine in combination with methotrexate. In other aspects, the acute GVHD standard of care is tacrolimus in combination with methotrexate.

The IL-22 Fc fusion protein (e.g., as described herein, e.g., an IL-22 Fc fusion protein comprising the amino acid sequence set forth in SEQ ID NO: 8), or a composition thereof (e.g., a pharmaceutical composition), and/or any additional therapeutic agent(s) may be administered by any suitable route. In some aspects, the administering is performed intravitreally, intramuscularly, intravenously, intradermally, percutaneously, intraarterially, intraperitoneally, intralesionally, intracranially, intraarticularly, intraprostatically, intrapleurally, intratracheally, intrathecally, intranasally, intravaginally, intrarectally, topically, intratumorally, peritoneally, subcutaneously, subconjunctivally, intravesicularly, mucosally, intrapericardially, intraumbilically, intraocularly, intraorbitally, orally, topically, transdermally, periocularly, conjunctivally, subtenonly, intracamerally, subretinally, retrobulbarly, intracanalicularly, by inhalation, by injection, by implantation, by infusion, by continuous infusion, by localized perfusion bathing target cells directly, by catheter, by lavage, in cremes, or in lipid compositions. In some aspects, the administering is preferably by intravenous infusion. In other aspects, the administering is by subcutaneous administration.

Any of the dosing regimens may further include one or more further dosing cycles. For example, in some aspects, the dosing regimen further includes one, two, three, four, five, six, seven, eight, nine, ten, eleven, twelve, thirteen, fourteen, fifteen, sixteen, seventeen, eighteen, nineteen, or twenty further dosing cycles. In particular aspects, the dosing regimen further comprises a second dosing cycle.

In some aspects, the dose(s) in the further (e.g., second) dosing cycle(s) are administered to the subject every week (q1w), every two weeks (q2w), every three weeks (q3w), every four weeks, (q4w), every five weeks (q5w), every six weeks (q6w), every seven weeks (q7w), every eight weeks (q8w), every nine weeks (q9w), every ten weeks (q10w), every 12 weeks (q12w), every fourteen weeks (q14w), every sixteen weeks (q16w), every eighteen weeks (q18w), or every twenty weeks (q20w).

In some aspects, the length of the further (e.g., second) dosing cycle is between about 5 weeks and about 80 weeks, e.g., about 5 weeks, about 6 weeks, about 7 weeks, about 9 weeks, about 10 weeks, about 11 weeks, about 12 weeks, about 14 weeks, about 16 weeks, about 18 weeks, about 20 weeks, about 22 weeks, about 24 weeks, about 26 weeks, about 28 weeks, about 30 weeks, about 32 weeks, about 34 weeks, about 36 weeks, about 38 weeks, about 40 weeks, about 42 weeks, about 44 weeks, about 46 weeks, about 48 weeks, about 50 weeks, about 52 weeks, about 54 weeks, about 56 weeks, about 58 weeks, about 60 weeks, about 62 weeks, about 64 weeks, about 66 weeks, about 68 weeks, about 70 weeks, about 72 weeks, about 74 weeks, about 76 weeks, about 78 weeks, or about 80 weeks.

For example, in some aspects of any of the preceding methods, the length of the further (e.g., second) dosing cycle is between about 5 weeks and about 80 weeks, between about 5 weeks and about 75 weeks, between about 5 weeks and about 70 weeks, between about 5 weeks and about 65 weeks, between about 5 weeks and about 60 weeks, between about 5 weeks and about 55 weeks, between about 5 weeks and about 50 weeks, between about 5 weeks and about 45 weeks, between about 5 weeks and about 40 weeks, between about 5 weeks and about 35 weeks, between about 5 weeks and about 30 weeks, between about 5 weeks and about 25 weeks, between about 5 weeks and about 20 weeks, between about 5 weeks and about 15 weeks, between about 5 weeks and about 10 weeks, between about 10 weeks and about 80 weeks, between about 10 weeks and about 75 weeks, between about 10 weeks and about 70 weeks, between about 10 weeks and about 65 weeks, between about 10 weeks and about 60 weeks, between about 10 weeks and about 55 weeks, between about 10 weeks and about 50 weeks, between about 10 weeks and about 45 weeks, between about 10 weeks and about 40 weeks, between about 10 weeks and about 35 weeks, between about 10 weeks and about 30 weeks, between about 10 weeks and about 25 weeks, between about 10 weeks and about 20 weeks, between about 10 weeks and about 15 weeks, between about 15 weeks and about 80 weeks, between about 15 weeks and about 75 weeks, between about 15 weeks and about 70 weeks, between about 15 weeks and about 65 weeks, between about 15 weeks and about 60 weeks, between about 15 weeks and about 55 weeks, between about 15 weeks and about 50 weeks, between about 15 weeks and about 45 weeks, between about 15 weeks and about 40 weeks, between about 15 weeks and about 35 weeks, between about 15 weeks and about 30 weeks, between about 15 weeks and about 25 weeks, between about 15 weeks and about 20 weeks, between about 20 weeks and about 80 weeks, between about 20 weeks and about 75 weeks, between about 20 weeks and about 70 weeks, between about 20 weeks and about 65 weeks, between about 20 weeks and about 60 weeks, between about 20 weeks and about 55 weeks, between about 20 weeks and about 50 weeks, between about 20 weeks and about 45 weeks, between about 20 weeks and about 40 weeks, between about 20 weeks and about 35 weeks, between about 20 weeks and about 30 weeks, between about 20 weeks and about 25 weeks, between about 25 weeks and about 80 weeks, between about 25 weeks and about 75 weeks, between about 25 weeks and about 70 weeks, between about 25 weeks and about 65 weeks, between about 25 weeks and about 60 weeks, between about 25 weeks and about 55 weeks, between about 25 weeks and about 50 weeks, between about 25 weeks and about 45 weeks, between about 25 weeks and about 40 weeks, between about 25 weeks and about 35 weeks, between about 25 weeks and about 30 weeks, between about 30 weeks and about 80 weeks, between about 30 weeks and about 75 weeks, between about 30 weeks and about 70 weeks, between about 30 weeks and about 65 weeks, between about 30 weeks and about 60 weeks, between about 30 weeks and about 55 weeks, between about 30 weeks and about 50 weeks, between about 30 weeks and about 45 weeks, between about 30 weeks and about 40 weeks, between about 30 weeks and about 35 weeks, between about 35 weeks and about 80 weeks, between about 35 weeks and about 75 weeks, between about 35 weeks and about 70 weeks, between about 35 weeks and about 65 weeks, between about 35 weeks and about 60 weeks, between about 35 weeks and about 55 weeks, between about 35 weeks and about 50 weeks, between about 35 weeks and about 45 weeks, between about 35 weeks and about 40 weeks, between about 40 weeks and about 80 weeks, between about 40 weeks and about 75 weeks, between about 40 weeks and about 70 weeks, between about 40 weeks and about 65 weeks, between about 40 weeks and about 60 weeks, between about 40 weeks and about 55 weeks, between about 40 weeks and about 50 weeks, between about 40 weeks and about 45 weeks, between about 45 weeks and about 80 weeks, between about 45 weeks and about 75 weeks, between about 45 weeks and about 70 weeks, between about 45 weeks and about 65 weeks, between about 45 weeks and about 60 weeks, between about 45 weeks and about 55 weeks, between about 45 weeks and about 50 weeks, between about 50 weeks and about 80 weeks, between about 50 weeks and about 75 weeks, between about 50 weeks and about 70 weeks, between about 50 weeks and about 65 weeks, between about 50 weeks and about 60 weeks, between about 50 weeks and about 55 weeks, between about 55 weeks and about 80 weeks, between about 55 weeks and about 75 weeks, between about 55 weeks and about 70 weeks, between about 55 weeks and about 65 weeks, between about 55 weeks and about 60 weeks, between about 60 weeks and about 80 weeks, between about 60 weeks and about 75 weeks, between about 60 weeks and about 70 weeks, between about 60 weeks and about 65 weeks, between about 65 weeks and about 80 weeks, between about 65 weeks and about 75 weeks, between about 65 weeks and about 70 weeks, between about 70 weeks and about 80 weeks, between about 70 weeks and about 75 weeks, or between about 75 weeks and about 80 weeks. In some aspects, the length of the further (e.g., second) dosing cycle is between about 10 weeks and about 40 weeks. In some aspects, the length of the further (e.g., second) dosing cycle is between about 15 weeks and about 25 weeks. In particular aspects, the length of the further (e.g., second) dosing cycle is about 20 weeks.

Any of the uses described herein may be used to treat GVHD in any organ, including the intestines, skin, and the liver. In some aspects, the GVHD is intestinal GVHD.

Any of the uses described herein may prevent GVHD, e.g., acute GVHD or chronic GVHD. The method, composition, or use may result in improved prophylaxis as compared to treatment without the IL-22 Fc fusion protein. For example, the method, composition, or use may result in improved prophylaxis as compared to standard of care for acute or chronic GVHD.

Any of the uses described herein may prevent Grade II-IV acute GVHD, for example, as assessed by the MAGIC GVHD Target Organ Staging (see, e.g., Harris et al. *supra*). In some embodiments, the methods, compositions, or uses prevent Grade II-IV acute GVHD, as assessed by the MAGIC GVHD Target Organ Staging. In some aspects, the uses prevent Grade II-IV acute GVHD, as assessed by the MAGIC GVHD Target Organ Staging, at Day 100 after the allo-HSCT. In some aspects, the uses prevent Grade II-IV acute GVHD, as assessed by the MAGIC GVHD Target Organ Staging, at Day 180 after the allo-HSCT.

Any of the uses described herein may reduce the incidence of Stage 1, Stage 2, Stage 3, or Stage 4 acute GVHD of the skin, gut, and/or liver, for example, as assessed by the MAGIC GVHD Target Organ Staging..

Any of the uses described herein may reduce the incidence of Grade I, Grade II, Grade III, or Grade IV acute GVHD, for example, as assessed by the MAGIC GVHD Target Organ Staging.

Any of the uses described herein may (i) improve the overall survival of the subject (e.g., at Day 180 after the allo-HSCT); (ii) improve the non-relapse mortality (NRM) rate of the subject (e.g., at Day 180 after the allo-HSCT); and/or (iii) improve the lower GI acute GVHD-free survival rate (e.g., at Day 100 after the allo-HSCT), as compared to treatment without the IL-22 Fc fusion protein.

Any of the uses described herein may reduce the cumulative incidence of Grade II-IV aGVHD, e.g., by Day 180 post-transplant, for example, as compared to treatment without the IL-22 Fc fusion protein.

Any of the uses described herein may reduce the cumulative incidence of Grade II-IV aGVHD by Day 100 post-transplant, for example, as compared to treatment without the IL-22 Fc fusion protein.

Any of the uses described herein may improve the OS rate, e.g., at Day 180 post-transplant, for example, as compared to treatment without the IL-22 Fc fusion protein (e.g., with no GVHD prophylaxis or with a standard-of-care prophylaxis (e.g. immunosuppressive agent)).

Any of the uses described herein may improve the NRM rate, e.g., at Day 180 post-transplant, for example, as compared to treatment without the IL-22 Fc fusion protein.

Any of the uses described herein may improve the lower GI aGVHD-free survival rate at Day 180 post-transplant, for example, as compared to treatment without the IL-22 Fc fusion protein.

Any of the uses described herein may reduce the cumulative incidence of Grade III-IV aGVHD by Day 100 and Day 180 post-transplant, for example, as compared to treatment without the IL-22 Fc fusion protein.

Any of the uses described herein may reduce the cumulative incidence of Stage 1-4 organ-specific aGVHD (skin, gut, and liver) by Day 100 and Day 180 post-transplant, for example, as compared to treatment without the IL-22 Fc fusion protein.

Any of the uses described herein may reduce the cumulative incidence of cGVHD, e.g., as independently assessed according to the National Institutes of Health Chronic GVHD Diagnosis and Staging score, for example, by Day 365 post-transplant, for example, as compared to treatment without the IL-22 Fc fusion protein.

Any of the uses described herein may improve the OS rate at Day 365 post-transplant, for example, as compared to treatment without the IL-22 Fc fusion protein.

Any of the uses described herein may improve the NRM rate at Day 365 post-transplant, for example, as compared to treatment without the IL-22 Fc fusion protein.

Any of the uses described herein may improve the disease-free survival (DFS) rate, e.g., at Day 365 post-transplant, for example, as compared to treatment without the IL-22 Fc fusion protein.

Any of the uses described herein may improve the GVHD-free/relapse-free survival (GRFS) rate at Day 365 post-transplant, for example, as compared to treatment without the IL-22 Fc fusion protein.

Any of the uses described herein may reduce the cumulative incidence of corticosteroid-refractory aGVHD, e.g., by Day 180 post-transplant, for example, as compared to treatment without the IL-22 Fc fusion protein.

Any of the uses described herein may reduce the systemic corticosteroid use for the treatment of aGVHD, e.g., by Day 100 and Day 180 post-transplant, for example, as compared to treatment without the IL-22 Fc fusion protein.

In the event a subject develops lower GI aGVHD, in someaspects, any of the uses described herein may reduce the lower GI aGVHD stage, improve the lower GI aGVHD partial response rate, and/or improve the lower GI aGVHD complete response rate, e.g., at Day 190 post-transplant, for example, as compared to treatment without the IL-22 Fc fusion protein.

Any of the uses described herein may improve the time from HSCT to neutrophil engraftment, for example, as compared to treatment without the IL-22 Fc fusion protein.

Any of the uses described herein may reduce the proportion of patients with high-risk GVHD at the time of aGVHD diagnosis, for example, as compared to treatment without the IL-22 Fc fusion protein.

Any of the uses described herein may improve the maximum oral mucositis grade, for example, as compared to treatment without the IL-22 Fc fusion protein.

Any of the uses described herein may reduce the cumulative days of total parenteral nutrition use, for example, as compared to treatment without the IL-22 Fc fusion protein.

### 1. Exemplary IL-22 Fc Fusion Proteins for Use

Any suitable IL-22 Fc fusion protein can be used herein. In general, the IL-22 Fc fusion proteins include an IL-22 polypeptide linked to an Fc region by a linker. Any of the IL-22 Fc fusion proteins described in U.S. Patent No. 9,815,880, may be used in the uses described herein. In some aspects of any of the preceding IL-22 Fc fusion proteins, the Fc region is not glycosylated. In some aspects, the amino acid residue at position 297 as in the EU index of the Fc region is Gly. In some aspects, the amino acid residue at position 297 as in the EU index of the Fc region is Ala. In some aspects, the amino acid residue at position 299 as in the EU index of the Fc region is Ala, Gly, or Val. In some aspects, the Fc region comprises the CH2 and CH3 domain of IgG1 or IgG4. In some aspects, the Fc region comprises the CH2 and CH3 domain of IgG4.

In some embodiments, the IL-22 Fc fusion protein comprises the amino acid sequence of SEQ ID NO:8. In some embodiments, the IL-22 Fc fusion protein consists of the amino acid sequence of SEQ ID NO:8.

Any of the preceding IL-22 Fc fusion proteins can be a dimeric IL-22 Fc fusion protein. In other embodiments, any of the preceding IL-22 Fc fusion proteins can be a monomeric IL-22 Fc fusion protein.

Any of the preceding IL-22 Fc fusion proteins can include a human IL-22 polypeptide.

In certain aspects, any of the IL-22 Fc fusion proteins described herein binds to and induces IL-22 receptor activity or signaling and/or is an agonist of IL-22 receptor activity.

In some aspects of any of the preceding aspects, the composition has an average sialic acid content in the range of 8 to 9 moles of sialic acid per mole of the IL-22 Fc fusion protein. In some aspects, the composition has an average sialic acid content of 8 or 9 moles of sialic acid per mole of the IL-22 Fc fusion protein. In some aspects, the composition has an average sialic acid content of 8 moles of sialic acid per mole of the IL-22 Fc fusion protein. In other embodiments, the composition has an average sialic acid content of 9 moles of sialic acid per mole of the IL-22 Fc fusion protein.

In any of the compositions described herein, the sialic acid may be N-acetylneuraminic acid (NANA).

Any of the compositions may have an average NGNA content of less than 1 mole of NGNA per mole of the IL-22 Fc fusion protein.

In some aspects of any of the preceding aspects, the composition has an average N-glycolylneuraminic acid (NGNA) content of less than 1 mole of NGNA per mole of the IL-22 Fc fusion protein.

In some aspects of any of the preceding aspects, the composition is a liquid composition.

In some aspects of any of the preceding aspects: (i) the IL-22 Fc fusion protein has a maximum observed concentration ($C_{max}$) of about 8,000 ng/mL to about 19,000 ng; (ii) the IL-22 Fc fusion protein has an area under the serum concentration-time curve from time 0 to the last measureable time point ($AUC_{last}$) of about 7,000 day·ng/mL to about 25,000 day·ng/mL; and/or (iii) the IL-22 Fc fusion protein has a clearance (CL) of about 40 mL/kg/day to about 140 mL/kg/day. In some embodiments, the $C_{max}$, $AUC_{last}$, and/or CL is assessed following intravenous administration of about 1,000 µg/kg of the IL-22 Fc fusion protein to a CD1 mouse.

In any of the compositions, the IL-22 polypeptide may include N-glycans having monoantennary, biantennary, triantennary, and/or tetraantennary structure. In some aspects: (i) about 0.1% to about 2% of the N-glycans have monoantennary structure; (ii) about 10% to about 25% of the N-glycans have biantennary structure; (iii) about 25% to about 40% of the N-glycans have triantennary structure; and/or (iv) about 30% to about 51% of the N-glycans have tetraantennary structure. In some aspects (i) 0.1% to 2% of the N-glycans have monoantennary structure; (ii) 10% to 25% of the N-glycans have biantennary structure; (iii) 25% to 40% of the N-glycans have triantennary structure; and/or (iv) 30% to 51% of the N-glycans have tetraantennary structure.

In any of the compositions, the IL-22 Fc fusion protein may include N-glycans including zero, one, two, three, or four galactose moieties. In some aspects: (i) about 9% to about 32% of the N-glycans include zero galactose moieties; (ii) about 10% to about 20% of the N-glycans include one galactose moiety; (iii) about 8% to about 25% of the N-glycans include two galactose moieties; (iv) about 12% to about 25% of the N-glycans include three galactose moieties; and/or (v) about 12% to about 30% of the N-glycans include four galactose moieties. In some aspects: (i) 9% to 32% of the N-glycans include zero galactose moieties; (ii) 10% to 20% of the N-glycans include one galactose moiety; (iii) 8% to 25% of the N-glycans include two galactose moieties; (iv) 12% to 25% of the N-glycans include three galactose moieties; and/or (v) 12% to 30% of the N-glycans include four galactose moieties.

In any of the compositions, the IL-22 Fc fusion protein may include N-glycans including zero, one, two, three, or four sialic acid moieties. In some aspects: (i) about 12% to about 35% of the N-glycans include zero sialic acid moieties; (ii) about 10% to about 30% of the N-glycans include one sialic acid moiety; (iii) about 10% to about 30% of the N-glycans include two sialic acid moieties; (iv) about 10% to about 30% of the N-glycans include three sialic acid moieties; and/or (v) about 1% to about 20% of the N-glycans include four sialic acid moieties. In some aspects: (i) 12% to 35% of the N-glycans include zero sialic acid moieties; (ii) 10% to 30% of the N-glycans include one sialic acid moiety; (iii) 10% to 30% of the N-glycans include two sialic acid moieties; (iv) 10% to 30% of the N-glycans include three sialic acid moieties; and/or (v) 1% to 20% of the N-glycans include four sialic acid moieties.

In any of the compositions, (i) the IL-22 polypeptide may include about 0% to about 10% N-glycans including a terminal mannose moiety; and/or (ii) the IL-22 polypeptide includes about 30% to about 55% N-glycans including a terminal N-acetylglucosamine (GlcNAc) moiety. In some aspects, (i) the IL-22 polypeptide includes 0% to 10% N-glycans including a terminal mannose moiety; and/or (ii) the IL-22 polypeptide includes 30% to 55% N-glycans including a terminal GlcNAc moiety. In some aspects, the IL-22 polypeptide includes 0% to 10% N-glycans including a terminal mannose moiety. In some embodiments, the IL-22 polypeptide includes 30% to 55% N-glycans including a terminal GlcNAc moiety.

In any of the compositions, the N-glycans may include one, two, three, or four terminal GlcNAc moieties. In some aspects: (i) about 1% to about 20% of the N-glycans include one terminal GlcNAc moiety; (ii) about 1% to about 20% of the N-glycans include two terminal GlcNAc moieties; (iii) about 5% to about 25% of the N-glycans include three terminal GlcNAc moieties; and/or (iv) about 0% to about 15% of the N-glycans include four terminal GlcNAc moieties. In some aspects: (i) 1% to 20% of the N-glycans include one terminal GlcNAc moiety; (ii) 1% to 20% of the N-glycans include two terminal GlcNAc moieties; (iii) 5% to 25% of the N-glycans include three terminal GlcNAc moieties; and/or (iv) 0% to 15% of the N-glycans include four terminal GlcNAc moieties.

In any of the compositions, (i) the IL-22 polypeptide may include about 20% to about 45% N-glycans including a terminal galactose (Gal) moiety; and/or (ii) the N-glycans include one, two, or three terminal Gal moieties. In some aspects, (i) the IL-22 polypeptide includes 20% to 45% N-glycans including a terminal Gal moiety; and/or (ii) the N-glycans include one, two, or three terminal Gal moieties.

In any of the compositions: (i) about 15% to about 30% of the N-glycans may include one terminal Gal moiety; (ii) about 1% to about 15% of the N-glycans may include two terminal Gal moieties; and/or (iii) about 0.1% to about 6% of the N-glycans may include three terminal Gal moieties. In some aspects: (i) 15% to 30% of the N-glycans include one terminal Gal moiety; (ii) 1% to 15% of the N-glycans include two terminal Gal moieties; and/or (iii) 0.1% to 6% of the N-glycans include three terminal Gal moieties.

In any of the compositions: (i) the IL-22 polypeptide may include N-glycans including galactose N-acetylglucosamine (LacNAc) repeats; (ii) the IL-22 polypeptide may include N-glycans including fucosylated N-glycans; and/or (iii) the IL-22 polypeptide may include N-glycans including afucosylated N-glycans.

Any suitable concentration of the IL-22 Fc fusion protein may be used. For example, in some aspects, the concentration of the IL-22 Fc fusion protein may be about 0.5 mg/mL to about 20 mg/mL. In some aspects, the concentration of the IL-22 Fc fusion protein is about 0.5 mg/mL to about 5 mg/mL. In someaspects, the concentration of the IL-22 Fc fusion protein is about 1 mg/mL. In some aspects, the concentration of the IL-22 Fc fusion protein is about 8 mg/mL to about 12 mg/mL. In some aspects, the concentration of the IL-22 Fc fusion protein is about 10 mg/mL.

**a) Glycosylation variants**

In certain aspects, an IL-22 Fc fusion protein described herein is altered to increase or decrease the extent to which the fusion protein or a portion thereof (e.g., the Fc portion of the fusion protein) is glycosylated. Addition or deletion of glycosylation sites to a protein may be conveniently accomplished by altering the amino acid sequence such that one or more glycosylation sites is created or removed.

Where the fusion protein comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody or the Fc region of an antibody may be made in order to create Fc variants with certain improved properties.

The amount of fucose attached to the CH2 domain of the Fc region can be determined by calculating the average amount of fucose within the sugar chain at Asn297, relative to the sum of all glycostructures attached to Asn 297 or N297 (e. g. complex, hybrid and high mannose structures) as measured by MALDI-TOF mass spectrometry, as described in WO 2008/077546, for example. Asn297 refers to the asparagine residue located at about position 297 in the Fc region (EU numbering of Fc region residues); however, Asn297 may also be located about $\pm$ 3 amino acids upstream or downstream of position 297, i.e., between positions 294 and 300, due to minor sequence variations in antibodies. Such fucosylation variants may have improved ADCC function. See, e.g., US Patent Publication Nos. US 2003/0157108; US 2004/0093621. Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Led 3 CHO cells deficient in protein fucosylation (Ripka et al. Arch. Biochem. Biophys. 249:533-545 (1986); U.S. Pat. Appl. No. US 2003/0157108 A1; and WO 2004/056312 A1, especially at Example 11), and knockout cell lines, such as alpha-1,6-fucosyltransferase gene, *FUT8,* knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO2003/085107).

Antibodies variants are further provided with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878; US Patent No. 6,602,684; and US 2005/0123546. Antibody variants with at least one galactose residue in the oligosaccharide attached to the Fc region are also provided. Such antibody variants may have improved CDC function. Such antibody variants are described, e.g., in WO 1997/30087; WO 1998/58964; and WO 1999/22764.

**b) Fc region variants**

In certain aspects, one or more amino acid modifications may be introduced into the Fc region of an Fc fusion protein provided herein, thereby generating an Fc region variant. The Fc region variant may comprise a human Fc region sequence (e.g., a human IgG1, IgG2, IgG3, or IgG4 Fc region) comprising an amino acid modification (e.g., a substitution) at one or more amino acid positions. For example, the hinge may include a Ser to Pro substitution, for example, as shown in the bolded and underlined Pro residue in the amino acid sequence of CP**P**CP (SEQ ID NO:31). Such a Ser to Pro substitution may increase the stability of the molecule.

In certain aspects, an Fc variant possesses some but not all effector functions, which make it a desirable candidate for applications in which the half-life of the antibody or a fusion protein comprising an Fc region *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody or Fc lacks FcγR binding (hence likely lacking ADCC activity), but retains FcRn binding ability. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch et al., Annu. Rev. Immunol. 9:457-492 (1991). Nonlimiting examples of *in vitro* assays to assess ADCC activity of a molecule of interest is described in U.S. Patent No. 5,500,362 (see, e.g. Hellstrom et al., Proc. Nat'l Acad. Sci. USA 83:7059-7063 (1986) and Hellstrom et al., Proc. Nat'l Acad. Sci. USA 82:1499-1502 (1985); U.S. Patent No. 5,821,337 (see Bruggemann et al., J. Exp. Med. 166:1351-1361 (1987)). Alternatively, non-radioactive assays methods may be employed (see, for example, ACTI™ non-radioactive cytotoxicity assay for flow cytometry (CellTechnology, Inc. Mountain View, CA; and CYTOTOX 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes et al. Proc. Nat'l Acad. Sci. USA 95:652-656 (1998). C1q binding assays may also be carried out to confirm that the antibody or Fc is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg et al., Blood 101:1045-1052 (2003); and Cragg etal., Blood 103:2738-2743 (2004)). FcRn binding and *in vivo* clearance/half-life determinations can also be performed using methods known in the art (see, e.g., Petkova et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody or Fc variants with improved or diminished binding to FcRs are described. (See, e.g., U.S. Patent No. 6,737,056; WO 2004/056312, and Shields et al., J. Biol. Chem. 9(2): 6591-6604 (2001).)

In certain aspects, an IL-22 Fc fusion protein comprises an Fc variant with one or more amino acid substitutions which reduce ADCC, e.g., substitution at position 297 of the Fc region to remove the N-glycosylation site and yet retain FcRn binding activity (EU numbering of residues).

In some aspects, alterations are made in the Fc region that result in diminished C1q binding and/or Complement Dependent Cytotoxicity (CDC), e.g., as described in US Patent No. 6,194,551, WO 99/51642, and Idusogie et al. J. Immunol. 164: 4178-4184 (2000).

Antibodies with increased half-lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

See also Duncan & Winter, Nature 322:738-40 (1988); U.S. Patent No. 5,648,260; U.S. Patent No. 5,624,821; and WO 94/29351 concerning other examples of Fc region variants.

**c) Cysteine engineered variants**

In certain aspects, it may be desirable to create cysteine engineered Fc fusion protein, in which one or more residues of the Fc region of an antibody are substituted with cysteine residues. In particular embodiments, the substituted residues occur at accessible sites of the Fc. By substituting those residues with cysteine, reactive thiol groups are thereby positioned at accessible sites of the Fc and may be used to conjugate the Fc to other moieties, such as drug moieties or linker-drug moieties, to create an immunoconjugate, as described further herein. For example, S400 (EU numbering) of the heavy chain Fc region can be substituted with Cys. See, e.g., U.S. Patent No. 7,521,541.

**2. Exemplary IL-22 Polypeptides**

Any suitable IL-22 polypeptide can be included in the IL-22 Fc fusion proteins used in the methods, uses, articles of manufacture, and kits described herein. For example, in any of the IL-22 Fc fusion proteins described herein, the IL-22 polypeptide can include a polypeptide comprising an amino acid sequence comprising SEQ ID NO:71 (human IL-22 with the endogenous IL-22 leader sequence), or a polypeptide comprising an amino acid sequence that has at least 80% sequence identity (e.g., at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity) with SEQ ID NO:71. In certain embodiments, the IL-22 polypeptide comprises an amino acid sequence comprising SEQ ID NO:4 (human IL-22 without a leader sequence) or a polypeptide comprising an amino acid sequence that has at least 80% sequence identity (e.g., at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, or at least 99% sequence identity) with SEQ ID NO:4. In certain embodiments, the IL-22 polypeptide comprises an amino acid sequence comprising SEQ ID NO:4.

The preparation of native IL-22 molecules, along with their nucleic acid and polypeptide sequences, can be achieved through methods known to those of ordinary skill in the art. For example, IL-22 polypeptides can be produced by culturing cells transformed or transfected with a vector containing IL-22 nucleic acid. It is, of course, contemplated that alternative methods, which are well known in the art, can be employed to prepare IL-22. For instance, the IL-22 sequence, or portions thereof, can be produced by direct peptide synthesis using solid-phase techniques (see, e.g., Stewart et al., 1969, Solid-Phase Peptide Synthesis, W.H. Freeman Co., San Francisco, Calif. (1969); Merrifield, J. Am. Chem. Soc., 1963, 85:2149-2154). In vitro protein synthesis can be performed using manual techniques or by automation. Automated synthesis can be accomplished, for instance, using an Applied Biosystems Peptide Synthesizer (Foster City, Calif.) using manufacturer's instructions. Various portions of IL-22 can be chemically synthesized separately and combined using chemical or enzymatic methods to produce the full-length IL-22.

IL-22 variants can be prepared by introducing appropriate nucleotide changes into the DNA encoding a native sequence IL-22 polypeptide, or by synthesis of the desired IL-22 polypeptide. Those skilled in the art will appreciate that amino acid changes can alter post-translational processes of IL-22, such as changing the number or position of glycosylation sites or altering the membrane anchoring characteristics.

Variations in the native sequence IL-22 polypeptides described herein can be made, for example, using any of the techniques and guidelines for conservative and non-conservative mutations set forth, for instance, in U.S. Pat. No. 5,364,934. Variations can be a substitution, deletion, or insertion of one or more codons encoding a native sequence or variant IL-22 that results in a change in its amino acid sequence as compared with a corresponding native sequence or variant IL-22. Optionally the variation is by substitution of at least one amino acid with any other amino acid in one or more of the domains of a native sequence IL-22 polypeptide. Guidance in determining which amino acid residue can be inserted, substituted or deleted without adversely affecting the desired activity can be found by comparing the sequence of the IL-22 with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions can optionally be in the range of 1 to 5 amino acids. The variation allowed can be determined by systematically making insertions, deletions or substitutions of amino acids in the sequence and testing the resulting variants for activity, for example, in the in vitro assay described in the Examples below.

## B. Methods of Making IL-22 Fc Fusion Proteins

The IL-22 Fc fusion proteins used in the methods, uses, articles of manufacture, and kits described herein can be prepared by any suitable method, e.g., culturing cells transformed or transfected with a vector containing a nucleic acid encoding an IL-22 Fc fusion protein, a fragment, or a variant thereof. In some embodiments, the IL-22 Fc fusion proteins are produced by a method described in International Patent Application No. PCT/US2019/015277. Host cells comprising any such vector are also provided. Any suitable host cell can be used, e.g., mammalian cells (e.g., CHO cells), *E. coli,* or yeast. Processes for producing any of the herein described IL-22 Fc fusion proteins are further provided and, in general, involve culturing host cells under conditions suitable for expression of the desired IL-22 Fc fusion protein and recovering, and optionally purifying, the desired IL-22 Fc fusion protein from the cell culture.

Host cells are transfected or transformed with expression or cloning vectors described herein for IL-22 polypeptide production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: A Practical Approach, M. Butler, ed. (IRL Press, 1991) and Sambrook et al., *supra.*

Methods of transfection are known to the ordinarily skilled artisan, for example, by $CaPO_4$ and electroporation.

Depending on the host cell used, transformation is performed using standard techniques appropriate to such cells. The calcium treatment employing calcium chloride, as described in Sambrook et al., supra, or electroporation is generally used for prokaryotes or other cells that contain substantial cell-wall barriers. Infection with *Agrobacterium tumefaciens* is used for transformation of certain plant cells, as described by Shaw et al., Gene, 23:315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transformations have been described in U.S. Pat. No. 4,399,216. Transformations into yeast are typically carried out according to the method of Van Solingen et al., J. Bact, 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, e.g., polybrene, polyornithine, can also be used. For various techniques for transforming mammalian cells, see Keown et al., Methods in Enzymology, 185:527-537 (1990) and Mansour et al., Nature, 336:348-352 (1988).

Recombinantly expressed polypeptides can be recovered from culture medium or from host cell lysates. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of a polypeptide of the present invention. Various methods of protein purification can be employed and such methods are known in the art and described for example in Deutscher, Methods in Enzymology, 182 (1990); Scopes, Protein Purification: Principles and Practice, Springer-Verlag, New York (1982). The purification step(s) selected will depend, for example, on the nature of the production process used and the particular polypeptide produced.

Alternative methods, which are well known in the art, can be employed to prepare a polypeptide. For example, a sequence encoding a polypeptide or portion thereof, can be produced by direct peptide synthesis using solid-phase techniques (see, e.g., Stewart et al., 1969, Solid-Phase Peptide Synthesis, W.H. Freeman Co., San Francisco, CA; Merrifield, J. 1963, Am. Chem. Soc., 85:2149-2154. *In vitro* protein synthesis can be performed using manual techniques or by automation. Automated synthesis can be accomplished, for instance, using an Applied Biosystems Peptide Synthesizer (Foster City, CA) using manufacturer's instructions. Various portions of a polypeptide of the present invention or portion thereof can be chemically synthesized separately and combined using chemical or enzymatic methods to produce the full-length polypeptide or portion thereof.

Suitable host cells for cloning or expressing the DNA in the vectors herein include prokaryote, yeast, or higher eukaryote cells. Suitable prokaryotes include eubacteria, such as Gram-negative or Gram-positive organisms, for example, Enterobacteriaceae such as E. coli. Various E. coli strains are publicly available, such as E. coli K12 strain MM294 (ATCC 31,446); E. coli X1776 (ATCC 31,537); E. coli strain W3110 (ATCC 27,325) and K5 772 (ATCC 53,635).

In addition to prokaryotes, eukaryotic microbes such as filamentous fungi or yeast are suitable cloning or expression hosts for IL-22-encoding vectors. Saccharomyces cerevisiae is a commonly used lower eukaryotic host microorganism.

Suitable host cells for the expression of glycosylated IL-22 are derived from multicellular organisms. Examples of invertebrate cells include insect cells such as Drosophila S2 and Spodoptera Sf9, as well as plant cells. Examples of useful mammalian host cell lines include Chinese hamster ovary (CHO) and COS cells. More specific examples include monkey kidney CV1 cells transformed by SV40 (COS-7, ATCC CRL 1651); human embryonic kidney cells (293 or 293 cells subcloned for growth in suspension culture, Graham et al., J. Gen Virol., 36:59 (1977)); Chinese hamster ovary cells/-DHFR (CHO, Urlaub and Chasin, Proc. Natl. Acad. Sci. USA, 77:4216 (1980)); mouse sertoli cells (TM4, Mather, Biol. Reprod., 23:243-251 (1980)); human lung cells (W138, ATCC CCL 75); human liver cells (Hep G2, HB 8065); and mouse mammary tumor cells (MMT 060562, ATCC CCL51). The selection of the appropriate host cell is deemed to be within the skill in the art.

## C. Assays

The compositions (e.g., IL-22 Fc fusion proteins, or pharmaceutical compositions thereof) for use described herein may be identified, screened for, or characterized for their physical/chemical properties and/or biological activities by various assays known in the art.

### 1. Binding assays and other assays

In one aspect, an IL-22 Fc fusion protein is tested for its receptor binding activity, e.g., by known methods such as ELISA, western blotting analysis, cell surface binding by Scatchard, surface plasmon resonance. In another aspect, competition assays may be used to identify an antibody that competes with the IL-22 Fc fusion protein for binding to the IL-22 receptor. In a further aspect, an IL-22 Fc fusion protein of the invention can be used for detecting the presence or amount of IL-22 receptor or IL22-Binding Protein (soluble receptor) present in a biological sample. In a further aspect, an IL-22 Fc fusion protein of the invention can be used for detecting the presence or amount of IL-22 receptor present in a biological sample. In certain embodiments, the biological sample is first blocked with a non-specific isotype control antibody to saturate any Fc receptors in the sample. Exemplary assays are described in Example 1 of International Patent Application No. International Patent Application No. PCT/US2019/015268, which is incorporated herein by reference in its entirety.

*2. Activity assays*

In one aspect, assays are provided for identifying biological activity of a composition (e.g., an IL-22 Fc fusion protein or a pharmaceutical composition thereof). Biological activity of an IL-22 polypeptide or IL-22 Fc fusion protein in a composition (e.g., a pharmaceutical composition) may include, e.g., binding to IL-22 receptor, stimulating IL-22 signaling, and inducing STAT3, or REG3 (also known as PAP or HIP/PAP (hepatocarcinoma-intestine-pancrease/pancreatic associated protein) expression. Further, in the case of a cardiovascular disease or condition, the biological activity may include affecting the formation of atherosclerotic plaques, in particular to inhibit formation of atherosclerotic plaque formation. Inhibition of plaque formation can be assessed by any suitable imaging method known to those of ordinary skill in the art.

*3. Stability assays*

In one aspect, assays are provided for determining the stability of a composition (e.g., an IL-22 Fc fusion protein or a pharmaceutical composition thereof). For example, a composition (e.g., a pharmaceutical composition) can be evaluated qualitatively and/or quantitatively in a variety of different ways, including evaluation of aggregate formation (for example, using size exclusion chromatography, by measuring turbidity, and/or by visual inspection); evaluation of ROS formation (for example, by using a light stress assay or an 2,2'-azobis(2-amidinopropane) dihydrochloride (AAPH) stress assay); oxidation of specific amino acid residues of the protein (for example, a Met residue of an IL-22 Fc fusion protein); by assessing charge heterogeneity using cation exchange chromatography, image capillary isoelectric focusing (icIEF) or capillary zone electrophoresis; amino-terminal or carboxy-terminal sequence analysis; mass spectrometric analysis; SDS-PAGE analysis to compare reduced and intact polypeptides (e.g., IL-22 Fc fusion proteins); peptide map (for example, tryptic or LYS-C) analysis; evaluating biological activity or target binding function of the protein (e.g., binding of an IL-22 Fc fusion protein to an IL-22 receptor); and the like. Instability may involve any one or more of: aggregation, deamidation (e.g., Asn deamidation), oxidation (e.g., Met oxidation and/or Trp oxidation), isomerization (e.g., Asp isomerization), clipping/hydrolysis/fragmentation (e.g., hinge region fragmentation), succinimide formation, unpaired cysteine(s), N-terminal extension, C-terminal processing, glycosylation differences, and the like.

**D. Pharmaceutical Formulations**

Also provided herein are compositions (e.g., pharmaceutical compositions) that include IL-22 Fc fusion proteins for use described herein. Any of the IL-22 Fc fusion proteins described herein can be used in the compositions. In some aspects, any of the pharmaceutical compositions described in International Patent Application No. PCT/US2019/015268 may be used herein. Any of the pharmaceutical formulations provided herein can be for use in preventing or treating GVHD (e.g., aGVHD).

Pharmaceutical formulations can be prepared using standard methods known in the art by mixing the active ingredient having the desired degree of purity with one or more optional pharmaceutically acceptable carriers (see, e.g., Remington's Pharmaceutical Sciences 16th edition, Osol, A. Ed. (1980) and Remington's Pharmaceutical Sciences 20th edition, ed. A. Gennaro, 2000, Lippincott, Williams & Wilkins, Philadelphia, Pa), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Optionally, the formulation contains a pharmaceutically acceptable salt, preferably sodium chloride, and preferably at about physiological concentrations.

Optionally, the formulations of the invention can contain a pharmaceutically acceptable preservative. In some embodiments the preservative concentration ranges from 0.1 to 2.0%, typically v/v. Suitable preservatives include those known in the pharmaceutical arts. Benzyl alcohol, phenol, m-cresol, methylparaben, benzalkonium chloride and propylparaben are preferred preservatives. Optionally, the formulations of the invention can include a pharmaceutically acceptable surfactant, e.g., at a concentration of 0.005 to 0.02%.

The formulation herein can also contain more than one active compound as necessary for the particular indication being treated, preferably those with complementary activities that do not adversely affect each other. Such molecules are suitably present in combination in amounts that are effective for the purpose intended.

Exemplary lyophilized formulations are described in US Patent No. 6,267,958. Aqueous formulations include those described in US Patent No. 6,171,586 and WO2006/044908, the latter formulations including a histidine-acetate buffer.

The formulation herein may also contain more than one active ingredients as necessary for the particular indication being treated, preferably those with complementary activities that do not adversely affect each other. For example, it may be desirable to further provide a steroid, TNF antagonist or other anti-inflammatory therapeutics. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles, and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the IL-22 Fc fusion protein, which matrices are in the form of shaped articles, e.g., films or microcapsules. Examples of sustained-release matrices include polyesters, hydrogels (for example, poly(2-hydroxyethylmethacrylate), or poly(vinylalcohol)), polylactides (U.S. Pat. No. 3,773,919), copolymers of L-glutamic acid and γethyl-L-glutamate, non-degradable ethylene -vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37 $^0$C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

A pharmaceutical composition for topical administration can be formulated, for example, in the form of a topical gel. See e.g., US 4,717,717; US 5,130,298; US 5,427,778; US 5,457,093; US 5,705,485; US 6,331,309; and WO2006/138,468. In certain embodiments, the composition can be formulated in the presence of cellulose derivatives. In certain other embodiments, the topical formulation can be reconstituted from lyophilized formulation with sufficient buffer or diluent before administration.

For obtaining a gel formulation, the IL-22 polypeptide or IL-22 Fc fusion protein formulated in a liquid composition may be mixed with an effective amount of a water-soluble polysaccharide or synthetic polymer to form a gel (e.g., a gelling agent) such as polyethylene glycol to form a formulation of the proper viscosity to be applied topically. The polysaccharide or gelling agent that may be used includes, for example, cellulose derivatives such as etherified cellulose derivatives, including alkyl celluloses, hydroxyalkyl celluloses, and alkylhydroxyalkyl celluloses, for example, methylcellulose, hydroxyethyl cellulose, carboxymethyl cellulose, hydroxypropyl methylcellulose, and hydroxypropyl cellulose; Sodium carboxymethyl cellulose; POE-POP block polymers: poloxamer USP in various grades; Hyaluronic acid; Polyacrylic acid such as carbopol 940; starch and fractionated starch; agar; alginic acid and alginates; gum Arabic; pullullan; agarose; carrageenan; dextrans; dextrin; fructans; inulin; mannans; xylans; arabinans; chitosans; glycogens; glucans; and synthetic biopolymers; as well as gums such as xanthan gum; guar gum; locust bean gum; gum Arabic; tragacanth gum; and karaya gum; and derivatives, combinations and mixtures thereof. In one embodiment of the invention, the gelling agent herein is one that is, e.g., inert to biological systems, nontoxic, simple to prepare, and/or not too runny or viscous, and will not destabilize the IL-22 polypeptide or IL-22 Fc fusion held within it.

In certain aspects, the polysaccharide is an etherified cellulose derivative, in another embodiment one that is well defined, purified, and listed in USP, e.g., methylcellulose and the hydroxyalkyl cellulose derivatives, such as hydroxypropyl cellulose, hydroxyethyl cellulose, and hydroxypropyl methylcellulose (all referred to as cellulosic agents). In some aspects, the polysaccharide is hydroxyethyl methylcellulose or hydroxypropyl methylcellulose.

The polyethylene glycol useful for gelling is typically a mixture of low and high molecular weight polyethylene glycols to obtain the proper viscosity. For example, a mixture of a polyethylene glycol of molecular weight 400-600 with one of molecular weight 1500 would be effective for this purpose when mixed in the proper ratio to obtain a paste.

The term "water soluble" as applied to the polysaccharides and polyethylene glycols is meant to include colloidal solutions and dispersions. In general, the solubility of the cellulose derivatives is determined by the degree of substitution of ether groups, and the stabilizing derivatives useful herein should have a sufficient quantity of such ether groups per anhydroglucose unit in the cellulose chain to render the derivatives water soluble. A degree of ether substitution of at least 0.35 ether groups per anhydroglucose unit is generally sufficient. Additionally, the cellulose derivatives may be in the form of alkali metal salts, for example, the Li, Na, K, or Cs salts.

The formulations to be used for *in vivo* administration are generally sterile. Sterility may be readily accomplished, e.g., by filtration through sterile filtration membranes.

The compounds described herein (e.g., IL-22 Fc fusion proteins and compositions (e.g., pharmaceutical compositions) thereof) for prevention or treatment of GVHD (e.g., acute GVHD) are typically administered by intravenous injection or subcutaneous injection. In some aspects the compounds described herein (e.g., IL-22 Fc fusion proteins and compositions (e.g., pharmaceutical compositions) thereof) for prevention or treatment of GVHD (e.g., acute GVHD) are administered by intravenous injection.

Other methods of administration can also be used, which includes but is not limited to, topical, parenteral, intraperitoneal, intrapulmonary, intranasal, ocular, intraocular, intravitreal, intralesional, intracerobrospinal, intra-articular, intrasynovial, intrathecal, oral, or inhalation administration. Parenteral infusions include intramuscular, intravenous, intraarterial, intraperitoneal, or subcutaneous administration. In addition, the compounds described herein are administered to a human subject, in accord with known methods, such as intravenous administration as a bolus or by continuous infusion over a period of time.

### E. Articles of Manufacture and Kits

In another aspect, an article of manufacture or kit containing materials useful for the methods and uses described herein is provided. The article of manufacture may include any of the compositions (e.g., IL-22 Fc fusion proteins or compositions thereof (e.g., pharmaceutical compositions)) provided herein. The articles of manufacture and kits may include a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). In some embodiments, at least one active agent in the composition is an IL-22 Fc fusion protein. The label or package insert indicates that the composition is used for treating the condition of choice. In some embodiments, the article of manufacture or the containers are protected from light. The articles of manufacture can include any of the compositions (e.g., pharmaceutical compositions) described herein.

### EXAMPLES

**Example 1: A Randomized, Double-Blind, Placebo-Controlled, Multicenter Study to Evaluate the Efficacy, Safety, and Pharmacokinetics of IL-22 Fc fusion protein (UTTR1147A) in Combination with Standard of Care in the Prevention of Acute Graft-Versus-Host Disease in Patients Undergoing Allogeneic Hematopoietic Stem Cell Transplantation**

Despite the use of SOC prophylaxis, Grade II-IV aGVHD develops in 35%-50% of patients who undergo HSCT, depending on the degree of HLA match, donor-recipient relatedness, conditioning regimen, source of graft, and aGVHD prophylactic regimen used. Patients with GI aGVHD have the highest rates of aGVHD-related morbidity and mortality. UTTR1147A is being developed as a novel non-immunosuppressive therapeutic, for example, and without limitation, to promote healing in the GI epithelia and/or restoring the GI barrier to help prevent development of aGVHD when given alone or in combination with SOC aGVHD prophylaxis.

Nonclinical data demonstrate that UTTR1147A has a beneficial effect on GI tract mucosal epithelia expressing IL-22R. Without wishing to be bound by one particular theory or mechanism of action, UTTR1147A may strengthen the intestinal barrier by increasing epithelial cell proliferation, stimulating mucus production, and/or decreasing crypt apoptosis through increasing intestinal production of REG3A, and is thus expected to be efficacious in the prevention of aGVHD.

This study will enroll patients undergoing HLA-matched related, HLA-matched unrelated, or single-antigen HLA-mismatched unrelated HSCT.

Patients will be randomly assigned in a 1:1 ratio to receive either UTTR1 147A 60 µg/kg IV Q2W or placebo IV Q2W for a total of 6 doses. Patients will receive SOC aGVHD prophylaxis treatment consisting of a CNI (cyclosporine or tacrolimus) plus methotrexate. Adjunctive administration of ATG prior to HSCT will be allowed per institutional guidelines. Dosing with study drug (UTTR1147A or placebo) will continue through the end of the treatment period (total of six doses) or until a criterion for study treatment discontinuation has been met (including for confirmed primary disease relapse or progression per investigator assessment or study drug missed between Day 1 and Day 15 post-transplant), whichever occurs first. Patients who discontinue study drug prematurely will return to the clinic within 1 day (+ 3 days) of study treatment discontinuation and will continue to be followed through the study completion visit (Day 365 post-transplant).

Patients will be assessed for Grade II-IV aGVHD, stage of organ-specific aGVHD, corticosteroid-refractory aGVHD, cGVHD, primary disease relapse or progression, and survival. Patients who develop symptoms of Grade II-IV aGVHD should return to the clinic for an aGVHD evaluation visit within 3 days after starting systemic corticosteroid therapy. In the course of Grade II-IV aGVHD evaluation, investigators may perform a skin biopsy for diagnosis of skin aGVHD and an endoscopic biopsy for diagnosis of lower GI aGVHD.

All patients will be closely monitored for adverse events throughout the study. Patients will be monitored for safety through changes from baseline in vital signs, laboratory results, and physical examination findings throughout the study and through monitoring of adverse events. All adverse events will be reported until 70 days after the final dose of study drug.

## Efficacy Objectives

For efficacy endpoint evaluation, aGVHD stage and grade will be assessed according to the MAGIC GVHD Target Organ Staging (Harris et al. *supra*), with aGVHD stage assessed on the basis of investigator quantification of patient signs and symptoms, and aGVHD grade assessed by the Sponsor on the basis of aGVHD stage.

## Primary Efficacy Objective

The primary efficacy objective for this study is to evaluate the efficacy of UTTR1 147A plus SOC versus placebo plus SOC, on the basis of the following endpoint:

- Cumulative incidence of Grade II-IV aGVHD by Day 180 post-transplant

## Secondary Efficacy Objective

The secondary efficacy objective for this study is to evaluate the efficacy of UTTR1 147A plus SOC versus placebo plus SOC, on the basis of the following endpoints:

- Cumulative incidence of Grade II-IV aGVHD by Day 100 post-transplant

- Overall survival (OS) rate at Day 180 post-transplant, defined as the proportion of patients who have not experienced death from any cause at Day 180 post-transplant

- Non-relapse mortality (NRM) rate at Day 180 post-transplant, defined as proportion of patients who experienced death without primary disease relapse or progression (as defined in Section 2.1.4.1 below) at Day 180 post-transplant

- Lower GI aGVHD-free survival rate at Day 180 post-transplant, defined as proportion of patients who have not experienced lower GI aGVHD or death from any cause at Day 180 post-transplant

## Exploratory Efficacy Objective

The exploratory efficacy objective for this study is to evaluate the efficacy of UTTR1 147A plus SOC versus placebo plus SOC, on the basis of the following endpoints:

- Cumulative incidence of Grade III-IV aGVHD by Day 100 and Day 180 post-transplant

- Cumulative incidence of Stage 1-4 organ-specific aGVHD (skin, gut, and liver) by Day 100 and Day 180 post-transplant

- Cumulative incidence of cGVHD, as independently assessed according to the National Institutes of Health Chronic GVHD Diagnosis and Staging score (see Jagasia et al. Biol. Blood Marrow Transplant. 21:389-401, 2015), on the basis of investigator quantification of patient cGVHD signs and symptoms, by Day 365 post-transplant

- OS rate at Day 365 post-transplant, defined as the proportion of patients who have not experienced death from any cause at Day 365 post-transplant

- NRM rate at Day 365 post-transplant, defined as proportion of patients who experienced death without primary disease relapse or progression (as defined in Section 2.1.4.1) at Day 365 post-transplant

- Disease-free survival (DFS) rate at Day 365 post-transplant, defined as the proportion of patients who have not experienced relapse or progression of primary disease (as defined in Section 2.1.4.1) or death from any cause at Day 365 post-transplant

- GVHD-free/relapse-free survival (GRFS) rate at Day 365 post-transplant, defined as proportion of patients who have not experienced Grade III-IV aGVHD, cGVHD requiring systemic immunosuppressive therapy, relapse or progression of primary disease (as defined in Section 2.1.4.1), or death from any cause at Day 365 post-transplant

- Cumulative incidence of corticosteroid-refractory aGVHD (as defined in Section 2.1.4.2) by Day 180 post-transplant

- Cumulative systemic corticosteroid use for the treatment of aGVHD by Day 100 and Day 180 post-transplant

- For patients who develop lower GI aGVHD:

  - Change in lower GI aGVHD stage from time of diagnosis to Day 180 post-transplant

  - Lower GI aGVHD partial response rate at Day 180 post-transplant, defined as the proportion of patients with > 1 decrease in stage and whose lower GI aGVHD stage has improved to Stage $\geq$ 1 at Day 180 post-transplant

  - Lower GI aGVHD complete response rate at Day 180 post-transplant, defined as the proportion of patients whose lower GI aGVHD stage has improved to Stage 0 at Day 180 post-transplant

- Time from HSCT to neutrophil engraftment, defined as ANC > 500/mm$^3$ for 3 different days or ANC > 2000/mm$^3$ for 1 day

- Proportion of patients with high-risk GVHD at time of aGVHD diagnosis, occurring up to Day 180 post-transplant, according to the Minnesota aGVHD Risk Scale on the basis of investigator quantification of organ involvement and Sponsor assessment of Minnesota risk score (see Section 3.3.1.2)

- Maximum oral mucositis grade, as determined by the investigator according to the National Cancer Institute Common Terminology Criteria for Adverse Events, Version 5.0 (NCI CTCAE v5.0) scale (see Section 3.3.3), by Day 100 post-transplant

- Cumulative days of total parenteral nutrition use by Day 180 post-transplant

**Key Definitions for the Study**

**Primary Disease Relapse or Progression**

Primary disease relapse or progression is characterized by either morphological or cytogenetic evidence of acute myeloid leukemia (AML) or myelodysplastic syndrome (MDS) consistent with pre-transplant features and defined as meeting any of the following criteria:

- Reappearance of leukemia blast cells in peripheral blood

- > 5% blasts in the bone marrow not attributable to another cause (e.g., bone marrow regeneration)

- Development of extramedullary leukemia or leukemic cells in the cerebral spinal fluid

- Reappearance of cytogenetic abnormalities present prior to transplantation

- For patients with MDS: appearance of previous or new dysplastic changes within the bone marrow, with or without falling donor chimerism

- Institution of any therapy with the intent to treat persistent, progressive, or minimal residual disease or relapsed disease, including, but not limited to, hypomethylating agents or donor lymphocyte infusion

**Corticosteroid-Refractory aGVHD**

Corticosteroid-refractory aGVHD is defined as meeting any of the following sets of criteria:

- Progression of aGVHD after 3 days of therapy with $\geq$ 2 mg/kg/day of prednisone (or equivalent)

- No improvement of aGVHD after 7 days of therapy with $\geq$ 2 mg/kg/day of prednisone (or equivalent)

- Skin and visceral organ-involved aGVHD and improvement in skin only after 7 days of therapy with > 2 mg/kg/day of prednisone (or equivalent)

**Rationale for UTTR1147A Dose and Schedule**

This dosing regimen was selected on the basis of safety, tolerability, efficacy, and PK data from evaluation of UTTR1147A in Study GA29468 in healthy volunteers (HVs) and Study GA29469 in HVs and patients with inflammatory bowel disease. UTTR1147A 60 $\mu$g/kg IV Q2W for six doses was tolerated in both HVs and patients with ulcerative colitis (UC). Dermatologic adverse events at this dose exposure level included dry skin, rash, and pruritus that were manageable and reversible. Additionally, the proposed UTTR1147A dosing regimen has been selected to maximize drug exposure to help promote intestinal barrier healing from damage due to the conditioning regimen and increase the likelihood of preventing aGVHD.

A preliminary Phase Ib PK analysis has demonstrated that UTTR1147A exposures in patients with UC were 25-29% lower than those in HVs, potentially due to leakage of UTTR1 147A in the gut. At 60 $\mu$g/kg IV Q2W, the mean maximum serum concentration observed ($C_{max}$) at Day 56 was 1070 ng/mL in HVs and 789 ng/mL in patients with UC; the area under the concentration-time curve $(AUC)_{2wks,D56-70}$ was 7460 day·ng/mL in HVs and 5310 day·ng/mL in patients with UC.

Exposures for safety factor calculations were based on the observed exposure from the highest tolerable dose and the predicted exposure of the 60 $\mu$g/kg Q2W regimen using a preliminary population PK model developed from the interim Phase Ib PK data in HVs. Because UTTR1147A administered 60 $\mu$g/kg IV Q2W was tolerated in HVs and patients with UC, and assuming HV PK exposure, the exposure-based safety factor is expected to be at least 1. However, it is also expected that an additional treatment arm can be added, using a dose of 120 $\mu$g/kg UTTR1147A for a total of six doses Q2W to account for altered PK exposure in allo-HSCT patients.

Further, patients with GI damage may have faster drug clearance than HVs, a phenomenon observed in studies with infliximab and other biologics (Brandse et al. Gastroenterology 149(2):350-355, 2015; Fausel et al. Ther. Clin. Risk Manag. 11:63-73, 2015; Rosen et al. Aliment. Pharmacol. Ther. 41(11):1094-1103, 2015). This was also observed for UTTR1 147A in patients with UC (Study GA29469), where the group mean trough concentrations ($C_{min}$, D28, $C_{min}$,D84) after the first and last dose of UTTR1 147A in the 60 $\mu$g/kg IV Q4W cohort were approximately 55% of the exposure observed in HVs, although $C_{max}$ values were comparable. The AUC profile for the first and last dose interval ($AUC_{0-28}$, $AUC_{56-84}$) in patients with UC were 81% and 67%, respectively, of the exposure observed in HVs. Similarly, patients who undergo myeloablative conditioning prior to HSCT may also experience faster UTTR1147A clearance due to conditioning-mediated damage to the GI tract. Therefore, given the expectation that UTTR1147A exposure in patients undergoing myeloablative conditioning prior to HSCT is lower than HVs, and the 60 $\mu$g/kg IV Q2W regimen is tolerated in this study, an additional UTTR1 147A dose cohort may be added, with the maximum exposure capped by the maximum exposure tolerated by HVs (60 $\mu$g/kg IV Q2W; maximum $C_{max}$ of 1810 ng/mL and $AUC_{0-14}$ of 4310 ng·day/mL) in Study GA29469. For example, the additional dose cohorts may include dosages of 90 $\mu$g/kg and/or 120 $\mu$g/kg of UTTR1147A (e.g., 90 $\mu$g/kg and/or 120 $\mu$g/kg IV Q2W of UTTR1147A).

**Inclusion Criteria**

Patients must meet the following criteria for study entry:

- Planned HLA (*HLA-A, HLA-B, HLA-C,* and *HLA-DRB1*)-matched (eight out of eight) related, planned HLA-matched (eight out of eight) unrelated, or single-antigen HLA-mismatched (seven out of eight) unrelated HSCT, from either peripheral blood or bone marrow stem cells, for patients with either of the following diagnoses:

  - AML in first complete remission per institutional criteria, with no circulating blasts and < 5% blasts in the bone marrow

  - High-risk MDS (as defined by the Revised International Prognostic Scoring System), with no circulating blasts and < 10% blasts in the bone marrow

In the randomized, double-blind, placebo-controlled phase, patients undergoing HLA-matched, related HSCT will be limited to 40% of the total study population.

- Planned myeloablative conditioning regimen per institutional guidelines

- Planned aGvHD prophylaxis consisting of a CNI (cyclosporine or tacrolimus) and methotrexate

**Standard-of-Care aGVHD Prophylaxis**

All patients will be given SOC prophylaxis treatment for aGVHD consisting of a CNI (cyclosporine or tacrolimus) and methotrexate post-transplant. CNI and methotrexate will be administered prior to study drug. ATG, with dose and administration per institutional guidelines, may be administered prior to HSCT. The levels of cyclosporine and tacrolimus will be monitored and dose adjustments may be made per institutional guidelines.

**Study Assessments**

Patients will be closely monitored for safety and tolerability throughout the study. Patients will be assessed for toxicity prior to each dose; dosing will occur only if the clinical assessment and local laboratory test values are acceptable.

**aGVHD Assessment**

aGVHD stage and grade will be assessed according to the MAGIC GVHD Target Organ Staging and risk determined according to the Minnesota aGVHD Risk Scale, on the basis of investigator quantification of patient aGVHD signs and symptoms.

**MAGIC GVHD Target Organ Staging**

In the MAGIC GVHD Target Organ Staging, each of four aGVHD target organs (skin, liver, upper GI tract, and lower GI tract) is assigned a stage based on severity of involvement. The stages range from 0 to 4, with Stage 4 being the most severe. An overall grade ranging from 0 to IV is then determined on the basis of stages for the four target organs, with Grade IV being the most severe (Harris et al. *supra*).

**Minnesota aGVHD Risk Scale**

The Minnesota aGVHD Risk Scale grades signs and symptoms of aGVHD using the Minnesota grading system to categorize patients who develop aGVHD as either high risk or standard risk on the basis of the number of involved organs and severity of aGVHD at the time of diagnosis (MacMillan et al. 2015). Identification of aGVHD risk is used as a predictor of response, survival, and transplant-related mortality.

**cGVHD Assessment**

cGVHD severity will be independently assessed according to the National Institutes of Health Chronic GVHD Diagnosis and Staging score, on the basis of investigator quantification of patient cGVHD signs and symptoms. The cGVHD target organs (skin, mouth, eyes, GI tract, liver, lungs, joints and fascia, and genital tract) are scored from 0 to 3, with 3 as the most severe (Jagasia et al. Biol. Blood Marrow Transplant. 21:389-401, 2015).

**Oral Mucositis Grade**

The oral mucositis grade will be determined by the investigator according to the NCI CTCAE v5.0 scale, which is dependent on both objective and subjective variables and measures symptomatic as well as functional components of oral mucositis. Investigators assess the degree of oral erythema and ulceration and the ability of patients to tolerate oral liquids.

**Example 2: Alternate IL-22 Fc Fusion Protein Dosing Regimen for Prevention of Acute GVHD**

This example describes an alternate dosing regimen for the study described in Example 1. Patients will be randomly assigned in a 1:1 ratio to receive either UTTR1147A 30 $\mu$g/kg IV Q2W or placebo IV Q2W for a total of 6 doses. All patients will receive SOC aGVHD prophylaxis treatment consisting of a CNI (cyclosporine or tacrolimus) plus methotrexate. The first dose of study drug (UTTR1147A or placebo) will be administered on Day 1 (+ 1 day) (i.e., 1 or 2 days) after HSCT (Day 0); subsequent doses will be administered Q2W through Day 71 post-transplant. In one example, the patient population for the study is the same patient population as described in Example 1. Any of the efficacy endpoints described in Example 1 can be assessed.

**Example 3: Alternate IL-22 Fc Fusion Protein Dosing Regimen for Prevention of Acute GVHD**

This example describes an alternate dosing regimen for the study described in Example 1. Patients will be randomly assigned in a 1:1 ratio to receive either UTTR1147A 90 $\mu$g/kg or 120 $\mu$g/kg IV Q2W or placebo IV Q2W for a total of 6 doses. All patients will receive SOC aGVHD prophylaxis treatment consisting of a CNI (cyclosporine or tacrolimus) plus methotrexate. The first dose of study drug (UTTR1147A or placebo) will be administered on Day 1 (+ 1 day) after HSCT (Day 0); subsequent doses will be administered Q2W through Day 71 post-transplant. In one example, the patient population for the study is the same patient population as described in Example 1. Any of the efficacy endpoints described in Example 1 can be assessed.

**Example 4: Alternate IL-22 Fc Fusion Protein Dosing Regimen for Prevention of Acute GVHD**

This example describes an alternate dosing regimen for the study described in Example 1. Patients will be randomly assigned in a 1:1 ratio to receive either UTTR1147A 30 $\mu$g/kg IV Q2W or placebo IV Q2W for a total of 6 doses. All patients will receive SOC aGVHD prophylaxis treatment consisting of a CNI (cyclosporine or tacrolimus) plus methotrexate. The first dose of study drug (UTTR1147A or placebo) will be administered on Day 2 after HSCT (Day 0); subsequent doses will be administered Q2W. In one example, the patient population for the study is the same patient population as described in Example 1. Any of the efficacy endpoints described in Example 1 can be assessed.

**Example 5: Alternate IL-22 Fc Fusion Protein Dosing Regimen for Prevention of Acute GVHD**

This example describes an alternate dosing regimen for the study described in Example 1. Patients will be randomly assigned in a 1:1 ratio to receive either UTTR1147A 60 $\mu$g/kg IV Q2W or placebo IV Q2W for a total of 6 doses. All patients will receive SOC aGVHD prophylaxis treatment consisting of a CNI (cyclosporine or tacrolimus) plus methotrexate. The first dose of study drug (UTTR1147A or placebo) will be administered on Day 2 after HSCT (Day 0); subsequent doses will be administered Q2W. In one example, the patient population for the study is the same patient population as described in Example 1. Any of the efficacy endpoints described in Example 1 can be assessed.

**Example 6: Alternate IL-22 Fc Fusion Protein Dosing Regimen for Prevention of Acute GVHD**

This example describes an alternate dosing regimen for the study described in Example 1. Patients will be randomly assigned in a 1:1 ratio to receive either UTTR1147A 90 $\mu$g/kg or 120 $\mu$g/kg IV Q2W or placebo IV Q2W for a total of 6 doses. All patients will receive SOC aGVHD prophylaxis treatment consisting of a CNI (cyclosporine or tacrolimus) plus methotrexate. The first dose of study drug (UTTR1147A or placebo) will be administered on Day 2 after HSCT (Day 0); subsequent doses will be administered Q2W. In one example, the patient population for the study is the same patient population as described in Example 1. Any of the efficacy endpoints described in Example 1 can be assessed.

**Example 7: Alternate IL-22 Fc Fusion Protein Dosing Regimen for Prevention of Acute GVHD**

This example describes an alternate dosing regimen for the study described in Example 1. Patients will be randomly assigned in a 1:1 ratio to receive either UTTR1147A 30 $\mu$g/kg IV Q2W or placebo IV Q2W for a total of 6 doses. All patients will receive SOC aGVHD prophylaxis treatment consisting of a CNI (cyclosporine or tacrolimus) plus methotrexate. The first dose of study drug (UTTR1147A or placebo) will be administered on Day 3 after HSCT (Day 0); subsequent doses will be administered Q2W. In one example, the patient population for the study is the same patient population as described in Example 1. Any of the efficacy endpoints described in Example 1 can be assessed.

**Example 8: Alternate IL-22 Fc Fusion Protein Dosing Regimen for Prevention of Acute GVHD**

This example describes an alternate dosing regimen for the study described in Example 1. Patients will be randomly assigned in a 1:1 ratio to receive either UTTR1147A 60 $\mu$g/kg IV Q2W or placebo IV Q2W for a total of 6 doses. All patients will receive SOC aGVHD prophylaxis treatment consisting of a CNI (cyclosporine or tacrolimus) plus methotrexate. The first dose of study drug (UTTR1147A or placebo) will be administered on Day 3 after HSCT (Day 0); subsequent doses will be administered Q2W. In one example, the patient population for the study is the same patient population as described in Example 1. Any of the efficacy endpoints described in Example 1 can be assessed.

**Example 9: Alternate IL-22 Fc Fusion Protein Dosing Regimen for Prevention of Acute GVHD**

This example describes an alternate dosing regimen for the study described in Example 1. Patients will be randomly assigned in a 1:1 ratio to receive either UTTR1147A 90 $\mu$g/kg or 120 $\mu$g/kg IV Q2W or placebo IV Q2W for a total of 6 doses. All patients will receive SOC aGVHD prophylaxis treatment consisting of a CNI (cyclosporine or tacrolimus) plus methotrexate. The first dose of study drug (UTTR1147A or placebo) will be administered on Day 3 after HSCT (Day 0); subsequent doses will be administered Q2W. In one example, the patient population for the study is the same patient population as described in Example 1. Any of the efficacy endpoints described in Example 1 can be assessed.

**Example 10: IL-22 Fc Fusion Protein Monotherapy for Prevention of Acute GVHD**

This study will evaluate the efficacy, safety, and pharmacokinetics of IL-22 Fc fusion protein (UTTR1147A) versus placebo when administered as a monotherapy (i.e., not in combination with SOC) for aGVHD prophylaxis to prevent aGVHD in patients undergoing allo-HSCT.

Patients will be randomly assigned in a 1:1 ratio to receive either UTTR1147A 60 $\mu$g/kg IV Q2W or placebo IV Q2W for six doses. The first dose of study drug (UTTR1147A or placebo) will be administered on Day 1 (+ 1 day) after HSCT (Day 0); subsequent doses will be administered Q2W through Day 71 post-transplant. In one example, the patient population for the study is the same patient population as described in Example 1. Any of the efficacy endpoints described in Example 1 can be assessed.

**Example 11: Alternate IL-22 Fc Fusion Protein Monotherapy Dosing Regimen for Prevention of Acute GVHD**

This example describes an alternate dosing regimen for the study described in Example 10. Patients will be randomly assigned in a 1:1 ratio to receive either UTTR1147A 30 $\mu$g/kg IV Q2W or placebo IV Q2W for a total of 6 doses. UTTR1147A is administered as a monotherapy. The first dose of study drug (UTTR1147A or placebo) will be administered on Day 1 (+ 1 day) after HSCT (Day 0); subsequent doses will be administered Q2W through Day 71 post-transplant. In one example, the patient population for the study is the same patient population as described in Example 1. Any of the efficacy endpoints described in Example 1 can be assessed.

**Example 12: Alternate IL-22 Fc Fusion Protein Monotherapy Dosing Regimen for Prevention of Acute GVHD**

This example describes an alternate dosing regimen for the study described in Example 10. Patients will be randomly assigned in a 1:1 ratio to receive either UTTR1147A 90 $\mu$g/kg or 120 $\mu$g/kg IV Q2W or placebo IV Q2W for a total of 6 doses. UTTR1 147A is administered as a monotherapy. The first dose of study drug (UTTR1147A or placebo) will be administered on Day 1 (+ 1 day) after HSCT (Day 0); subsequent doses will be administered Q2W through Day 71 post-transplant. In one example, the patient population for the study is the same patient population as described in Example 1. Any of the efficacy endpoints described in Example 1 can be assessed.

**Example 13: Alternate IL-22 Fc Fusion Protein Monotherapy Dosing Regimen for Prevention of Acute GVHD**

This example describes an alternate dosing regimen for the study described in Example 10. Patients will be randomly assigned in a 1:1 ratio to receive either UTTR1147A 30 μg/kg IV Q2W or placebo IV Q2W for a total of 6 doses. UTTR1147A is administered as a monotherapy. The first dose of study drug (UTTR1147A or placebo) will be administered on Day 2 after HSCT (Day 0); subsequent doses will be administered Q2W. In one example, the patient population for the study is the same patient population as described in Example 1. Any of the efficacy endpoints described in Example 1 can be assessed.

**Example 14: Alternate IL-22 Fc Fusion Protein Monotherapy Dosing Regimen for Prevention of Acute GVHD**

This example describes an alternate dosing regimen for the study described in Example 10. Patients will be randomly assigned in a 1:1 ratio to receive either UTTR1147A 60 μg/kg IV Q2W or placebo IV Q2W for a total of 6 doses. UTTR1147A is administered as a monotherapy. The first dose of study drug (UTTR1147A or placebo) will be administered on Day 2 after HSCT (Day 0); subsequent doses will be administered Q2W. In one example, the patient population for the study is the same patient population as described in Example 1. Any of the efficacy endpoints described in Example 1 can be assessed.

**Example 15: Alternate IL-22 Fc Fusion Protein Monotherapy Dosing Regimen for Prevention of Acute GVHD**

This example describes an alternate dosing regimen for the study described in Example 10. Patients will be randomly assigned in a 1:1 ratio to receive either UTTR1147A 90 μg/kg or 120 μg/kg IV Q2W or placebo IV Q2W for a total of 6 doses. UTTR1 147A is administered as a monotherapy. The first dose of study drug (UTTR1147A or placebo) will be administered on Day 2 after HSCT (Day 0); subsequent doses will be administered Q2W. In one example, the patient population for the study is the same patient population as described in Example 1. Any of the efficacy endpoints described in Example 1 can be assessed.

**Example 16: Alternate IL-22 Fc Fusion Protein Monotherapy Dosing Regimen for Prevention of Acute GVHD**

This example describes an alternate dosing regimen for the study described in Example 10. Patients will be randomly assigned in a 1:1 ratio to receive either UTTR1147A 30 μg/kg IV Q2W or placebo IV Q2W for a total of 6 doses. UTTR1147A is administered as a monotherapy. The first dose of study drug (UTTR1147A or placebo) will be administered on Day 3 after HSCT (Day 0); subsequent doses will be administered Q2W. In one example, the patient population for the study is the same patient population as described in Example 1. Any of the efficacy endpoints described in Example 1 can be assessed.

**Example 17: Alternate IL-22 Fc Fusion Protein Monotherapy Dosing Regimen for Prevention of Acute GVHD**

This example describes an alternate dosing regimen for the study described in Example 10. Patients will be randomly assigned in a 1:1 ratio to receive either UTTR1147A 60 μg/kg IV Q2W or placebo IV Q2W for a total of 6 doses. UTTR1147A is administered as a monotherapy. The first dose of study drug (UTTR1147A or placebo) will be administered on Day 3 after HSCT (Day 0); subsequent doses will be administered Q2W. In one example, the patient population for the study is the same patient population as described in Example 1. Any of the efficacy endpoints described in Example 1 can be assessed.

**Example 18: Alternate IL-22 Fc Fusion Protein Monotherapy Dosing Regimen for Prevention of Acute GVHD**

This example describes an alternate dosing regimen for the study described in Example 10. Patients will be randomly assigned in a 1:1 ratio to receive either UTTR1147A 90 μg/kg or 120 μg/kg IV Q2W or placebo IV Q2W for a total of 6 doses. UTTR1 147A is administered as a monotherapy. The first dose of study drug (UTTR1147A or placebo) will be administered on Day 3 after HSCT (Day 0); subsequent doses will be administered Q2W. In one example, the patient population for the study is the same patient population as described in Example 1. Any of the efficacy endpoints described in Example 1 can be assessed.

**Example 19: Alternate IL-22 Fc Fusion Protein Monotherapy Dosing Regimen for Prevention of Acute GVHD**

This example describes an alternate dosing regimen for the study described in Example 10. Patients will be randomly assigned in a 1:1 ratio to receive either UTTR1147A 30 μg/kg, 60 μg/kg, 90 μg/kg, or 120 μg/kg IV Q2W or placebo IV Q2W for a total of 6 doses. UTTR1147A is administered as a monotherapy. The first dose of study drug (UTTR1147A or placebo) will be administered on Day 3, Day 2, or Day 1 prior to HSCT, or on the day of HSCT; subsequent doses will be administered Q2W. In one example, the patient population for the study is the same patient population as described in Example 1. Any of the efficacy endpoints described in Example 1 can be assessed.

**Example 20: A Randomized, Observed-Blinded, Phase 1b, Multiple Ascending-Dose Study of UTTR1147A, an IL-22 Fc Fusion Protein, in Healthy Volunteers and Ulcerative Colitis Patients**

**Methods**

*Study Design*

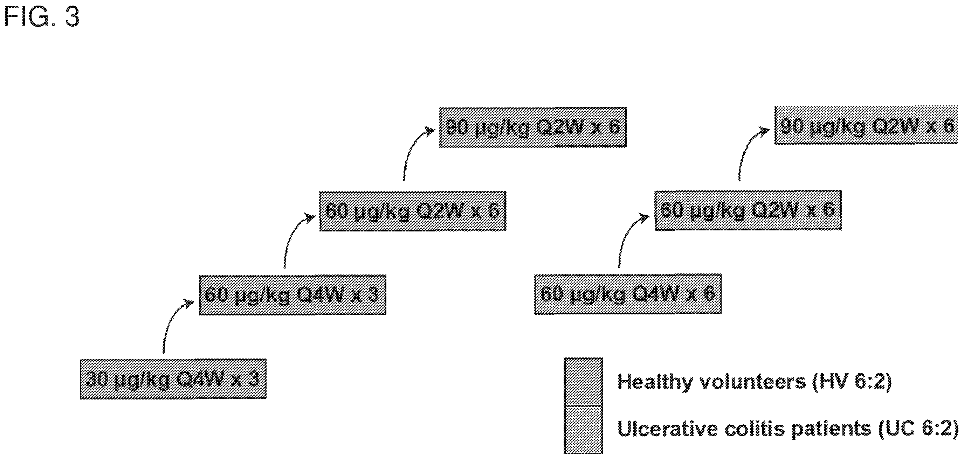

A phase I study (NCT02749630) was conducted to evaluate the safety, tolerability, pharmacokinetics (PK), and pharmacodynamics (PD) of repeat intravenous (IV) dosing of UTTR1147A in healthy volunteers (HVs) and ulcerative colitis (UC) patients. This study is a randomized, observer-blinded, phase 1b, multiple-ascending dose study. UTTR1147A was administered at doses ranging from 30-90 $\mu$g/kg either biweekly (Q2W) or monthly (Q4W). A study schema is shown in Fig. 3.

*Subjects*

The subjects in this study were healthy volunteers or UC patients. Key inclusion criteria for UC patients included documentation of age-appropriate cancer screening based on local/country-specific guidelines; disease duration of $\geq$12 weeks; and centrally read Mayo endoscopic subscore of $\geq$2 (moderate to severe disease). Key exclusion criteria for UC patients included history of malignancy, inflammatory skin disorders, or primary sclerosing cholangitis; and active anti-tumor necrosis factor (TNF)-induced psoriasiform or eczematous lesions.

*Outcomes and Assessments*

To evaluate safety outcomes, the nature, frequency, severity, and timing of adverse events (AEs) were assessed.
To evaluate PK outcomes, the PK profile of UTTR1147A in HVs and UC patients was characterized.
To evaluate PD outcomes, serum CRP and REG3A levels pre-dose and at defined post-dose time points were measured for all subjects.
To evaluate clinical outcomes (for UC patients), the following were evaluated:

- Change in Mayo Clinic Score (MCS) at baseline, day 30, and day 85

  ∘ Stool frequency, rectal bleeding, and endoscopic score were determined by flexible sigmoidoscopy and centrally read video recording

- Clinical remission: modified MCS (mMCS, maximum score of 9) $\leq$2 and a rectal bleeding subscore of 0, with other subscores $\leq$1
- Clinical response (one of the following): $\geq$3 point decrease from baseline in mMCS *and* either $\geq$1 point decrease from baseline in rectal bleeding subscore or a rectal bleeding subscore of 0 or 1, or achieved clinical remission

**Results**

*Subjects*

Table 2 shows a summary of subject demographics and baseline characteristics.

**Table 2: Subject Demographics and Baseline Characteristics**

| | HV | | | | | | UC | | | | | All Subjects (N=56) |
|---|---|---|---|---|---|---|---|---|---|---|---|---|
| | Placebo (n=8) | 30 μg/kg Q4W × 3 (n=6) | 60 μg/kg Q4W × 3 (n=6) | 60 μg/kg Q2W × 6 (n=6) | 90 μg/kg Q2W × 6 (n=6) | All Treated (n=24) | Placebo (n=6) | 60 μg/kg Q4W × 3 (n=6) | 60 μg/kg Q2W × 6 (n=6) | 90 μg/kg Q2W × 6 (n=6) | All Treated (n=18) | |
| Age (y) | 39 (7) | 34 (7) | 37 (9) | 37 (10) | 42 (7) | 38 (8) | 40 (13) | 41 (16) | 40 (16) | 45 (7) | 42 (13) | 39 (10) |
| Sex, male | 8 (100%) | 6 (100%) | 6 (100%) | 6 (100%) | 6 (100%) | 24 (100%) | 3 (50%) | 5 (83%) | 3 (50%) | 6 (100%) | 14 (78%) | 49 (88%) |
| Ethnicity, not Hispanic or Latino | 8 (100%) | 6 (100%) | 6 (100%) | 6 (100%) | 6 (100%) | 24 (100%) | 6 (100%) | 6 (100%) | 6 (100%) | 6 (100%) | 18 (100%) | 56 (100%) |
| Race, White | 8 (100%) | 6 (100%) | 6 (100%) | 6 (100%) | 6 (100%) | 24 (100%) | 6 (100%) | 6 (100%) | 6 (100%) | 6 (100%) | 18 (100%) | 56 (100%) |
| Weight (kg) | 81 (8) | 80 (10) | 82 (8) | 87 (12) | 84 (8) | 84 (9) | 83 (26) | 80 (13) | 81 (18) | 91 (17) | 84 (16) | 83 (14) |
| Height (cm) | 181 (6) | 179 (9) | 176 (8) | 182 (6) | 178 (12) | 179 (8) | 176 (14) | 178 (5) | 175 (8) | 184 (6) | 179 (7) | 179 (8) |
| Disease duration (y) | - | - | - | - | - | - | 8 (7) | 6 (6) | 9 (7) | 11 (14) | 9 (9) | - |
| Azathioprine | - | - | - | - | - | - | 1 (17%) | 1 (17%) | 1 (17%) | 2 | 4 (22%) | - |
| Budesonide | - | - | - | - | - | - | 2 (33%) | 2 (33%) | 1 (17%) | 0 | 3 (17%) | - |
| Hydrocortisone | - | - | - | - | - | - | 1 (17%) | 0 | 0 | 0 | 0 | - |
| Infliximab | - | - | - | - | - | - | 0 | 0 | 1 (17%) | 0 | 1 (6%) | - |
| Mesalazine | - | - | - | - | - | - | 5 (83%) | 6 (100%) | 3 (50%) | 5 (83%) | 14 (78%) | - |
| Prednisolone | - | - | - | - | - | - | 4 (67%) | 2 (33%) | 3 (50%) | 0 | 5 (28%) | - |
| Vedolizumab | - | - | - | - | - | - | 1 (17%) | 1 (17%) | 1 (17%) | 0 | 2 (11%) | - |
| Endoscopic score | - | - | - | - | - | - | 2.7 (0.5) | 2.7 (0.5) | 2.7 (0.5) | 2.3 (0.5) | 2.6 (0.5) | - |
| Modified MCS | - | - | - | - | - | - | 5.8 (2.0) | 6.7 (0.8) | 5.8 (1.5) | 3.2 (1.5) | 5.2 (2.0) | - |
| Data are mean (SD) or n (%). | | | | | | | | | | | | |

*Safety*

56 subjects received at least 1 dose of study drug. Table 3 shows a summary of AEs that occurred in ≥10% of all subjects. The most common drug-related AEs (≥10%) were dry skin, erythema, dry lip, skin discomfort, skin exfoliation, and pruritis, with a trend towards lower incidence and severity in UC patients. No treatment-related serious AEs (SAEs), deaths, life-threatening AEs, or suspected unexpected serious adverse reactions (SUSARs) were observed. Four subjects withdrew treatment due to AEs related to treatment: 1 HV subject (60 μg/kg Q4W): "feeling abnormal" and "balance disorder"; 2 HV subjects (90 μg/kg Q2W): cutaneous dose-limiting AEs (DLAEs) of severe dry skin, erythema, and exfoliation; 1 UC subject (90 μg/kg Q2W): DLAE of erythema. Two SAEs not related to study drug that eventually resolved were observed: ankle fracture (HV 30 μg/kg Q4W) and CMV infection (UC: 60 μg/kg Q2W). The maximum tolerated dose in HV was 60 μg/kg Q2W.

**Table 3: Adverse Events (AEs) in ≥10% of All Subjects**

| | HV | | | | | | UC | | | | | All Subjects (N=56) |
|---|---|---|---|---|---|---|---|---|---|---|---|---|
| | Placebo (n=8) | 30 μg/kg Q4W × 3 (n=6) | 60 μg/kg Q4W × 3 (n=6) | 60 μg/kg Q2W × 6 (n=6) | 90 μg/kg Q2W × 6 (n=6) | All Treated (n=24) | Placebo (n=6) | 60 μg/kg Q4W × 3 (n=6) | 60 μg/kg Q2W × 6 (n=6) | 90 μg/kg Q2W × 6 (n=6) | All Treated (n=18) | |
| Total number of subjects with at least one AE | 5 (63) | 5 (83) | 6 (100) | 6 (100) | 6 (100) | 23 (61) | 6 (100) | 6 (100) | 6 (100) | 6 (100) | 18 (75) | 52 (93) |
| Overall total number AEs | 13 | 24 | 37 | 36 | 40 | 137 | 65 | 61 | 64 | 53 | 178 | 393 |
| Lip dry | 0 | 3 (50) | 3 (50) | 5 (83) | 4 (67) | 15 (40) | 1 (17) | 4 (67) | 4 (67) | 5 (83) | 15 (40) | 29 (52) |
| Nasopharyngitis | 3 (38) | 3 (50) | 3 (50) | 5 (83) | 1 (17) | 12 (32) | 3 (50) | 4 (67) | 4 (67) | 5 (83) | 13 (54) | 31 (55) |
| Headache | 2 (25) | 0 | 2 (33) | 0 | 1 (17) | 3 (8) | 3 (50) | 2 (33) | 2 (33) | 2 (33) | 6 (25) | 14 (25) |
| Oropharyngeal pain | 1 (13) | 1 (17) | 1 (17) | 0 | 1 (17) | 3 (8) | 1 (17) | 0 | 2 (33) | 1 (17) | 3 (13) | 8 (14) |
| Dry skin | 1 (13) | 3 (50) | 6 (100) | 5 (83) | 6 (100) | 20 (53) | 2 (33) | 5 (83) | 4 (67) | 6 (100) | 15 (63) | 38 (68) |
| Erythema | 0 | 2 (33) | 1 (17) | 4 (67) | 4 (67) | 11 (29) | 2 (33) | 2 (33) | 1 (17) | 5 (83) | 8 (33) | 21 (38) |
| Skin discomfort | 0 | 0 | 1 (17) | 3 (50) | 4 (67) | 8 (21) | 1 (17) | 1 (17) | 0 | 5 (83) | 6 (25) | 15 (27) |
| Skin exfoliation | 0 | 1 (17) | 1 (17) | 3 (50) | 4 (67) | 9 (24) | 1 (17) | 0 | 2 (33) | 0 | 2 (8) | 12 (21) |
| Pruritus | 0 | 0 | 2 (33) | 1 (17) | 0 | 3 (8) | 0 | 3 (50) | 3 (50) | 1 (17) | 7 (29) | 10 (18) |
| Skin burning sensation | 0 | 0 | 0 | 2 (33) | 2 (33) | 4 (11) | 0 | 1 (17) | 1 (17) | 0 | 2 (8) | 6 (11) |
| Data are n (%) except where otherwise indicated. | | | | | | | | | | | | |
| Data are n (%) except where otherwise indicated. | | | | | | | | | | | | |

*Pharmacokinetics*

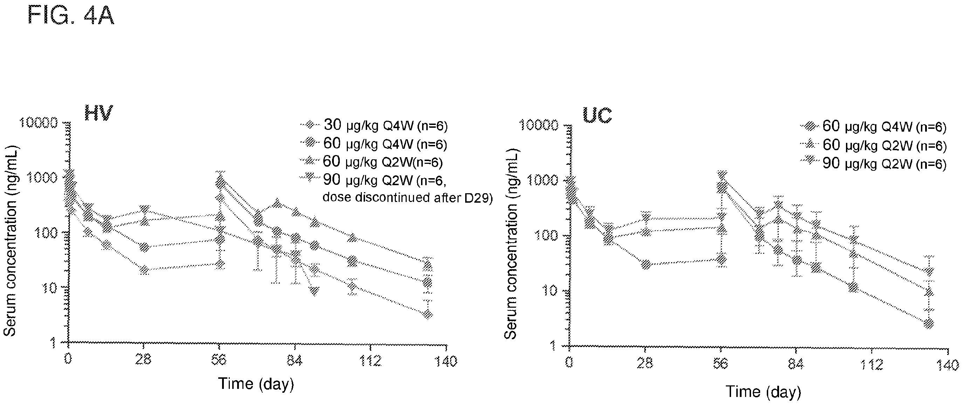

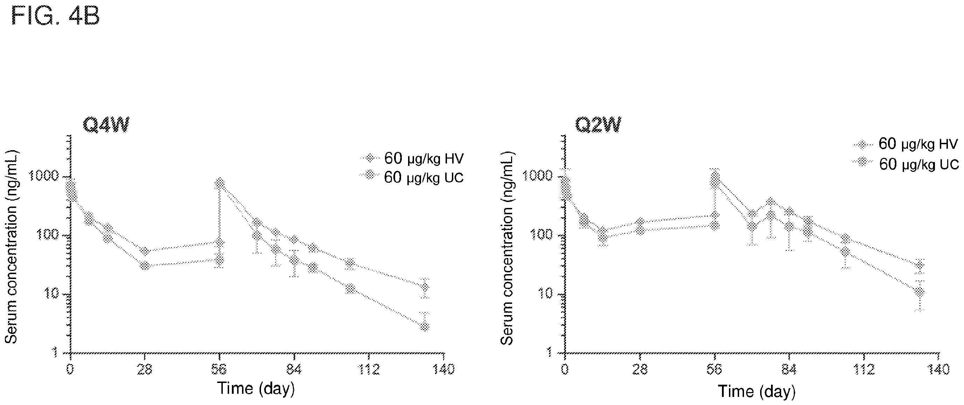

Figs. 4A and 4B show a summary of results of UTTR1147A pharmacokinetics in HVs and UC patients as assessed in serum. UTTR1147A exposures were, in general, dose proportional within HVs and within UC patients, with a mean elimination half-life of approximately 16 days and approximately 12 days, respectively. At the same dose level, UC patients showed relatively lower drug exposures than HVs. Compared to HV exposure, $C_{trough}$ levels in UC patients were approximately 55% with the Q4W regimen and approximately 70% with the Q2W regimen. $AUC_T$ was calculated as the area under the concentration-time curve for a dosing period starting from Day 56. Group means of $AUC_T$ in UC patients were approximately 67% and approximately 60% of those in HVs with the Q4W and Q2W regimens, respectively.

*Pharmacodynamic Biomarkers*

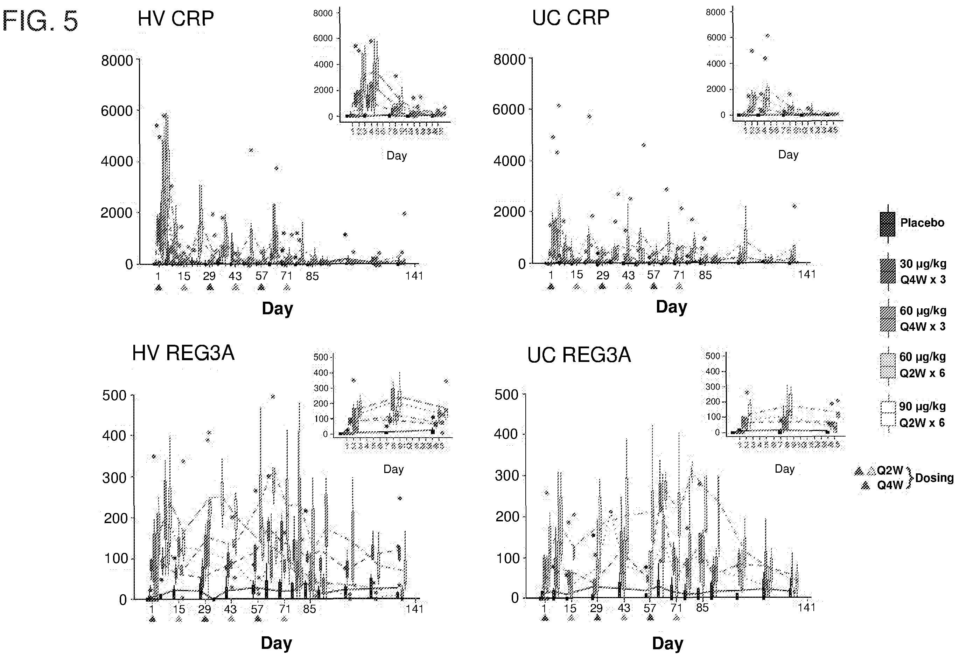

UTTR1147A directly induced dose-dependent increases in serum PD biomarkers REG3A and CRP at all dose cohorts tested compared to placebo (Fig. 5). Notably, UC patients appeared to have attenuated serum PD responses compared to HV.

*Clinical Response*

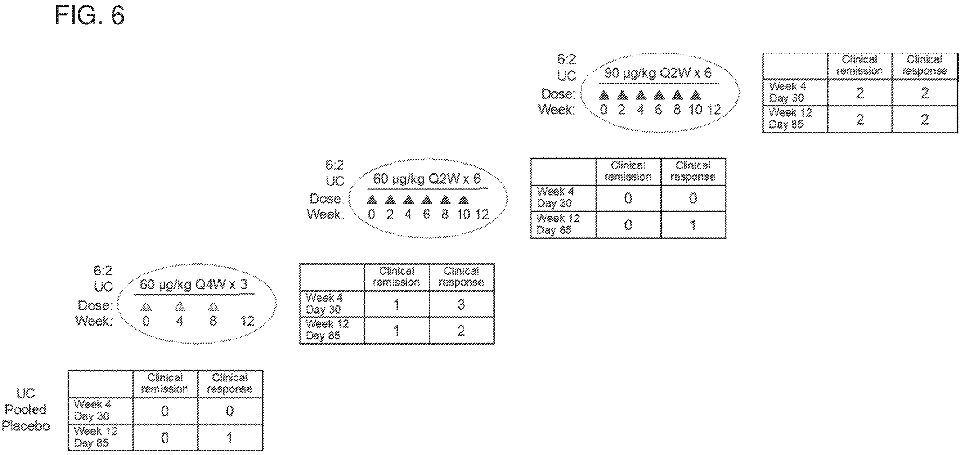

Fig. 6 shows a summary of clinical response and clinical remission in UC patients by Week 4 and Week 12. At Week 4 and/or Week 12, clinical response was observed in 7/18 and 1/6 patients treated with UTTR1147A or placebo, respectively, while clinical remission was observed in 5/18 and 0/6 patients treated with UTTR1147A or placebo, respectively.

**Conclusions**

UTTR1147A demonstrated an adequate safety and PK profile in healthy volunteers and UC patients. The most common AEs were on-target dermatological effects (dry skin, erythema, and pruritis) that were manageable, monitorable, and reversible. PD biomarker data demonstrated dose-dependent pharmacological activity of UTTR1 147A, providing evidence of IL-22R pathway activation. Together with the preliminary signals of efficacy, these data support non-immunosuppressive IL-22 Fc fusion protein therapy in IBD.

**Example 21: A Phase 1b, Open-Label, Dose-Escalation Study to Evaluate the Safety and Pharmacokinetics of UTTR1147A in Combination with Standard of Care in Patients Undergoing Allogeneic Hematopoietic Stem Cell Transplantation**

This Example describes a Phase 1b, open-label, multi-center, dose-escalation study to evaluate the safety, tolerability, and pharmacokinetics of UTTR1147A and to assess activity of UTTR1147A in combination with SOC in the prevention of aGVHD in patients undergoing HSCT. Patients will undergo a screening period, a treatment period of up to approximately 70 days after HSCT, and a follow-up period of approximately 300 days. Approximately 18-24 patients are expected to be enrolled in this study.

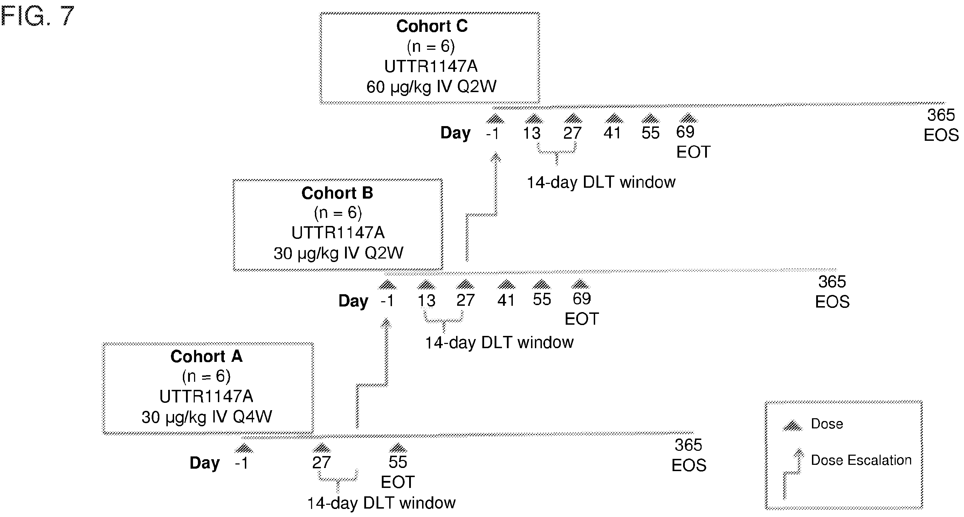

A study schema is provided in Figure 7. Patients receive SOC aGVHD prophylaxis treatment of tacrolimus plus methotrexate. In cases of tacrolimus intolerance, cyclosporine or sirolimus may be used as substitute. Study drug dosing continues in the event of diagnosis of aGVHD. Study drug is discontinued for patients who develop and are treated for corticosteroid-refractory aGVHD and for patients with confirmed primary disease relapse or progression per investigator assessment.

All patients are closely monitored for adverse events during the treatment period, and for at least 70 days after the final dose of study treatment. In particular, patients will be closely monitored for adverse events during a DLT assessment window, defined as the period between administration of the first dose until 14 days after the second dose of study drug. Adverse events are graded according to NCI CTCAE v5.0. A skin biopsy is to be performed on patients at onset of Grade 2 or higher erythematous maculopapular skin rash to characterize the histopathology and to further understand the characteristics of IL-22 Fc-mediated rash versus other sources of rash, including cutaneous aGVHD.

To characterize the PK properties of UTTR1147A, blood samples are taken at various timepoints before and after dosing.

Patients are assessed for Grade II-IV aGVHD, stage of organ-specific aGVHD, corticosteroid-refractory aGVHD, cGVHD, primary disease relapse or progression, and survival. For patients who develop symptoms of Grade II-IV aGVHD, an aGVHD evaluation visit is to be performed within 3 days after starting systemic corticosteroid therapy. In patients with clinical symptoms consistent with aGVHD involving the GI tract, an endoscopic biopsy is encouraged for diagnosis of lower GI aGVHD and should be performed per institutional guidelines.

*Dose-Escalation Stage*

Cohorts of approximately 6 patients each are treated at escalating doses of UTTR1 147A in accordance with the dose-escalation rules described below. The first dose of UTTR1147A is administered on Day -1 (1 day before HSCT), with a window of + 2 days; subsequent doses are administered through Day 69 ($\pm$ 3 days) post-transplant.

During the dose-escalation stage, the planned UTTR1147A dose regimens are as follows:

- Cohort A: UTTR1147A 30 $\mu$g/kg IV every 4 weeks (Q4W) for 3 doses

- Cohort B: UTTR1147A 30 $\mu$g/kg IV Q2W for 6 doses

- Cohort C: UTTR1147A 60 $\mu$g/kg IV Q2W for 6 doses

A cohort mean exposure limit has been set based on the exposure at 60 $\mu$g/kg IV Q2W for HVs in the Phase 1b study in healthy volunteers and UC patients described above in Example 20. In that cohort, the observed maximum $C_{max}$ was 1810 ng/mL and $AUC_{0-14}$ was 4310 ng•day/mL. In this study, if the UTTR1147A 60 $\mu$g/kg IV Q2W dose is tolerated and the observed cohort mean exposures ($C_{max}$ and $AUC_{0-14}$) are lower than the defined exposure limit, an additional cohort may be added (at a dose regimen above 60 $\mu$g/kg IV Q2W but below the expected exposure limit) based on UTTR1147A safety, tolerability, and PK data in the study to date. For example, an additional cohort at 120 $\mu$g/kg IV (e.g., Q2W for 6 doses) may be added.

**Claims**

1. An interleukin-22 (IL-22) Fc fusion protein for use in preventing acute GVHD, reducing the risk of developing chronic GVHD, or reducing the risk of corticosteroid-refractory acute GVHD in a subject comprising administering to a subject in need thereof an IL-22 Fc fusion protein in a dosing regimen comprising a dosing cycle, wherein the dosing cycle comprises up to and no more than six total doses of the IL-22 Fc fusion protein, wherein the dosing cycle comprises a first dose (C1D1) and one or more further doses, wherein each dose is about 60 $\mu$g/kg, and wherein the doses are administered to the subject q1w, (every week) q2w, (every two weeks) q3w, (every three weeks) or q4w, (every four weeks) and wherein the IL-22 Fc fusion protein comprises the amino acid sequence of SEQ ID NO:8.

2. The IL-22 Fc fusion protein for use of claim 1, wherein the C1D1 is administered to the subject prior to, concurrently with, or after allo-HSCT.

3. The IL-22 Fc fusion protein for use of claim 2, wherein the C1D1 is administered to the subject prior to allo-HSCT.

4. The IL-22 Fc fusion protein for use of claim 3, wherein the C1D1 is administered to the subject 1 to 3 days prior to allo-HSCT.

5. The IL-22 Fc fusion protein for use of claim 4, wherein the C1D1 is administered to the subject 1 day prior to allo-HSCT.

6. The IL-22 Fc fusion protein for use of any one of claims 1-5, wherein:

    (i) the one or more further doses comprise at least a second dose (C1D2);
    (ii) the one or more further doses comprise at least a C1D2 and a third dose (C1D3);
    (iii) the one or more further doses comprise at least a C1D2, a C1D3, and a fourth dose (C1D4);
    (iv) the one or more further doses comprise at least a C1D2, a C1D3, a C1D4, and a fifth dose (C1D5);
    (v) the dosing cycle comprises the C1D1, a C1D2, a C1D3, a C1D4, a C1D5, and a sixth dose (C1D6) of the IL-22 Fc fusion protein; and/or
    (vi) the doses are administered to the subject q2w.

7. The IL-22 Fc fusion protein for use of any one of claims 1-6, wherein the dosing cycle has a length of about 70 ($\pm$3) days.

8. The IL-22 Fc fusion protein for use of claim 7, wherein:

    (i) the dosing cycle has a length of about 70 days; and/or

(ii) the dosing cycle consists of a C1D1, a C1D2, a C1D3, a C1D4, a C1D5, and a C1D6, and wherein the C1D1 is administered to the subject 1 day prior to allo-HSCT, the C1D2 is administered to the subject 13 days after allo-HSCT, the C1D3 is administered to the subject 27 days after allo-HSCT, the C1D4 is administered to the subject 41 days after allo-HSCT, the C1D5 is administered to the subject 55 days after allo-HSCT, and the C1D6 is administered to the subject 69 days after allo-HSCT.

9. The IL-22 Fc fusion protein for use of any one of claims 1-2, wherein the C1D1 is administered to the subject after allo-HSCT.

10. The IL-22 Fc fusion protein for use of any one of claims 1-2 and 9, wherein the C1D1 is administered to the subject 1 to 3 days after allo-HSCT.

11. The IL-22 Fc fusion protein for use of any one of claims 1-2, 9, and 10, wherein the C1D1 is administered to the subject within 2 days of allo-HSCT.

12. The IL-22 Fc fusion protein for use of claim 10 or 11, wherein the C1D1 is administered to the subject one day after allo-HSCT.

13. The IL-22 Fc fusion protein for use of any one of claims 1-12, wherein the IL-22 Fc fusion protein is administered to the subject in a pharmaceutical composition.

14. The IL-22 Fc fusion protein for use of any one of claims 1-13, wherein the IL-22 Fc fusion protein is administered to the subject as a monotherapy.

15. The IL-22 Fc fusion protein for use of any one of claims 1-14, wherein the IL-22 Fc fusion protein is administered to the subject as a combination therapy.


**Patentansprüche**

1. Interleukin-22-(IL-22)-Fc-Fusionsprotein zur Verwendung beim Vorbeugen von akuter GVHD, Reduzieren des Risikos des Entwickelns chronischer GVHD oder Reduzieren des Risikos für Corticosteroid-refraktäre akute GVHD bei einem Individuum, umfassend das Verabreichen eines IL-22-Fc-Fusionsproteins an ein Individuum, das eines solchen bedarf, in einem Dosierregime, das einen Dosierzyklus umfasst, wobei der Dosierzyklus bis zu und nicht mehr als sechs Gesamtdosen des IL-22-Fc-Fusionsproteins umfasst, wobei der Dosierzyklus eine erste Dosis (C1D1) und eine oder mehrere weitere Dosis/Dosen umfasst, wobei jede Dosis etwa 60 $\mu$g/kg beträgt und wobei die Dosen dem Individuum q1w (jede Woche), q2w (alle zwei Wochen), q3w (alle drei Wochen) oder q4w (alle vier Wochen) verabreicht werden und wobei das IL-22-Fc-Fusionsprotein die Aminosäuresequenz von SEQ ID NO:8 umfasst.

2. IL-22-Fc-Fusionsprotein zur Verwendung nach Anspruch 1, wobei die C1D1 dem Individuum vor, gleichzeitig mit oder nach allo-HSCT verabreicht wird.

3. IL-22-Fc-Fusionsprotein zur Verwendung nach Anspruch 2, wobei die C1D1 dem Individuum vor allo-HSCT verabreicht wird.

4. IL-22-Fc-Fusionsprotein zur Verwendung nach Anspruch 3, wobei die C1D1 dem Individuum 1 bis 3 Tage vor allo-HSCT verabreicht wird.

5. IL-22-Fc-Fusionsprotein zur Verwendung nach Anspruch 4, wobei die C1D1 dem Individuum 1 Tag vor allo-HSCT verabreicht wird.

6. IL-22-Fc-Fusionsprotein zur Verwendung nach einem der Ansprüche 1-5, wobei:

(i) die eine weitere Dosis oder mehreren weiteren Dosen mindestens eine zweite Dosis (C1D2) umfasst/umfassen;
(ii) die eine weitere Dosis oder mehreren weiteren Dosen mindestens eine C1D2 und eine dritte Dosis (C1D3) umfasst/umfassen;

(iii) die eine weitere Dosis oder mehreren weiteren Dosen mindestens eine C1D2, eine C1D3 und eine vierte Dosis (C1D4) umfasst/umfassen;

(iv) die eine weitere Dosis oder mehreren weiteren Dosen mindestens eine C1D2, eine C1D3, eine C1D4 und eine fünfte Dosis (C1D5) umfasst/umfassen;

(v) der Dosierzyklus die C1D1, eine C1D2, eine C1D3, eine C1D4, eine C1D5 und eine sechste Dosis (C1D6) des IL-22-Fc-Fusionsproteins umfasst, und/oder

(vi) die Dosen dem Individuum q2w verabreicht werden.

7.  IL-22-Fc-Fusionsprotein zur Verwendung nach einem der Ansprüche 1-6, wobei der Dosierzyklus eine Länge von etwa 70 ($\pm$ 3) Tagen hat.

8.  IL-22-Fc-Fusionsprotein zur Verwendung nach Anspruch 7, wobei:

(i) der Dosierzyklus eine Länge von etwa 70 Tagen hat; und/oder

(ii) der Dosierzyklus aus einer C1D1, einer C1D2, einer C1D3, einer C1D4, einer C1D5 und einer C1D6 besteht und wobei die C1D1 dem Individuum 1 Tag vor allo-HSCT verabreicht wird, die C1D2 dem Individuum 13 Tage nach allo-HSCT verabreicht wird, die C1D3 dem Individuum 27 Tage nach allo-HSCT verabreicht wird, die C1D4 dem Individuum 41 Tage nach allo-HSCT verabreicht wird, die C1D5 dem Individuum 55 Tage nach allo-HSCT verabreicht wird und die C1D6 dem Individuum 69 Tage nach allo-HSCT verabreicht wird.

9.  IL-22-Fc-Fusionsprotein zur Verwendung nach einem der Ansprüche 1-2, wobei die C1D1 dem Individuum nach allo-HSCT verabreicht wird.

10.  IL-22-Fc-Fusionsprotein zur Verwendung nach einem der Ansprüche 1-2 und 9, wobei die C1D1 dem Individuum 1 bis 3 Tage nach allo-HSCT verabreicht wird.

11.  IL-22-Fc-Fusionsprotein zur Verwendung nach einem der Ansprüche 1-2, 9 und 10, wobei die C1D1 dem Individuum innerhalb von 2 Tagen nach allo-HSCT verabreicht wird.

12.  IL-22-Fc-Fusionsprotein zur Verwendung nach Anspruch 10 oder 11, wobei die C1D1 dem Individuum einen Tag nach allo-HSCT verabreicht wird.

13.  IL-22-Fc-Fusionsprotein zur Verwendung nach einem der Ansprüche 1-12, wobei das IL-22-Fc-Fusionsprotein dem Individuum in einer pharmazeutischen Zusammensetzung verabreicht wird.

14.  IL-22-Fc-Fusionsprotein zur Verwendung nach einem der Ansprüche 1-13, wobei das IL-22-Fc-Fusionsprotein dem Individuum als eine Monotherapie verabreicht wird.

15.  IL-22-Fc-Fusionsprotein zur Verwendung nach einem der Ansprüche 1-14, wobei das IL-22-Fc-Fusionsprotein dem Individuum als eine Kombinationstherapie verabreicht wird.


**Revendications**

1.  Protéine de fusion interleukine-22 (IL-22)-Fc pour une utilisation dans la prévention de la GVHD aiguë, la réduction du risque de développement d'une GVHD chronique ou la réduction du risque de GVHD aiguë réfractaire aux corticoïdes chez un sujet comprenant l'administration à un sujet en ayant besoin d'une protéine de fusion IL-22-Fc dans un schéma de dosage comprenant un cycle de dosage, dans laquelle le cycle de dosage comprend jusqu'à et pas plus de six doses totales de la protéine de fusion IL-22-Fc, dans laquelle le cycle de dosage comprend une première dose (C1D1) et une ou plusieurs doses supplémentaires, dans laquelle chaque dose est d'environ 60 µg/kg, et dans laquelle les doses sont administrées au sujet q1w (toutes les semaines), q2w (toutes les deux semaines), q3w (toutes les trois semaines) ou q4w (toutes les quatre semaines) et dans laquelle la protéine de fusion IL-22-Fc comprend la séquence d'acides aminés de SEQ ID NO : 8.

2.  Protéine de fusion IL-22-Fc pour une utilisation selon la revendication 1, dans laquelle la C1D1 est administrée au sujet avant, simultanément avec ou après une allo-GCSH.

3.  Protéine de fusion IL-22-Fc pour une utilisation selon la revendication 2, dans laquelle la C1D1 est administrée au sujet avant une allo-GCSH.

4. Protéine de fusion IL-22-Fc pour une utilisation selon la revendication 3, dans laquelle la C1D1 est administrée au sujet 1 à 3 jours avant une allo-GCSH.

5. Protéine de fusion IL-22-Fc pour une utilisation selon la revendication 4, dans laquelle la C1D1 est administrée au sujet 1 jour avant une allo-GCSH.

6. Protéine de fusion IL-22-Fc pour une utilisation selon l'une quelconque des revendications 1 à 5, dans laquelle :

(i) la ou les doses supplémentaires comprennent au moins une deuxième dose (C1D2) ;
(ii) la ou les doses supplémentaires comprennent au moins une C1D2 et une troisième dose (C1D3) ;
(iii) la ou les doses supplémentaires comprennent au moins une C1D2, une C1D3 et une quatrième dose (C1D4) ;
(iv) la ou les doses supplémentaires comprennent au moins une C1D2, une C1D3, une C1D4 et une cinquième dose (C1D5) ;
(v) le cycle de dosage comprend la C1D1, une C1D2, une C1D3, une C1D4, une C1D5 et une sixième dose (C1D6) de la protéine de fusion IL-22-Fc ; et/ou
(vi) les doses sont administrées au sujet q2w.

7. Protéine de fusion IL-22-Fc pour une utilisation selon l'une quelconque des revendications 1 à 6, dans laquelle le cycle de dosage a une longueur d'environ 70 ($\pm$ 3) jours.

8. Protéine de fusion IL-22-Fc pour une utilisation selon la revendication 7, dans laquelle :

(i) le cycle de dosage a une longueur d'environ 70 jours ; et/ou
(ii) le cycle de dosage est constitué d'une C1D1, d'une C1D2, d'une C1D3, d'une C1D4, d'une C1D5 et d'une C1D6, et dans laquelle la C1D1 est administrée au sujet 1 jour avant l'allo-GCSH, la C1D2 est administrée au sujet 13 jours après l'allo-GCSH, la C1D3 est administrée au sujet 27 jours après l'allo-GCSH, la C1D4 est administrée au sujet 41 jours après l'allo-GCSH, la C1D5 est administrée au sujet 55 jours après l'allo-GCSH et la C1D6 est administrée au sujet 69 jours après l'allo-GCSH.

9. Protéine de fusion IL-22-Fc pour une utilisation selon l'une quelconque des revendications 1 à 2, dans laquelle la C1D1 est administrée au sujet après l'allo-GCSH.

10. Protéine de fusion IL-22-Fc pour une utilisation selon l'une quelconque des revendications 1 à 2 et 9, dans laquelle la C1D1 est administrée au sujet 1 à 3 jours après l'allo-GCSH.

11. Protéine de fusion IL-22-Fc pour une utilisation selon l'une quelconque des revendications 1 à 2, 9 et 10, dans laquelle la C1D1 est administrée au sujet dans les 2 jours suivant l'allo-GCSH.

12. Protéine de fusion IL-22-Fc pour une utilisation selon la revendication 10 ou 11, dans laquelle la C1D1 est administrée au sujet un jour après l'allo-GCSH.

13. Protéine de fusion IL-22-Fc pour une utilisation selon l'une quelconque des revendications 1 à 12, dans laquelle la protéine de fusion IL-22-Fc est administrée au sujet dans une composition pharmaceutique.

14. Protéine de fusion IL-22-Fc pour une utilisation selon l'une quelconque des revendications 1 à 13, dans laquelle la protéine de fusion IL-22-Fc est administrée au sujet en monothérapie.

15. Protéine de fusion IL-22-Fc pour une utilisation selon l'une quelconque des revendications 1 à 14, dans laquelle la protéine de fusion IL-22-Fc est administrée au sujet en thérapie combinatoire.

# FIG. 1

HSCT (Day 0)
Enrollment (Day 1)

| Open-Label Safety Run-in Phase | Screening | UTTR1147A 60 µg/kg IV + SOC*ᵃ (n= 10) | Follow-Up |

Up to Day -31    Day 1ᵇ   15   29   43   57   71    Primary Efficacy EP Day 180     Day 365

⬇ Enrollment in the placebo-controlled RCT phase (Section 3.1.2)

HSCT (Day 0)
1:1 Randomization (Day 1)

| Randomized Double-Blind Phase | Screening | UTTR1147A 60 µg/kg IV + SOC*ᵃ (n= 60) / UTTR1147A placebo + SOC*ᵃ (n= 60) | Follow-Up |

Up to Day -31    Day 1ᵇ   15   29   43   57   71    Primary Efficacy EP Day 180     Day 365

▲ Study drug dosing

EP 4 003 400 B1

98

# FIG. 2

**IL-22 Amino Acid Sequences Alignment**

```
Human (Q9GZX6)              apisshcrldksnfqgpyitnrtfmlakeasladnntdvrligeklfhgvsmsercylmk   60
Chimpanzee (XP_003313906)   apisshcrldkssfqgpyitnrtfmlakeasladnntdvrligeklfhgvsmsercylmk
Orangutan (XP_002823544)    apisshcrldksnfqgpyitnrtfmlakeasladnntdvrligeklfrgvsmsercylmk
Mouse (Q9JJY9)              lpvntrcklevsnfqgpyivnrtfmlakeasladnntdvrligeklfrgvsakdqcylmk
Dog (XP_538274)             lpisshcrldksnfqgpyitnrtfmlakeasladnntdvrligeklfhgvnmgercylml
                             *      *  *   * ******  ********************************  **      *****


Human (Q9GZX6)              qvlnftleevlfpqsdrfqpymqevvpflarlsnrlstchiegddlhigrnvqklkdtvk  120
Chimpanzee (XP_003313906)   qvlnftleevlfpqsdrfqpymqevvpflarlsnrlstchiegddlhigrnvqklkdtvk
Orangutan (XP_002823544)    qvlnftleevlfpqsdrfqpymqevvpflarlsnrlstchiegddlhigrnvqklkdtvk
Mouse (Q9JJY9)             qvlnftledvllpqsdrfqpymqevvpfltklsnqlsschisgddqniqknvrrlketvk
Dog (XP_538274)            evlnftleevllpqsdrfqpymqevvpflarlsnklsqchienddghigrnvqklkdtvq
                            *******  ** *******************  ***  **  ***   **   **  **   **  **


Human (Q9GZX6)              klgesgeikaigeldllfmslrnaci   146      (SEQ ID NO:4)
Chimpanzee (XP_003313906)   klgengeikaigeldllfmslrnaci            (SEQ ID NO:48)
Orangutan (XP_002823544)    klgesgeikaigeldllfmslrnaci            (SEQ ID NO:49)
Mouse (Q9JJY9)             klgesgeikaigeldllfmslrnacv            (SEQ ID NO:50)
Dog (XP_538274)            klgengeikaigeldllfmalrnacv            (SEQ ID NO:51)
                            ****  ***************  *****
```

FIG. 3

90 µg/kg Q2W x 6

90 µg/kg Q2W x 6

60 µg/kg Q2W x 6

60 µg/kg Q2W x 6

60 µg/kg Q4W x 3

60 µg/kg Q4W x 6

30 µg/kg Q4W x 3

Healthy volunteers (HV 6:2)

Ulcerative colitis patients (UC 6:2)

EP 4 003 400 B1

FIG. 4A

HV

Serum concentration (ng/mL)

- 30 µg/kg Q4W (n=6)
- 60 µg/kg Q4W (n=6)
- 60 µg/kg Q2W (n=6)
- 90 µg/kg Q2W (n=6, dose discontinued after D29)

10000
1000
100
10
1

0    28    56    84    112    140

Time (day)

UC

Serum concentration (ng/mL)

- 60 µg/kg Q4W (n=6)
- 60 µg/kg Q2W (n=6)
- 90 µg/kg Q2W (n=6)

10000
1000
100
10
1

0    28    56    84    112    140

Time (day)

FIG. 4B

FIG. 5

HV CRP

UC CRP

HV REG3A

UC REG3A

Legend: Placebo; 30 µg/kg Q4W x 3; 60 µg/kg Q4W x 3; 60 µg/kg Q2W x 6; 90 µg/kg Q2W x 6; Q2W Dosing; Q4W Dosing

# FIG. 6

6:2
UC

90 µg/kg Q2W x 6

Dose: ▲ ▲ ▲ ▲ ▲ ▲
Week: 0 2 4 6 8 10 12

| | Clinical remission | Clinical response |
|---|---|---|
| Week 4 Day 30 | 2 | 2 |
| Week 12 Day 85 | 2 | 2 |

6:2
UC

60 µg/kg Q2W x 6

Dose: ▲ ▲ ▲ ▲ ▲ ▲
Week: 0 2 4 6 8 10 12

| | Clinical remission | Clinical response |
|---|---|---|
| Week 4 Day 30 | 0 | 0 |
| Week 12 Day 85 | 0 | 1 |

6:2
UC

60 µg/kg Q4W x 3

Dose: ▲ ▲ ▲
Week: 0 4 8 12

| | Clinical remission | Clinical response |
|---|---|---|
| Week 4 Day 30 | 1 | 3 |
| Week 12 Day 85 | 1 | 2 |

UC
Pooled
Placebo

| | Clinical remission | Clinical response |
|---|---|---|
| Week 4 Day 30 | 0 | 0 |
| Week 12 Day 85 | 0 | 1 |

# FIG. 7

Cohort C
(n = 6)
UTTR1147A
60 µg/kg IV Q2W

Day  -1  13  27  41  55  69
EOT
14-day DLT window

365
EOS

Cohort B
(n = 6)
UTTR1147A
30 µg/kg IV Q2W

Day  -1  13  27  41  55  69
EOT
14-day DLT window

365
EOS

Cohort A
(n = 6)
UTTR1147A
30 µg/kg IV Q4W

Day  -1  27  55
EOT
14-day DLT window

365
EOS

Dose

Dose Escalation

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014145016 A2
- US 20180355009 A1
- WO 2015070077 A1
- US 5821333 A
- US 7642228 B
- US 20110287009 A
- US 2012059810 W
- US 9815880 B
- WO 2008077546 A
- US 20030157108 A
- US 20040093621 A
- WO 200061739 A
- WO 200129246 A
- US 20030115614 A
- US 20020164328 A
- US 20040132140 A
- US 20040110704 A
- US 20040110282 A
- US 20040109865 A
- WO 2003085119 A
- WO 2003084570 A
- WO 2005035586 A
- WO 2005035778 A
- WO 2005053742 A
- WO 2002031140 A
- US 20030157108 A1
- WO 2004056312 A1
- WO 2003085107 A
- WO 2003011878 A
- US 6602684 B
- US 20050123546 A
- WO 199730087 A
- WO 199858964 A
- WO 199922764 A

- US 5500362 A
- US 5821337 A
- WO 2006029879 A
- WO 2005100402 A
- US 6737056 B
- US 7332581 B
- WO 2004056312 A
- US 6194551 B
- WO 9951642 A
- US 20050014934 A1, Hinton
- US 7371826 B
- US 5648260 A
- US 5624821 A
- WO 9429351 A
- US 7521541 B
- US 5364934 A
- US 2019015277 W
- WO 8905859 A
- US 4399216 A
- US 2019015268 W
- US 20050260186 A
- US 20060104968 A
- US 6267958 B
- US 6171586 B
- WO 2006044908 A
- US 3773919 A
- US 4717717 A
- US 5130298 A
- US 5427778 A
- US 5457093 A
- US 5705485 A
- US 6331309 B
- WO 2006138468 A

**Non-patent literature cited in the description**

- **LINDEMANS et al.** *Blood,* 2014, vol. 124 (21), 651
- **LINDEMANS et al.** *Biology of Blood and Marrow Transplantation,* 2015, vol. 21 (2), S58-S59
- **OUYANG et al.** *Annu. Rev. Immunol.,* 2011, vol. 29, 159-63
- **KABAT et al.** Sequences of Proteins of Immunological Interest. Public Health Service, National Institutes of Health, 1991
- **VARKI et al.** Essentials of Glycobiology. Cold Spring Harbor Laboratory Press, 2015

- **KINDT et al.** Kuby Immunology. W.H. Freeman and Co, 2007, 91
- **PORTOLANO et al.** *J. Immunol.,* 1993, vol. 150, 880-887
- **CLARKSON et al.** *Nature,* 1991, vol. 352, 624-628
- **HARRIS et al.** *Biol. Blood Marrow Transplant.,* 2016, vol. 22 (4-10
- **GLUCKSBERG et al.** *Transplantation,* 1974, vol. 18, 295-304

- **FILIPOVICH et al.** *Biol. Blood. Marrow Transplant.,* 2005, vol. 11, 945-955
- **JAGASIA et al.** *Biol. Blood. Marrow Transplant.,* 2015, vol. 21 (3), 389-401
- **JAGASIA et al.** *Biol. Blood Marrow Transplant.,* 2015, vol. 21, 389-401
- **SAMBROOK et al.** Molecular Cloning: A Laboratory Manual. Cold Spring Harbor Laboratory Press, 1989
- **INNIS et al.** PCR Protocols: A Guide to Methods and Applications. Academic Press, 1990
- **HARLOW ; LANE.** Antibodies: A Laboratory Manual. Cold Spring Harbor Laboratory, 1988
- **GLUCKSBERG H et al.** *Transplantation,* 1974, vol. 18 (4), 295-304
- **HARRIS et al.** *Biol Blood Marrow Transplant,* 2016, vol. 22, 4-10
- **COOKE et al.** *Biol. Blood Marrow Transplant,* 2017, vol. 23, 211-234
- **GATZA et al.** *Int. J. Hematol. Oncol.,* 2015, vol. 4 (3), 113-126
- **WRIGHT et al.** *TIBTECH,* 1997, vol. 15, 26-32
- **OKAZAKI et al.** *J. Mol. Biol.,* 2004, vol. 336, 1239-1249
- **YAMANE-OHNUKI et al.** *Biotech. Bioeng.,* 2004, vol. 87, 614
- **RIPKA et al.** *Arch. Biochem. Biophys.,* 1986, vol. 249, 533-545
- **KANDA, Y. et al.** *Biotechnol. Bioeng.,* 2006, vol. 94 (4), 680-688
- **RAVETCH et al.** *Annu. Rev. Immunol.,* 1991, vol. 9, 457-492
- **HELLSTROM et al.** *Proc. Nat'l Acad. Sci. USA,* 1986, vol. 83, 7059-7063
- **HELLSTROM et al.** *Proc. Nat'l Acad. Sci. USA,* 1985, vol. 82, 1499-1502
- **BRUGGEMANN et al.** *J. Exp. Med.,* 1987, vol. 166, 1351-1361
- **CLYNES et al.** *Proc. Nat'l Acad. Sci. USA,* 1998, vol. 95, 652-656
- **GAZZANO-SANTORO et al.** *J. Immunol. Methods,* 1996, vol. 202, 163
- **CRAGG et al.** *Blood,* 2003, vol. 101, 1045-1052
- **CRAGG et al.** *Blood,* 2004, vol. 103, 2738-2743
- **PETKOVA et al.** *Int'l. Immunol.,* 2006, vol. 18 (12), 1759-1769
- **SHIELDS et al.** *J. Biol. Chem.,* 2001, vol. 9 (2), 6591-6604
- **IDUSOGIE et al.** *J. Immunol.,* 2000, vol. 164, 4178-4184
- **GUYER et al.** *J. Immunol.,* 1976, vol. 117, 587
- **KIM et al.** *J. Immunol.,* 1994, vol. 24, 249
- **DUNCAN ; WINTER.** *Nature,* 1988, vol. 322, 738-40
- **STEWART et al.** Solid-Phase Peptide Synthesis. W.H. Freeman Co, 1969
- **MERRIFIELD.** *J. Am. Chem. Soc.,* 1963, vol. 85, 2149-2154
- Mammalian Cell Biotechnology: A Practical Approach. IRL Press, 1991
- **SHAW et al.** *Gene,* 1983, vol. 23, 315
- **GRAHAM ; VAN DER EB.** *Virology,* 1978, vol. 52, 456-457
- **VAN SOLINGEN et al.** *J. Bact,* 1977, vol. 130, 946
- **HSIAO et al.** *Proc. Natl. Acad. Sci. (USA),* 1979, vol. 76, 3829
- **KEOWN et al.** *Methods in Enzymology,* 1990, vol. 185, 527-537
- **MANSOUR et al.** *Nature,* 1988, vol. 336, 348-352
- **DEUTSCHER.** *Methods in Enzymology,* 1990, vol. 182
- Scopes, Protein Purification: Principles and Practice. Springer-Verlag, 1982
- **MERRIFIELD, J.** *Am. Chem. Soc.,* 1963, vol. 85, 2149-2154
- **GRAHAM et al.** *J. Gen Virol.,* 1977, vol. 36, 59
- **URLAUB ; CHASIN.** *Proc. Natl. Acad. Sci. USA,* 1980, vol. 77, 4216
- **MATHER.** *Biol. Reprod.,* 1980, vol. 23, 243-251
- Remington's Pharmaceutical Sciences. 1980
- Remington's Pharmaceutical Sciences. Lippincott, Williams & Wilkins, 2000
- **BRANDSE et al.** *Gastroenterology,* 2015, vol. 149 (2), 350-355
- **FAUSEL et al.** *Ther. Clin. Risk Manag.,* 2015, vol. 11, 63-73
- **ROSEN et al.** *Aliment. Pharmacol. Ther.,* 2015, vol. 41 (11), 1094-1103